(19) Europäisches Patentamt
European Patent Office
Office européen des brevets

(11) EP 2 190 431 B1

# (12) EUROPEAN PATENT SPECIFICATION

(45) Date of publication and mention of the grant of the patent:
**03.07.2013 Bulletin 2013/27**

(21) Application number: **08797177.6**

(22) Date of filing: **05.08.2008**

(51) Int Cl.:
*A61K 31/47* (2006.01) *A61K 45/06* (2006.01)
*A61P 29/00* (2006.01)

(86) International application number:
**PCT/US2008/072201**

(87) International publication number:
**WO 2009/023471 (19.02.2009 Gazette 2009/08)**

## (54) COMPOSITIONS COMPRISING A DISSOCIATED GLUCOCORTICOID RECEPTOR AGONIST FOR TREATING OR CONTROLLING ANTERIOR-SEGMENT INFLAMMATION

ZUSAMMENSETZUNGEN MIT EINEM DISSOZIIERTEN GLUCOCORTICOID-REZEPTOR-AGONIST ZUR BEHANDLUNG ODER KONTROLLE VON ENTZÜNDUNGEN DES ANTERIOREN SEGMENTS

COMPOSITIONS COMPRENANT UN AGONISTE DU RECEPTEUR GLUCOCORTICOIDE DISSOCIE PERMETTANT DE TRAITER OU DE CONTROLER UNE INFLAMMATION DU SEGMENT ANTERIEUR

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **10.08.2007 US 955044 P**

(43) Date of publication of application:
**02.06.2010 Bulletin 2010/22**

(73) Proprietor: **Bausch & Lomb Incorporated**
**Rochester, NY 14604-2701 (US)**

(72) Inventors:
- **WARD, Keith, Wayne**
**Ontario, NY 14519 (US)**
- **BUCOLO, Claudio**
**I-95125 Catania (IT)**

(74) Representative: **Glas, Holger**
**Maiwald Patentanwalts GmbH**
**Elisenhof**
**Elisenstrasse 3**
**80335 München (DE)**

(56) References cited:
**WO-A1-2004/005229**
**WO-A2-2008/021728**
**WO-A2-2008/027796**
**WO-A1-2008/154129**
**WO-A2-2008/021729**
**US-A1- 2006 116 396**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

BACKGROUND OF THE INVENTION

The present invention relates to compositions for use in treating or controlling post-operative ocular inflammation of the anterior segment of an eye and methods for manufacturing such compositions . In particular, the present invention relates to compositions that comprise dissociated glucocorticoid receptor agonists ("DIGRAs") and methods for manufacturing such compositions.

Ocular inflammation is characterized by redness, swelling, and/or pain association with infection, irritation, or trauma to the eye. Common triggers of ocular inflammation include allergies, meibomian gland dysfunction, ocular diseases, and ophthalmic surgical procedures.

The anterior segment of the eye (the term, as used herein, includes the anterior portion of the globe of the eye and adjacent tissues) is continuously exposed to the environment and thus presents many potential opportunities for invasion by environmental virulent pathogens. The common types of microorganisms causing ophthalmic infections are viruses, bacteria, and fungi. These microorganisms may directly invade the surface of the eye or permeate into the globe of the eye through trauma or surgery. The microorganisms may attack any part of the eye structure, including the conjunctiva, the cornea, the uvea, the vitreous body, the retina, and the optic nerve. Ophthalmic infections can cause severe pain, swollen and red tissues in or around the eye, and blurred and decreased vision.

The body's innate cascade is activated soon after invasion by a foreign pathogen begins. Leukocytes (neutrophils, eosinophils, basophils, monocytes, and macrophages) are attracted to the site of infection in an attempt to eliminate the foreign pathogen through phagocytosis. Leukocytes and some affected tissue cells are activated by the pathogens to synthesize and release proinflammatory cytokines such as IL-1$\beta$, IL-3, IL-5, IL-6, IL-8, TNF-$\alpha$ (tumor necrosis factor-$\alpha$), GM-CSF (granulocyte-macrophage colony-stimulating factor), and MCP-1 (monocyte chemotactic protein-1). These released cytokines then further attract more immune cells to the infected site, amplifying the response of the immune system to defend the host against the foreign pathogen. For example, IL-8 and MCP-1 are potent chemoattractants for, and activators of, neutrophils and monocytes, respectively, while GM-CSF prolongs the survival of these cells and increases their response to other proinflammatory agonists. TNF-$\alpha$ can activate both types of cell and can stimulate further release of IL-8 and MCP-1 from them. IL-1 and TNF-$\alpha$ are potent chemoattractants for T and B lymphocytes, which are activated to produce antibodies against the foreign pathogen.

Although an inflammatory response is essential to clear pathogens from the site of infection, a prolonged or overactive inflammatory response can be damaging to the surrounding tissues. For example, inflammation causes the blood vessels at the infected site to dilate to increase blood flow to the site. As a result, these dilated vessels become leaky. After prolonged inflammation, the leaky vessels can produce serious edema in, and impair the proper functioning of, the surrounding tissues (see; e.g., V.W.M. van Hinsbergh, Arteriosclerosis, Thrombosis, and Vascular Biology, Vol. 17, 1018 (1997)). In addition, a continued dominating presence of macrophages at the injured site continues the production of toxins (such as reactive oxygen species) and matrix-degrading enzymes (such as matrix metalloproteinases) by these cells, which are injurious to both the pathogen and the host's tissues. Therefore, a prolonged or overactive inflammation should be controlled to limit the unintended damages to the body and to hasten the body's recovery process.

Glucocorticoids (also referred to herein as "corticosteroids") represent one of the most effective clinical treatment for a range of inflammatory conditions, including acute inflammation. However, steroidal drugs can have side effects that threaten the overall health of the patient.

It is known that certain glucocorticoids have a greater potential for elevating intraocular pressure ("IOP") than other compounds in this class. For example, it is known that prednisolone, which is a very potent ocular anti-inflammatory agent, has a greater tendency to elevate IOP than fluorometholone, which has moderate ocular anti-inflammatory activity. It is also known that the risk of IOP elevations associated with the topical ophthalmic use of glucocorticoids increases over time. In other words, the long-term use of these agents to treat or control persistent ocular conditions increases the risk of significant IOP elevations. In addition, use of corticosteroids is also known to increase the risk of cataract formation in a dose- and duration-dependent manner. Once cataracts develop, they may progress despite discontinuation of corticosteroid therapy.

Chronic administration of glucocorticoids also can lead to drug-induced osteoporosis by suppressing intestinal calcium absorption and inhibiting bone formation. Other adverse side effects of chronic administration of glucocorticoids include hypertension, hyperglycemia, hyperlipidemia (increased levels of triglycerides) and hypercholesterolemia (increased levels of cholesterol) because of the effects of these drugs on the body metabolic processes.

Therefore, there is a continued need to provide improved pharmaceutical compounds and compositions to treat or control inflammatory diseases, conditions, or disorders of the anterior segment. It is also desirable to improved compounds and compositions that cause a lower level of at least an adverse side effect than a composition comprising at least a prior-art glucocorticoid used to treat or control the same diseases, conditions, or disorders.

SUMMARY OF THE INVENTION

As used herein, the term "control" also includes reduction, alleviation, amelioration, and prevention.

In general, the present invention provides compounds or compositions for use in treating or controlling ophthalmic inflammatory diseases, conditions, or disorders (e.g., of the anterior segment of the eye (hereinafter "anterior segment")) in a subject. Such inflammatory diseases, conditions, or disorders have etiology in, or produce, inflammation. More precisely, the present invention provides a composition for use in treating or controlling post-operative ocular inflammation of the anterior segment of an eye, said inflammation resulting from a procedure selected from the groups consisting of photorefractive keratectomy cataract removal surgery, intraocular lens ("IOL") implantation, laser-assisted in situ keratomileusis ("LASIK"), conductive keratoplasty, and radial keratotomy said composition comprising a dissociated glucocorticoid receptor agonist ("DIGRA"). or a pharmaceutically acceptable salt thereof, wherein the DIGRA comprises a compound having Formula I

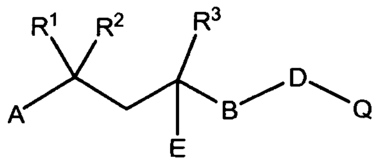

(I)

wherein A comprises a dihydrobenzofuranyl group substituted with a halogen atom: Q comprises a quinolinyl or isoguinolinyl group substituted with a $C_1$-$C_{10}$ alkyl group; $R^1$ and $R^2$ are independently selected from the group consisting of unsubstituted and substituted $C_1$-$C_5$ alkyl groups; B is a $C_1$-$C_3$ alkylene group: D is the -NH- group; E is the hydroxy group; and $R^3$ comprises a completely halogenated $C_1$-$C_{10}$ alkyl group.

In one aspect, the compounds and compositions of the present invention cause a lower level of at least an adverse side effect than a composition comprising at least a prior-art glucocorticoid used to treat or control the same diseases, conditions, or disorders.

The inflammatory diseases, conditions, or disorders of the anterior segment are post-operative (or post-surgical) ocular inflammations resulting from procedures selected from thr group consisting of photorefractive keratectomy, cataract removal surgery, intraocular lens ("IOL") implantation, laser-assisted in situ keratomileusis ("LASIK"), conductive keratoplasty, and radial keratotomy.

The pharmaceutical compositions of the present invention may comprises an ophthalmic topical formulation; injectable formulation; or implantable formulation, system, or device.

Such a formulation, system, or device may be applied or provided to the anterior segment of the eye.

Said at least an adverse side effect may be demonstrated in vitro or in vivo.

In another aspect a method for manufacturing a composition for treating or controlling post-operative ocular inflammation of the anterior segment of an eye is provided, said inflammation resulting from a procedure selected from the group consisting of photorefractive keratectomy, cataract removal surgery, intraocular lens ("IOL") implantation, laser-assisted in situ keratomileusis ("LASIK"), conductive keratoplasty, and radial keratotomy; the method comprising:

(a) providing a DIGRA, or a pharmaceutically acceptable salt thereof; and

(b) combining said DIGRA, or pharmaceutically acceptable salt thereof with a pharmaceutically acceptable carrier;

wherein the DIGRA comprises a compound having Formula I

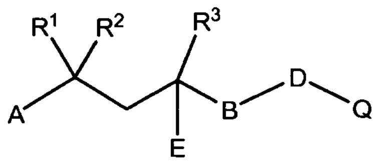

(I)

wherein A comprises a dihydrobenzofuranyl group substituted with a halogen atom; Q comprises a quinolinyl or isoquinolinyl group substituted with a $C_1$-$C_{10}$ alkyl group: $R^1$ and $R^2$ are independently selected from the group consisting of unsubstituted and substituted $C_1$-$C_5$ alkyl groups; B is a $C_1$-$C_3$ alkylene group; D is the NH- group; E is the hydroxy group: and $R^3$ comprises a completely halogenated $C_1$-$C_{10}$ alkyl group.

Other features and advantages of the present invention will become apparent from the following detailed description and claims.

BRIEF DESCRIPTION OF THE DRAWINGS

Figures 1A-1F show the effects of BOL-303242-X and dexamethasone on the IL-1β-stimulated production of Il-6, IL-7, TGF-α, TNF-α, VGEF, and MCP-1 in human corneal epithelium cells ("HCECs") at $p < 0.05$.

Figure 2 shows the effects of BOL-303242-X and dexamethasone on the IL-1β-stimulated production of G-CSF in HCECs at $p < 0.05$.

Figures 3A-1C show the effects of BOL-303242-X and dexamethasone on the IL-1β-stimulated production of GM-CSF, IL-8, and RANTES in HCECs at $p < 0.05$.

In these figures, "*" denotes comparison to control, and "**" to IL-1β.

DETAILED DESCRIPTION OF THE INVENTION

As used herein, a dissociated glucocorticoid receptor agonist ("DIGRA") is a compound that is capable of binding to the glucocorticoid receptor (which is a polypeptide) and, upon binding, is capable of producing differentiated levels of transrepression and transactivation of gene expression. A compound that binds to a polypeptide is sometimes herein referred to as a ligand.

As used herein, the term "alkyl" or "alkyl group" means a linear- or branched-chain saturated aliphatic hydrocarbon monovalent group, which may be unsubstituted or substituted. The group may be partially or completely substituted with halogen atoms (F, Cl, Br, or I). Non-limiting examples of alkyl groups include methyl, ethyl, n-propyl, 1-methylethyl (isopropyl), n-butyl, n-pentyl, 1,1-dimethylethyl (t-butyl), and the like. It may be abbreviated as "Alk".

As used herein, the term "alkenyl" or "alkenyl group" means a linear- or branched-chain aliphatic hydrocarbon monovalent radical containing at least one carbon-carbon double bond. This term is exemplified by groups such as ethenyl, propenyl, n-butenyl, isobutenyl, 3-methylbut-2-enyl, n-pentenyl, heptenyl, octenyl, decenyl, and the like.

As used herein, the term "alkynyl" or "alkynyl, group" means a linear- or branched-chain aliphatic hydrocarbon monovalent radical containing at least one carbon-carbon triple bond. This term is exemplified by groups such as ethynyl, propynyl, n-butynyl, 2-butynyl, 3-methylbutynyl, n-pentynyl, heptynyl, octynyl, decynyl, and the like.

As used herein, the term "alkylene" or "alkylene group" means a linear- or branched-chain saturated aliphatic hydrocarbon divalent radical having the specified number of carbon atoms. This term is exemplified by groups such as methylene, ethylene, propylene, n-butylene, and the like, and may alternatively and equivalently be denoted herein as "-(alkyl)-".

The term "alkenylene" or "alkenylene group" means a linear- or branched-chain aliphatic hydrocarbon divalent radical having the specified number of carbon atoms and at least one carbon-carbon double bond. This term is exemplified by groups such as ethenylene, propenylene, n-butenylene, and the like, and may alternatively and equivalently be denoted herein as "-(alkylenyl)-".

The term "alkynylene" or "alkynylene group" means a linear- or branched-chain aliphatic hydrocarbon divalent radical containing at least one carbon-carbon triple bond. This term is exemplified by groups such as ethynylene, propynylene, n-butynylene, 2-butynylene, 3-methylbutynylene, n-pentynylene, heptynylene, octynylene, decynylene, and the like, and may alternatively and equivalently be denoted herein as "-(alkynyl)-".

As used herein, the term "aryl" or "aryl group" means an aromatic carbocyclic monovalent or divalent radical of from 5 to 14 carbon atoms having a single ring (e.g., phenyl or phenylene), multiple condensed rings (e.g., naphthyl or anthranyl), or multiple bridged rings (e.g., biphenyl). Unless otherwise specified, the aryl ring may be attached at any suitable carbon atom which results in a stable structure and, if substituted, may be substituted at any suitable carbon atom which results in a stable structure. Non-limiting examples of aryl groups include phenyl, naphthyl, anthryl, phenanthryl, indanyl, indenyl, biphenyl, and the like. It may be abbreviated as "Ar".

The term "heteroaryl" or "heteroaryl group" means a stable aromatic 5- to 14-membered, monocyclic or polycyclic monovalent or divalent radical, which may comprise one or more fused or bridged ring(s), preferably a 5- to 7-membered monocyclic or 7- to 10-membered bicyclic radical, having from one to four heteroatoms in the ring(s) independently selected from nitrogen, oxygen, and sulfur, wherein any sulfur heteroatoms may optionally be oxidized and any nitrogen heteroatom may optionally be oxidized or be quaternized. Unless otherwise specified, the heteroaryl ring may be attached at any suitable heteroatom or carbon atom which results in a stable structure and, if substituted, may be substituted at any suitable heteroatom or carbon atom which results in a stable structure. Non-limiting examples of heteroaryls include

furanyl, thienyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridinyl, pyridazinyl, pyrimidinyl, pyrazinyl, triazinyl, indolizinyl, azaindolizinyl, indolyl, azaindolyl, diazaindolyl, dihydroindolyl, dihydroazaindoyl, isoindolyl, azaisoindolyl, benzofuranyl, furanopyridinyl, furanopyrimidinyl, furanopyrazinyl, furanopyridazinyl, dihydrobenzofuranyl, dihydrofuranopyridinyl, dihydrofuranopyrimidinyl, benzothienyl, thienopyridinyl, thienopyrimidinyl, thienopyrazinyl, thienopyridazinyl, dihydrobenzothienyl, dihydrothienopyridinyl, dihydrothienopyrimidinyl, indazolyl, azaindazolyl, diazaindazolyl, benzimidazolyl, imidazopyridinyl, benzthiazolyl, thiazolopyridinyl, thiazolopyrimidinyl, benzoxazolyl, benzoxazinyl, benzoxazinonyl, oxazolopyridinyl, oxazolopyrimidinyl, benzisoxazolyl, purinyl, chromanyl, azachromanyl, quinolizinyl, quinolinyl, dihydroquinolinyl, tetrahydroquinolinyl, isoquinolinyl, dihydroisoquinolinyl, tetrahydroisoquinolinyl, cinnolinyl, azacinnolinyl, phthalazinyl, azaphthalazinyl, quinazolinyl, azaquinazolinyl, quinoxalinyl, azaquinoxalinyl, naphthyridinyl, dihydronaphthyridinyl, tetrahydronaphthyridinyl, pteridinyl, carbazolyl, acridinyl, phenazinyl, phenothiazinyl, and phenoxazinyl, and the like.

The term "heterocycle", "heterocycle group", "heterocyclyl", "heterocyclyl group", "heterocyclic", or "heterocyclic group" means a stable non-aromatic 5- to 14-membered monocyclic or polycyclic, monovalent or divalent, ring which may comprise one or more fused or bridged ring(s), preferably a 5- to 7-membered monocyclic or 7- to 10-membered bicyclic ring, having from one to three heteroatoms in at least one ring independently selected from nitrogen, oxygen, and sulfur, wherein any sulfur heteroatoms may optionally be oxidized and any nitrogen heteroatom may optionally be oxidized or be quaternized. As used herein, a heterocyclyl group excludes heterocycloalkyl, heterocycloalkenyl, and heterocycloalkynyl groups. Unless otherwise specified, the heterocyclyl ring may be attached at any suitable heteroatom or carbon atom which results in a stable structure and, if substituted, may be substituted at any suitable heteroatom or carbon atom which results in a stable structure. Non-limiting examples of heterocycles include pyrrolinyl, pyrrolidinyl, pyrazolinyl, pyrazolidinyl, piperidinyl, morpholinyl, thiomorpholinyl, piperazinyl, tetrahydropyranyl, tetrahydrothiopyranyl, tetrahydrofuranyl, hexahydropyrimidinyl, hexahydropyridazinyl, and the like.

The term "cycloalkyl" or "cycloalkyl group" means a stable aliphatic saturated 3- to 15-membered monocyclic or polycyclic monovalent radical consisting solely of carbon and hydrogen atoms which may comprise one or more fused or bridged ring(s), preferably a 5- to 7-membered monocyclic or 7- to 10-membered bicyclic ring. Unless otherwise specified, the cycloalkyl ring may be attached at any carbon atom which results in a stable structure and, if substituted, may be substituted at any suitable carbon atom which results in a stable structure. Exemplary cycloalkyl groups include cyclopropyl, cyclobutyl, cyclopentyl, cyclohexyl, cycloheptyl, cyclooctyl, cyclononyl, cyclodecyl, norbornyl, adamantyl, tetrahydronaphthyl (tetralin), 1-decalinyl, bicyclo[2.2.2]octanyl, 1-methylcyclopropyl, 2-methylcyclopentyl, 2-methylcyclooctyl, and the like.

The term "cycloalkenyl" or "cycloalkenyl group" means a stable aliphatic 5-to 15-membered monocyclic or polycyclic monovalent radical having at least one carbon-carbon double bond and consisting solely of carbon and hydrogen atoms which may comprise one or more fused or bridged ring(s), preferably a 5- to 7-membered monocyclic or 7- to 10-membered bicyclic ring. Unless otherwise specified, the cycloalkenyl ring may be attached at any carbon atom which results in a stable structure and, if substituted, may be substituted at any suitable carbon atom which results in a stable structure. Exemplary cycloalkenyl groups include cyclopentenyl, cyclohexenyl, cycloheptenyl, cyclooctenyl, cyclononenyl, cyclodecenyl, norbornenyl, 2-methylcyclopentenyl, 2-methylcyclooctenyl, and the like.

The term "cycloalkynyl" or "cycloalkynyl group" means a stable aliphatic 8-to 15-membered monocyclic or polycyclic monovalent radical having at least one carbon-carbon triple bond and consisting solely of carbon and hydrogen atoms which may comprise one or more fused or bridged ring(s), preferably a 8- to 10-membered monocyclic or 12- to 15-membered bicyclic ring. Unless otherwise specified, the cycloalkynyl ring may be attached at any carbon atom which results in a stable structure and, if substituted, may be substituted at any suitable carbon atom which results in a stable structure. Exemplary cycloalkynyl groups include cyclooctynyl, cyclononynyl, cyclodecynyl, 2-methylcyclooctynyl, and the like.

The term "carbocycle" or "carbocyclic group" means a stable aliphatic 3- to 15-membered monocyclic or polycyclic monovalent or divalent radical consisting solely of carbon and hydrogen atoms which may comprise one or more fused or bridged rings, preferably a 5- to 7-membered monocyclic or 7- to 10-membered bicyclic ring. Unless otherwise specified, the carbocycle may be attached at any carbon atom which results in a stable structure and, if substituted, may be substituted at any suitable carbon atom which results in a stable structure. The term comprises cycloalkyl (including spiro cycloalkyl), cycloalkylene, cycloalkenyl, cycloalkenylene, cycloalkynyl, and cycloalkynylene, and the like.

The terms "heterocycloalkyl", "heterocycloalkenyl", and "heterocycloalkynyl" mean cycloalkyl, cycloalkenyl, and cycloalkynyl group, respectively, having at least a heteroatom in at least one ring, respectively.

Glucocorticoids ("GCs") are among the most potent drugs used for the treatment of allergic and chronic inflammatory diseases or of inflammation resulting from infections. However, as mentioned above, long-term treatment with GCs is often associated with numerous adverse side effects, such as diabetes, osteoporosis, hypertension, glaucoma, or cataract. These side effects, like other physiological manifestations, are results of aberrant expression of genes responsible for such diseases. Research in the last decade has provided important insights into the molecular basis of GC-mediated actions on the expression of GC-responsive genes. GCs exert most of their genomic effects by binding

to the cytoplasmic GC receptor ("GR"). The binding of GC to GR induces the translocation of the GC-GR complex to the cell nucleus where it modulates gene transcription either by a positive (transactivation) or negative (transrepression) mode of regulation. There has been growing evidence that both beneficial and undesirable effects of GC treatment are the results of undifferentiated levels of expression of these two mechanisms; in other words, they proceed at similar levels of effectiveness. Although it has not yet been possible to ascertain the most critical aspects of action of GCs in chronic inflammatory diseases, there has been evidence that it is likely that the inhibitory effects of GCs on cytokine synthesis are of particular importance. GCs inhibit the transcription, through the transrepression mechanism, of several cytokines that are relevant in inflammatory diseases, including IL-1β (interleukin-1β), IL-2, IL-3, IL-6, IL-11, TNF-α (tumor necrosis factor-α), GM-CSF (granulocyte-macrophage colony-stimulating factor), and chemokines that attract inflammatory cells to the site of inflammation, including IL-8, RANTES, MCP-1 (monocyte chemotactic protein-1), MCP-3, MCP-4, MIP-1α (macrophage-inflammatory protein-1α), and eotaxin. P.J. Barnes, Clin. Sci., Vol. 94, 557-572 (1998). On the other hand, there is persuasive evidence that the synthesis of IκB kinases, which are proteins having inhibitory effects on the NF-κB proinflammatory transcription factors, is increased by GCs. These proinflammatory transcription factors regulate the expression of genes that code for many inflammatory proteins, such as cytokines, inflammatory enzymes, adhesion molecules, and inflammatory receptors. S. Wissink et al., Mol. Endocrinol., Vol. 12, No. 3, 354-363 (1998); P.J. Barnes and M. Karin, New Engl. J. Med., Vol. 336, 1066-1077 (1997). Thus, both the transrepression and transactivation functions of GCs directed to different genes produce the beneficial effect of inflammatory inhibition. On the other hand, steroid-induced diabetes and glaucoma appear to be produced by the transactivation action of GCs on genes responsible for these diseases. H. Schäcke et al., Pharmacol. Ther., Vol. 96, 23-43 (2002). Thus, while the transactivation of certain genes by GCs produces beneficial effects, the transactivation of other genes by the same GCs can produce undesired side effects. Therefore, it is very desirable to provide pharmaceutical compounds and compositions that produce differentiated levels of transactivation and transrepression activity on GC-responsive genes to treat or control inflammatory diseases, conditions, or disorders, especially chronic inflammation.

In general, the present invention provides compounds or compositions for use in treating or controlling ophthalmic inflammatory diseases, conditions, or disorders

(e.g., of the anterior segment) in a subject. Such inflammatory diseases, conditions, or disorders have etiology in, or produce, inflammation.

In one aspect, the compounds and compositions of the present invention cause a lower level of at least an adverse side effect than a composition comprising at least a prior-art glucocorticoid used to treat or control the same diseases, conditions, or disorders.

Ocular inflammatory pathways commence with the triggering of the arachidonic acid cascade. This cascade is triggered either by mechanical stimuli (such as the case of unavoidable surgically-inflicted trauma) or by chemical stimuli (such as foreign substances (e.g., components of disintegrated pathogenic microorganisms) or allergens). Prostaglandins are generated in most tissues by activation of the arachidonic acid pathway. Phospholipids in the damaged cell membrane are the substrate for phospholipase A to generate arachidonic acid and, in turn, the cyclooxygenase ("COX") and lipoxygenase enzymes act on arachidonic acid to produce a family of pro-inflammatory prostaglandins, thromboxanes, and leukotrienes. These pro-inflammatory compounds recruit more immune cells (such as macrophages and neutrophils) to the site of injury, which then produce a greater amount of other pro-inflammatory cytokines and can further amplify the inflammation.

Cataract surgery with intraocular lens ("IOL") implantation and glaucoma filtering microsurgery (trabeculectomy) are among the common ophthalmic surgical operations. These procedures are usually associated with some post-operative inflammation. The use of anti-inflammatory agents post-operatively can rapidly resolve this event to relieve the patient from pain, discomfort, visual impairment, and to reduce the risk of further complications (such as the onset of cystoid macular edema).

In another aspect, such inflammatory diseases, conditions, or disorders of the anterior segment result from the physical trauma of ocular surgery.

Said post-operative (or post-surgical) ocular inflammation resulting from procedures such as photorefractive keratectomy, cataract removal surgery, intraocular lens ("IOL") implantation, laser-assisted in situ keratomileusis ("LASIK"), conductive keratoplasty, and radial keratotomy.

In yet another aspect, the compositions comprise at least a mimetic of a glucocorticoid for treating or controlling such diseases, conditions, or disorders.

In still another aspect, a level of said at least an adverse side effect is determined in vivo or in vitro. For example, a level of said at least an adverse side effect is determined in vitro by performing a cell culture and determining the level of a biomarker associated with said side effect. Such biomarkers can include proteins (e.g., enzymes), lipids, sugars, and derivatives thereof that participate in, or are the products of, the biochemical cascade resulting in the adverse side effect. Representative in vitro testing methods are further disclosed hereinbelow.

In yet another embodiment, a level of said at least an adverse side effect is determined at about one day after said composition is first administered to, and are present in, said subject. In another embodiment, a level of said at least

an adverse side effect is determined about 14 days after said composition is first administered to, and are present in, said subject. In still another embodiment, a level of said at least an adverse side effect is determined about 30 days after said composition is first administered to, and are present in, said subject. Alternatively, a level of said at least an adverse side effect is determined about 2, 3, 4, 5, or 6 months after said compounds or compositions are first administered to, and are present in, said subject.

In another aspect, said at least a prior-art glucocorticoid used to treat or control the same diseases, conditions, or disorders is administered to said subject at a dose and a frequency sufficient to produce an equivalent beneficial effect on said condition to a composition of the present invention after about the same elapsed time.

In still another aspect, said at least a prior-art glucocorticoid is selected from the group consisting of21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortarnate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, their physiologically acceptable salts, combinations thereof, and mixtures thereof. In one embodiment, said at least a prior-art glucocorticoid is selected from the group consisting of dexamethasone, prednisone, prednisolone, methylprednisolone, medrysone, triamcinolone, loteprednol etabonate, physiologically acceptable salts thereof, combinations thereof, and mixtures thereof. In another embodiment, said at least a prior-art glucocorticoid is acceptable for ophthalmic uses.

In one aspect, the compositions comprise at least a mimetic of a glucocorticoid in treating or controlling such diseases, conditions, or disorders.

In another aspect, the compositions comprise at least a dissociated glucocorticoid receptor agonist ("DIGRA,"). As used herein, a DIGRA can comprise any enantiomer of the molecule or a racemic mixture of the enantiomers.

In still another aspect, the compositions comprise a pharmaceutically acceptable salt of at least a DIGRA.

In still another aspect, the compositions comprise: (a) a DIGRA, or a pharmaceutically acceptable salt thereof; and (b) an anti-inflammatory agent other than said DIGRA, prodrug thereof, and salt thereof. Non-limiting examples of such anti-inflammatory agents are disclosed herein below.

In still another aspect, the compositions comprise: (a) a DIGRA, or a pharmaceutically acceptable salt thereof; and (b) an anti-infective agent. Non-limiting examples of anti-infective agents are disclosed herein below.

In still another aspect, the compositions comprise: (a) a DIGRA, or a pharmaceutically acceptable salt thereof; and (b) a material selected from the group consisting of: (1) an anti-inflammatory agent other than said DIGRA, prodrug thereof, and salt thereof; (2) an anti-infective agent; and (3) combinations thereof.

In still another aspect, said at least a DIGRA has Formula I.

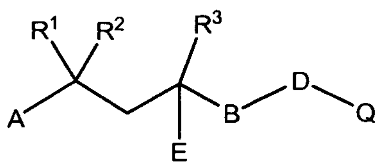

(I)

<u>wherein</u> A comprises a dihydrobenzofuranyl group substituted with a fluorine atom; Q comprises a quinolinyl or isoquinolinyl group substituted with a methyl group; $R^1$ and $R^2$ are independently selected from the group consisting of unsubstituted and substituted $C_1$-$C_5$ alkyl groups; B is a $C_1$-$C_3$ alkylene group; D is the -NH- group; E is the hydroxy group; and $R^3$ comprises a trifluoromethyl group.

In a further embodiment, said at least a DIGRA has Formula II or III.

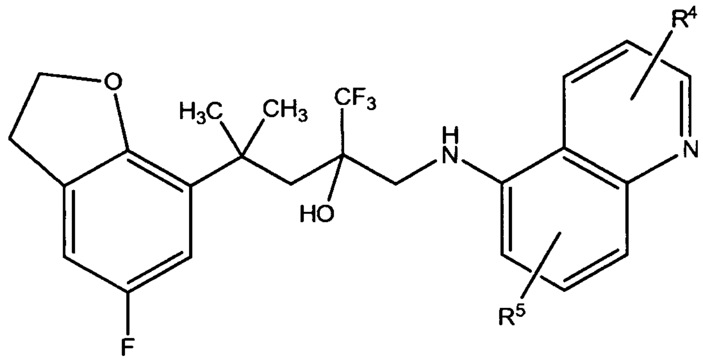

(II)

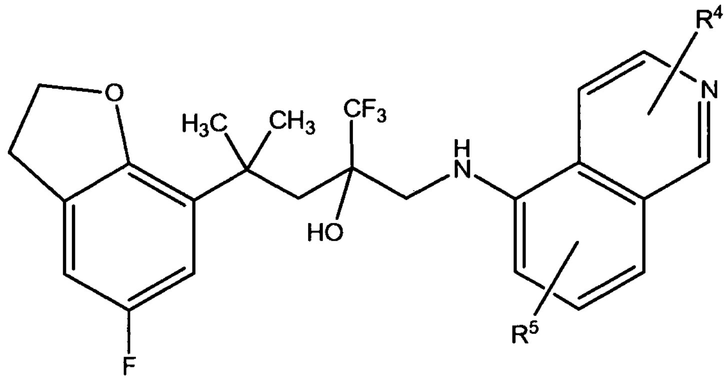

(III)

wherein $R^4$ and $R^5$ are independently selected from the group consisting of unsubstituted $C_1$-$C_{10}$ (alternatively, $C_1$-$C_5$ or $C_1$-$C_3$) linear or branched alkyl groups, substituted $C_1$-$C_{10}$ (alternatively, $C_1$-$C_5$ or $C_1$-$C_3$) linear or branched alkyl groups, unsubstituted $C_3$-$C_{10}$ (alternatively, $C_3$-$C_6$ or $C_3$-$C_5$) cyclic alkyl groups, and substituted $C_3$-$C_{10}$ (alternatively, $C_3$-$C_6$ or $C_3$-$C_5$) cyclic alkyl groups.

In still another embodiment, said at least a DIGRA has Formula IV.

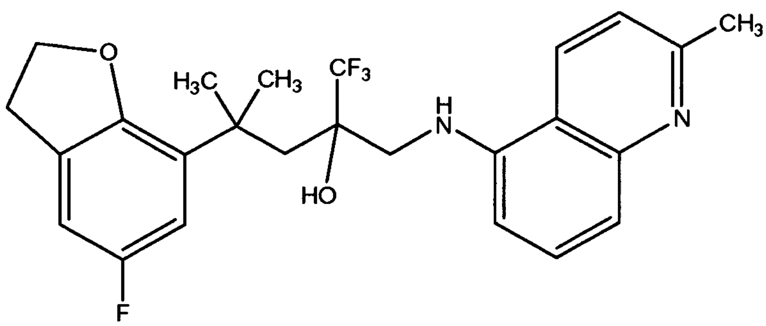

(IV)

Methods for preparing compounds of Formula I, II, III, or IV are disclosed, for example, in U.S. Patents 6,897,224; 6,903,215; 6,960,581. Still other methods for preparing such compounds also can be found in U.S. Patent Application Publication 2006/0116396, or PCT Patent Application WO 2006/050998 A1.

Non-limiting examples of compounds having Formula I include 5-[4-(5-fluoro-2,3-dihydrobenzofuran-7-yl)-2-hydroxy-4-methyl-2-trifluoromethyl-pentylamino]-2-methylquinoline, 5-[4-(5-fluoro-2,3-dihydrobenzofuran-7-yl)-2-hydroxy-4-methyl-2-trifluoromethyl-pentylamino]-1-methylisoquinoline, 5-[4-(5-fluoro-2,3-dihydrobenzofuran-7-yl)-2-hydroxy-4-methyl-2-trifluoromethyl-pentylamino]isoquinol-1(2H)-one, 5-[4-(5-fluoro-2,3-dihydrobenzofuran-7-yl)-2-hydroxy-4-methyl-2-trifluoromethyl-pentylamino]-2,6-dimethylquinoline, 5-[4-(5-fluoro-2,3-dihydrobenzofuran-7-yl)-2-hydroxy-4-methyl-2-trifluoromethyl-pentylamino]-6-chloro-2-methylquinoline, 5-[4-(5-fluoro-2,3-dihydrobenzofuran-7-yl)-2-hydroxy-4-methyl-2-trifluoromethyl-pentylamino]isoquinoline, 5-[4-(5-fluoro-2,3-dihydrobenzofuran-7-yl)-2-hydroxy-4-methyl-2-trifluoromethyl-pentylamino]quinoline, 5-[4-(2,3-dihydro-5-fluoro-7-benzofuranyl)-2-hydroxy-4-methyl-2-trifluoromethyl-pentylamino]quinolin-2[1H]-one, 6-fluro-5-[4-(5-fluoro-2,3-dihydrobenzofuran-7-yl)-2-hydroxy-4-methyl-2-trifluoromethyl-pentylamino]-2-methylquinoline, 8-fluoro-,5-[4-(5-fluoro-2,3-dihydrobenzofuran-7-yl)-2-hydroxy-4-methyl-2-trifluoromethyl-pentylamino]-2-methylquinoline, 5-[4-(5-fluoro-2,3-dihydrobenzofuran-7-yl)-2-hydroxy-4-methyl-2-trifluoromethyl-pentylamino]-2-methylisoquinol-1-[2h]-one, and enantiomers thereof.

A DIGRA can have Formula I, wherein

(a) A is an aryl group optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl;

(c) $R^3$ is the trifluoromethyl group;

(d) B is $C_1$-$C_5$ alkyl, $C_2$-$C_5$ alkenyl, or $C_2$-$C_5$ alkynyl, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q is an azaindolyl group optionally independently substituted with one to three substituent groups, wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaminosulfonyl, halogen, hydroxy, carboxy, cyano, trifluoromethyl, trifluoromethoxy, trifluoromethylthio, nitro, or amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, halogen, hydroxy, oxo, cyano, amino, and trifluoromethyl.

Examples of these compounds include 1,1,1-trifluoro-4-(5-fluoro-2-methoxyphenyl)-4-methyl-2-(1H-pyrrolo [2,3-c] pyridin-2-ylmethyl)pentan-2-ol; 1,1,1-trifluoro-4-(5-fluoro-2-methoxyphenyl)-4-methyl-2-(1H-pyrrolo[3,2-c] pyridin-2-ylmethyl)pentan-2-ol; 1,1,1-trifluoro-4-methyl-4-phenyl-2-(1H-pyrrolo[2,3-c]pyridin-2-ylmethyl)pentan-2-ol; 1,1,1-trifluoro-4-(4-fluoro-2-methoxyphenyl)-4-methyl-2-(1H-pyrrolo[2,3-c] pyridin-2-ylmethyl)pentan-2-ol; 1,1,1-trifluoro-4-methyl-4-phenyl-2-(1H-pyrrolo[3,2-c]pyridin-2-ylmethyl)pentan-2-ol; 1,1,1-trifluoro-4-(4-fluoro-2-methoxyphenyl)-4-methyl-2-(1H-pyrrolo[3,2-c] pyridin-2-ylmethyl)pentan-2-ol; 5-fluoro-2-[4,4,4-trifluoro-3-hydroxy-1,1-dimethyl-3-(1H-pyrrolo [2,3-c]pyridin-2-ylmethyl)butyl]phenol; 4-fluoro-2-[4,4,4-trifluoro-3-hydroxy-1,1-dimethyl-3-(1H-pyrrolo[2,3-c]pyridin-2-ylmethyl)butyl]phenol; 1,1,1-trifluoro-4-(5-fluoro-2-methoxyphenyl)-4-methyl-2-(1H-pyrrolo[3,2-c] pyridin-2-ylmethyl) pentan-2-ol; 1,1,1-trifluoro-4-(5-fluoro-2-methoxyphenyl)-4-methyl-2-(3-methyl-1H-pyrrolo[2,3-c]pyridin-2-ylmethyl) pentan-2-ol; and 4-fluoro-2-[4,4,4-trifluoro-3-hydroxy-1,1-dimethyl-3-(1H-pyrrolo[2,3-c]pyridin-2-ylmethyl)butyl]phenol.

For example, a DIGRA can have Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently

substituted with $C_1$-$C_5$ alkyl, $C_1$-$C_5$ alkylthio wherein the sulfur atom is optionally oxidized to a sulfoxide or sulfone;

(b) R' and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) B is the methylene or carbonyl group;

(d) $R^3$ is a carbocycle, heterocyclyl, aryl, heteroaryl, carbocycle-$C_1$-$C_8$ alkyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups;

(e) D is the -NH- group;

(f) E is the hydroxy group; and

(g) Q comprises a methylated benzoxazinone.

Examples of these compounds include 2-benzyl-4-(5-fluoro-2-methoxyphenyl)-2-hydroxy-4-methylpentanoic acid(4-methyl-1-oxo-1H-benzo[d][1,2]oxazin-6-yl)amide; 2-benzyl-4-(5-fluoro-2-hydroxyphenyl)-2-hydroxy-4-methylpentanoic acid(4-methyl-1-oxo-1H-benzo[d][1,2]oxazin-6-yl)amide; 2-cyclohexylmethyl-4-(5-fluoro-2-methoxyphenyl)-2-hydroxy-4-methylpentanoic acid(4-methyl-1-oxo-1H-benzo[d][1,2]oxazin-6-yl)amide; 2-cyclohexylmethyl-4-(5-fluoro-2-hydroxyphenyl)-2-hydroxy-4-methylpentanoic acid(4-methyl-1-oxo-1H-benzo[d][1,2]oxazin-6-yl)amide; 2-benzyl-2-hydroxy-4-methyl-4-methylpentanoic acid(4-methyl-1-oxo-1H-benzo[d][1,2]oxazin-6-yl)amide; and 2-cyclohexylmethyl-2-hydroxy-4-methylpentanoic acid(4-methyl-1-oxo-1H-benzo[d][1,2]oxazin-6-yl)amide.

For example a DIGRA can have Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) $R^3$ is the trifluoromethyl group;

(d) B is $C_1$-$C_5$ alkyl, $C_2$-$C_5$ alkenyl, or $C_2$-$C_5$ alkynyl, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q is an aryl or heteroaryl group one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein each substituent group of Q is optionally

independently substituted with one to three substituent groups selected from the group consisting of $C_1$-$C_3$ alkyl, $C_1$-$C_3$ alkoxy, acyl, $C_1$-$C_3$ silanyloxy, $C_1$-$C_5$ alkoxycarbonyl, carboxy, halogen, hydroxy, oxo, cyano, heteroaryl, heterocyclyl, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, and trifluoromethyl.

Examples of these compounds include 2-(3,5-difluorobenzyl)-1,1,1-trifluoro-4-(5-fluoro-2-methoxyphenyl)-4-methylpentan-2-ol; 2-biphenyl-4-ylmethyl-1,1,1-trifluoro-4-(5-fluoro-2-methoxyphenyl)-4-methylpentan-2-ol; 2-(3,5-dimethylbenzyl)-1,1,1-trifluoro-4-(5-fluoro-2-methoxyphenyl)-4-methylpentan-2-ol; 2-(3-bromobenzyl)-1,1,1-trifluoro-4-(5-fluoro-2-methoxyphenyl)-4-methylpentan-2-ol; 2-(3,5-dichlorobenzyl)-1,1,1-trifluoro-4-(5-fluoro-2-methoxyphenyl)-4-methylpentan-2-ol; 2-(3,5-bis-trifluoromethylbenzyl)-1,1,1-trifluoro-4-(5-fluoro-2-methoxyphenyl)-4-methylpentan-2-ol; 1,1,1-trifluoro-4-(5-fluoro-2-methoxyphenyl)-2-(3-fluoro-5-trifluoromethylbenzyl)-4-methylpentan-2-ol; 2-(3-chloro-2-fluoro-5-trifluoromethylbenzyl-)-1,1,1-trifluoro-4-(5-fluoro-2-methoxyphenyl)-4-methylpentan-2-ol; 4-[4-(5-fluoro-2-methoxyphenyl)-2-hydroxy-4-methyl-2-trifluoromethylpentyl]benzonitrile; 2-(3,5-dibromobenzyl)-1,1,1-trifluoro-4-(5-fluoro-2-methoxyphenyl)-4-methylpentan-2-ol; 1,1,1-trifluoro-4-(5-fluoro-2-methoxyphenyl)-2-(2-fluoro-3-trifluoromethylbenzyl)-4-methylpentan-2-ol; 1,1,1-trifluoro-4-(5-fluoro-2-methoxyphenyl)-2-(2-fluoro-5-trifluoromethylbenzyl)-4-methylpentan-2-ol.

For example, a DIGRA can have Formula I, wherein

(a) A is an aryl, heteroaryl, or $C_5$-$C_{15}$ cycloalkyl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen, $C_1$-$C_5$ alkyl, $C_5$-$C_{15}$ arylalkyl, or R' and $R^2$ together with the carbon atom they are commonly attached to form a $C_3$-$C_8$ spiro cycloalkyl ring;

(c) $R^3$ is the trifluoromethyl group;

(d) B is the carbonyl group or methylene group, which is optionally independently substituted with one or two substituent groups selected from $C_1$-$C_5$ alkyl, hydroxy, and halogen;

(e) D is absent;

(f) E is the hydroxy group or amino group wherein the nitrogen atom is optionally independently mono- or di-substituted by $C_1$-$C_5$ alkyl; and

(g) Q comprises a pyrrolidine, morpholine, thiomorpholine, piperazine, piperidine, 1H-pyridin-4-one, 1H-pyridin-2-one, 1H-pyridin-4-ylideneamine, 1H-quinolin-4-ylideneamine, pyran, tetrahydropyran, 1,4-diazepane, 2,5-diazabicyclo[2.2.1]heptane, 2,3,4,5-tetrahydrobenzo[b][1,4]diazepine, dihydroquinoline, tetrahydroquinoline, 5,6,7,8-tetrahydro-1H-quinolin-4-one, tetrahydroisoquinoline, decahydroisoquinoline, 2,3-dihydro-1H-isoindole, 2,3-dihydro-1H-indole, chroman, 1,2,3,4-tetrahydroquinoxaline, 1,2-dihydroindazol-3-one, 3,4-dihydro-2H-benzo[1,4]oxazine, 4H-benzo[1,4]thiazine, 3,4-dihydro-2H-benzo[1,4]thiazine, 1,2-dihydrobenzo[d] [1,3]oxazin4-one, 3,4-dihydrobenzo [1,4]oxazin4-one, 3H-quinazolin4-one, 3,4-dihydro-1H-quinoxalin-2-one, 1H-quinnolin-4-one, 1H-quinazolin4-one, 1H-[1,5]naphthyridin-4-one, 5,6,7,8-tetrahydro-1H-[1,- 5]naphthyridin-4-one, 2,3-dihydro-1H-[1,5]naphthyridin-4-one, 1,2-dihydropyrido[3,2-d][1,3]oxazin-4-one, pyrrolo[3,4-c]pyridine-1,3-dione, 1,2-dihydropyrrolo[3,4-c]pyridin-3-one, or tetrahydro[b][1,4]diazepinone group, each optionally independently substituted with one to three substituent groups, wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, oxo, cyano, trifluoromethyl, trifluoromethoxy, trifluoromethylthio, nitro, amino wherein the nitrogen atom is optionally

independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone, wherein each substituent group of Q is optionally independently substituted with one to three substituent groups selected from $C_1$-$C_3$ alkyl, $C_1$-$C_3$ alkoxy, $C_1$-$C_3$ alkoxycarbonyl, acyl, aryl, benzyl, heteroaryl, heterocyclyl, halogen, hydroxy, oxo, cyano, amino wherein the nitrogen atom is optionally independently mono- or di-substituted by $C_1$-$C_5$ alkyl, or ureido wherein either nitrogen atom is optionally independently substituted with $C_1$-$C_5$ alkyl.

Examples of these compounds include 2-(2,6-dimethylmorpholin-4-ylmethyl)-1,1,1-trifluoro-4-(5-fluoro-2-methoxyphenyl)-4-methylpentan-2-ol; 1-[4-(5-fluoro-2-methoxyphenyl)-2-hydroxy-4-methyl-2-trifluoromethylpentyl]-1H-quinolin-4-one; 1-[4-(5-fluoro-2-methoxyphenyl)-2-hydroxy-4-methyl-2-trifluoromethylpentyl]-3,5-dimethylpiperidin-4-one; 1-[4-(5-fluoro-2-methoxyphenyl)-2-hydroxy-4-methyl-2-trifluoromethylpentyl]-3-methyl-1H-quinolin-4-one; 1-[4-(5-fluoro-2-methoxyphenyl)-2-hydroxy-4-methyl-2-trifluoromethylpentyl]-2,3-dihydro-1H-quinolin-4-one; 1-[4-(4-fluorophenyl)-2-hydroxy-4-methyl-2-trifluoromethylpentyl]-1H-quinolin-4-one; 1-[4-(3-fluorophenyl)-2-hydroxy-4-methyl-2-trifluoromethylpentyl]-1H-quinolin-4-one; 1-[4-(4-fluoro-2-hydroxyphenyl)-2-hydroxy-4-methyl-2-trifluoromethylpentyl]-1 H-quinolin-4-one; 1-[4-phenyl-2-hydroxy-4-methyl--2-trifluoromethylpentyl]-1H-quinolin-4-one; 1-[4-(5-fluoro-2,3-dihydrobenzofuran-7-yl)-2-hydroxy-4-methyl-2-trifluoromethylpentyl]-1H-quinolin-4-one; 1-[4-(5-bromo-2,3-dihydrobenzofuran-7-yl)-2-hydroxy-4-methyl-2-trifluoromethylpentyl]-1H-quinolin-4-one; 1-[4-(5-methyl-2,3-dihydrobenzofuran-7-y-1)-2-hydroxy-4-methyl-2-trifluoromethylpentyl]-1H-quinolin-4-one; 1-[4-(5-chloro-2,3-dihydrobenzofuran-7-yl)-2-hydroxy-4-methyl-2-trifluoromethylpentyl]-1H-quinolin-4-one; 1-[4-(2,3-dihydrobenzofuran-7-yl)-2-hydroxy-4-methyl-2-trifluoromethylpentyl]-1H-quinolin-4-one; 1-[4-(5-fluoro-2-hydroxyphenyl)-2-hydroxy-4-methyl-2-trifluoromethylpentyl]-1H-[1,5]naphthyridin-4-one; 1-[4-(5-fluoro-2-methoxyphenyl)-2-hydroxy-2,4-dimethylpentyl]-3,5-dimethyl-1H-pyridin-4-one; 1-[2-hydroxy-4-(2-methoxy-5-thiophen-2-ylphenyl)-4-methyl-2-trifluoromethylpentyl]-1H-quinolin-4-one; 1-[4-(6-bromobenzo [1,3]dioxol-4-yl)-2-hydroxy-4-methyl-2-trifluoromethylpentyl]-1 H-quinolin-4-one; 1-[4-(5-fluoro-2-hydroxyphenyl)-2-hydroxy-4-methyl-2-trifluoromethylpentyl]-3-methyl-1H-quinolin-4-one; 1-[2-hydroxy-4-(4-hydroxybiphenyl-3-yl)-4-methyl-2-trifluoromethylpentyl]-1H-quinolin-4-one; 1-{4-[5-(3,5-dimethylisoxazol-4-yl)-2-hydroxyphenyl]-2-hydroxy-4-methyl-2-trifluoromethylpentyl}-1H-quinolin-4-one; 1-[2-hydroxy-4-(2-hydroxy-5-thiophen-3-ylphenyl)-4-methyl-2-trifluoromethylpentyl]-1H-quinolin-4-one; 1-{4-[5-(3,5-dimethylisoxazol-4-yl)-2-methoxyphenyl]-2-hydroxy-4-methyl-2-trifluoromethylpentyl}-1H-quinolin-4-one; 1-[2-hydroxy-4-methyl-4-(3-pyridin-3-ylphenyl)-2-trifluoromethylpentyl]-1H-quinolin-4-one; 4-methoxy-3-[4,4,4-trifluoro-3-hydroxy-1,1-dimethyl-3-(4-oxo-4H-quinolin-1-ylmethyl)butyl]benzaldehyde; 1-[2-hydroxy-4-(2-methoxy-5-thiophen-3-ylphenyl)-4-methyl-2-trifluoromethylpentyl]-1H-quinolin-4-one; 1-[4-(5-furan-3-yl-2-methoxyphenyl)-2-hydroxy-4-methyl-2-trifluoromethylpentyl]-1H-quinolin-4-one; 1-[2-hydroxy-4-(4-methoxybiphenyl-3-yl)-4-methyl-2-trifluoromethylpentyl]-1H-quinolin-4-one; 1-[4-(5-acetyl-2-hydroxyphenyl)-2-hydroxy-4-methyl-2-trifluoromethylpentyl]-1H-quinolin-4-one; 1-[3,3,3-trifluoro-2-(6-fluoro-4-methylchroman-4-ylmethyl)-2-hydroxypropyl]-1H-quinolin-4-one; 1-(4-{3-[1-(benzyloxyimino)ethyl]phenyl}-2-hydroxy-4-methyl-2-trifluoromethylpentyl)-1H-quinolin-4-one; 1-[4-(5-acetyl-2-methoxyphenyl)-2-hydroxy-4-methyl-2-trifluoromethylpentyl]-1H-quinolin-4-one; 1-(2-hydroxy-4-{3-[1-(methoxyimino)ethyl]phenyl}-4-methyl-2-trifluoromethylpentyl)-1H-quinolin-4-one; 1-[4-(5-bromo-2-hydroxyphenyl)-2-hydroxy-4-methyl-2-trifluoromethylpentyl]-1H-quinolin-4-one; 1-(2-hydroxy-4-{3-[1-(hydroxyimino)ethyl]phenyl}-4-methyl-2-trifluoromethylpentyl)-1H-quinolin-4-one; 1-[4-(5-bromo-2-methoxyphenyl)-2-hydroxy-4-methyl-2-trifluoromethylpentyl]-1H-quinolin-4-one; 1-[4-(3,5-difluorophenyl)-2-hydroxy-4-methyl-2-trifluoromethylpentyl]-1H-quinolin-4-one; 1-[4-(3,5-dimethylphenyl)-2-hydroxy-4-methyl-2-trifluoromethylpentyl]-1H-quinolin-4-one; 1-{2-hydroxy-4-methyl-4-[3-(2-methyl-[1,3]dioxolan-2-yl)phenyl]-2-trifluoromethylpentyl}-1H-quinolin-4-one; 1-[4-(2,3-dihydrobenzofuran-7-yl)-2-hydroxy-4-methyl-2-trifluoromethylpentyl]-1H-[1,5]naphthyridin-4-one; 1-[4-(3-[1,3]dioxan-2-ylphenyl)-2-hydroxy-4-methyl-2-trifluoromethylpentyl]-1H-quinolin-4-one; 1-{4-[3-(3,5-dimethylisoxazol-4-yl)phenyl]-2-hydroxy-4-methyl-2-trifluoromethylpentyl}-1H-quinolin-4-one; 1-[4-(2,3-dihydrobenzofuran-7-yl)-2-hydroxy-4-methyl-2-trifluoromethylpentyl]-3,5-dimethyl-1H-pyridin-4-one; 1-[4-(5-fluoro-2-methoxyphenyl)-2-hydroxy-4-methyl-2-trifluoromethylpentyl]-2-hydroxymethyl-3,5-dimethyl-1H-pyridin-4-one; 1-[4-(5-fluoro-2-hydroxyphenyl)-2-hydroxy-4-methyl-2-trifluoromethylpentyl]-3-hydroxymethyl-1H-quinolin-4-one; 1-[4-(3-bromophenyl)-2-hydroxy-4-methyl-2-trifluoromethylpentyl]-1H-quinolin-4-one; 1-[4-(5-fluoro-2-methoxyphenyl)-2-hydroxy-4-methyl-2-trifluoromethylpentyl]-6-methyl-1H-quinolin-4-one; 6-chloro-1-[4-(5-fluoro-2-hydroxyphenyl)-2-hydroxy-4-methyl-2-trifluoromethylpentyl]-1H-quinolin-4-one; 1-[-4-(2-difluoromethoxy-5-fluorophenyl)-2-hydroxy-4-methyl-2-trifluoromethylpentyl]-1H-quinolin-4-one; 1-(4-biphenyl-3-yl-2-hydroxy-4-methyl-2-trifluoromethylpentyl)-1H-quinolin-4-one; 1-[2-hydroxy-4-(2-hydroxy-5-methylphenyl)-4-methyl-2-trifluoromethylpentyl]-1H-quinolin-4-one; 1-[2-hydroxy-4-(3-isopropoxyphenyl)-4-methyl-2-trifluoromethylpentyl]-1H-quinolin-4-one; 1-[4-(3-ethoxyphenyl)-2-hydroxy-4-methyl-2-trifluoromethylpentyl]-1H-quinolin-4-one; 1-[2-hydroxy-4-(2-methoxy-5-methylphenyl)-4-methyl-2-trifluoromethylpentyl]-1 H-quinolin-4-one; 1-[4-(2,5-dimethylphenyl)-2-hydroxy-4-methyl-2-tnfluoromethylpentyl]-1H-quinolin-4-one; 1-[2-hydroxy-4-(3-methoxyphenyl)-4-methyl-2-trifluoromethylpentyl]-1H-quinolin-4-one; 1-[4-(5-fluoro-2-hydroxyphenyl)-2-hydroxy-4-methyl-2-trifluoromethylpentyl]-1 ,2-dihydroindazol-3-one; 7-fluoro-1-[4-(5-fluoro-2-hydroxyphenyl)-2-hydroxy-4-methyl-2-trifluoromethylpentyl]-1H-quinolin-4-one; 1-[4-(5-fluoro-2-hydroxyphenyl)-2-hydroxy-4-methyl-2-trifluoromethylpentyl]-3,5-dimethyl-1H-pyridin-4-one; 7-fluoro-1-[4-(5-fluoro-2-methoxyphenyl)-2-hydroxy-4-methyl-2-trifluoromethylpentyl]-1H-quinolin-4-one; 1-(2-hydroxy-4-methyl-4-phenyl-2-trifluoromethylhexyl)-1H-quinolin-4-one; 1-[4-(4-fluoro-2-methylphenyl)-2-hydroxy-4-methyl-2-trifluoromethylpentyl]-H-quinolin-4-one; 1-[4-(3,4-dimethylphenyl)-2-hydroxy-4-methyl-2-trifluoromethylpentyl]-1H-quinolin-4-one; 8-fluoro-1-[4-(5-fluoro-2-hydroxyphenyl)-2-hydroxy-4-methyl-2-trifluoromethylpentyl]-1H-quinolin-4-one; 6-fluoro-1-[4-(5-fluoro-2-hydroxyphenyl)-2-hydroxy-4-methyl-2-trifluoromethylpentyl]-1H-quinolin-4-one; 7-chloro-1-[4-(5-fluoro-2-hydroxyphenyl)-2-hydroxy-4-methyl-2-trifluoromethylpentyl]-1H-quinolin-4-one; 1-[4-(5-fluoro-2-isopropoxyphenyl)-2-hydroxy-4-methyl-2-trifluoromethylpentyl]-H-quinolin-4-one; 1-[4-(2-ethoxy-5-fluorophenyl)-2-hydroxy-4-methyl-2-trifluoromethylpentyl]-1H-quinolin-4-one; 8-fluoro-1-[4-(5-fluoro-2-methoxyphenyl)-2-hydroxy-4-methyl-2-trifluoromethylpentyl]-1H-quinolin-4-one; 6-fluoro-1-[4-(5-fluoro-2-methoxyphenyl)-2-hydroxy-4-methyl-2-trifluoromethylpentyl]-1H-quinolin-4-one; 1-[2-hydroxy-4-(5-methanesulfonyl-2,3-dihydrobenzofuran-7-yl)-4-methyl-2-trifluoromethylpentyl]-1H-quinolin-4-one; 1-[2-hydroxy-4-methyl-4-(5-methylsulfanyl-2,3-dihydrobenzofuran-7-yl)-2-trifluoromethylpentyl]-1H-quinolin-4-one; 7-chloro-1-[4-(5-fluoro-2-methoxyphenyl)-2-hydroxy-4-methyl-2-trifluoromethylpentyl]-1H-quinolin-4-one; 3-chloro-1-[4-(5-fluoro-2-methoxyphenyl)-2-hydroxy-4-methyl-2-trifluoromethylpentyl]-5-trifluoromethyl-1H-pyridin-2-one; 1-[2-hydroxy-4-(5-methanesulfonyl-2,3-dihydrobenzofuran-7-yl)-4-methyl-2-trifluoromethylpentyl]-3-methyl-1H-quinolin-4-one; 1-[2-hydroxy-4-(2-methoxy-5-pyridin-3-ylphenyl)-4-methyl-2-trifluoromethylpentyl]-1H-quinolin-4-one; 1-[2-hydroxy-4-(2-hydroxy-3,5-dimethylphenyl)-4-methyl-2-trifluoromethylpentyl]-H-quinolin-4-one; 1-[4-(3-[1,3]dioxan-2-yl-4-fluorophenyl)-2-hydroxy-4-methyl-2-trifluoromethylpentyl]-1H-quinolin-4-one; 2-(1,1-dioxo-2,3-dihydro-1H-1$\lambda^6$-benzo[1,4]thiazin-4-ylmethyl)-1,1,1-trifluoro-4-(5-fluoro-2-methoxyphenyl)-4-methylpentan-2-ol; 2-(2,3-dihydrobenzo[1,4]oxazin4-ylmethyl)-1,1,1-trifluoro-4-(5-fluoro-2-methoxyphenyl)-4-methylpentan-2-ol; 1-[4-(5-fluoro-2-hydroxyphenyl)-2-hydroxy-4-methyl-2-trifluoromethylpentyl]-H-quinolin-4-one; 1-[4-(5-fluoro-2-hydroxyphenyl)-2-hydroxy-4-methyl-2-trifluoromethylpentyl]-H-[1,5]naphthyridin-4-one; 1-[4-(5-fluoro-2-methylphenyl)-2-hydroxy-4-methyl-2-trifluoromethylpentyl]-H-quinolin-4-one; 1-[4-(2,4-dimethylphenyl)-2-hydroxy-4-methyl-2-trifluoromethylpentyl]-1H-quinolin-4-one; 1-[4-(4-fluoro-2-methoxyphenyl)-2-hydroxy-4-methyl-2-trifluoromethylpentyl]-H-quinolin-4-one; 1-[4-(3-fluoro-4-methoxyphenyl)-2-hydroxy-4-methyl-2-trifluoromethylpentyl]-1H-quinolin-4-one; 1-(4-benzo[1,3]dioxol-4-yl-2-hydroxy-4-methyl-2-trifluoromethylpentyl)-1H-quinolin-4-one; 1-[4-(5-fluoro-2-methoxyphenyl)-2-hydroxy-4-methyl-2-trifluoromethylpentyl]-1,2-dihydroindazol-3-one; 1,1,1-trifluoro-4-(5-fluoro-2-methoxyphenyl)-4-methyl-2-(1-oxo-2,3-dihydro-1H-1$\lambda^4$-benzo[1,4-]thiazin-4-ylmethyl)pentan-2-ol; 1-[4-(5-fluoro-2-methoxyphenyl)-2-hydroxy-4-methyl-2-trifluoromethylpentyl]-2-hydroxymethyl-3,5-dimethyl-1H-pyridin--4-one; 1-[4-(2,3-dihydrobenzofuran-7-yl)-2-hydroxy-4-methyl-2-trifluoromethylpentyl]-3-methyl-1H-quinolin-4-one; 1-[2-hydroxy-4-(2-methoxy-3,5-dimethylphenyl)-4-methyl-2-trifluoromethylpentyl]-1H-quinolin-4-one; 1-[2-hydroxy-4-(2-hydroxy-5-pyridin-3-ylphenyl)-4-methyl-2-trifluoromethylpentyl]-1H-quinolin-4-one; and 1-[2-hydroxy-4-(2-hydroxy-5-pyridin-5-ylphenyl)-4-methyl-2-trifluoromethylpentyl]-1H-quinolin-4-one.

For example, a DIGRA has-can have Formula I, wherein A, $R^1$, $R^2$, B, D, E, and Q have the meanings disclosed immediately above, and $R^3$ is hydrogen, $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein $R^3$ cannot be trifluoromethyl.

For example, a DIGRA can have Formula I, wherein

(a) A is an aryl, heteroaryl, or $C_5$-$C_{15}$ cycloalkyl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) $R^3$ is the trifluoromethyl group;

(d) B is the carbonyl group;

(e) D is the -NH- group;

(f) E is the hydroxy group; and

(g) Q comprises an optionally substituted phenyl group having the formula

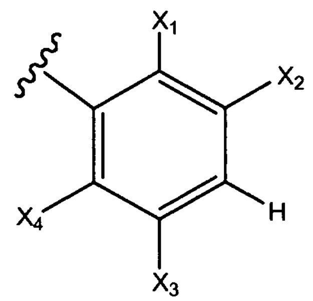

wherein $X_1$, $X_2$, $X_3$ and $X_4$ are each independently selected from the group consisting of hydrogen, halogen, hydroxy, trifluoromethyl, trifluoromethoxy, $C_1$-$C_5$ alkyl, $C_2$-$C_5$ alkenyl, $C_2$-$C_5$ alkynyl, $C_1$-$C_5$ alkoxy, $C_1$-$C_5$ alkylthio wherein the sulfur atom is optionally oxidized to a sulfoxide or sulfone, $C_1$-$C_5$ alkanoyl, $C_1$-$C_5$ alkoxycarbonyl, $C_1$-$C_5$ acyloxy, $C_1$-$C_5$ alkanoylamino, $C_1$-$C_5$ carbamoyloxy, urea, aryl, and amino wherein the nitrogen atom may be independently mono- or di-substituted by $C_1$-$C_5$ alkyl, and wherein said aryl group is optionally substituted by one or more hydroxy or $C_1$-$C_5$ alkoxy groups, and wherein either nitrogen atom of the urea group may be independently substituted by $C_1$-$C_5$ alkyl; or Q is an aromatic 5- to 7-membered monocyclic ring having from one to four heteroatoms in the ring independently selected from nitrogen, oxygen, and sulfur, optionally independently substituted with one to three substituent groups selected from the group consisting of hydrogen, halogen, hydroxy, trifluoromethyl, trifluoromethoxy, $C_1$-$C_5$ alkyl, $C_2$-$C_5$ alkenyl, $C_2$-$C_5$ alkynyl, $C_1$-$C_5$ alkoxy, $C_1$-$C_5$ alkylthio wherein the sulfur atom is optionally oxidized to a sulfoxide or sulfone, $C_1$-$C_5$ alkanoyl, $C_1$-$C_5$ alkoxycarbonyl, $C_1$-$C_5$ acyloxy, $C_1$-$C_5$ alkanoylamino, $C_1$-$C_5$ carbamoyloxy, urea, aryl optionally substituted by one or more hydroxy or $C_1$-$C_5$ alkoxy groups, and amino wherein the nitrogen atom may be independently mono- or di-substituted by $C_1$-$C_5$ alkyl, and wherein either nitrogen atom of the urea group may be independently substituted by $C_1$-$C_5$ alkyl.

Examples of these compounds include 4-(5-fluoro-2-hydroxyphenyl)-2-hydroxy-4-methyl-2-trifluoromethyl-pentanoic acid (3,5-dichloro-phenyl)-amide; 4-(5-fluoro-2-hydroxy-phenyl)-2-hydroxy-4-methyl-2-trifluoromethyl-pentanoic acid (3-chloro-phenyl)-amide; 4-(5-fluoro-2-hydroxy-phenyl)-2-hydroxy-4-methyl-2-trifluoromethyl-pentanoic acid (2-chloro-phenyl)-amide; 4-(5-fluoro-2-hydroxy-phenyl)-2-hydroxy-4-methyl-2-trifluoromethyl-pentanoic acid (2,6-dichloro-pyrimidin-4-yl)-amide; 4-(5-fluoro-2-hydroxy-phenyl)-2-hydroxy-4-methyl-2-trifluoromethyl-pentanoic acid (2,6-dichloro-pyridin-4-yl)-amide; 4-(5-fluoro-2-hydroxy-phenyl)-2-hydroxy-4-methyl-2-trifluoromethyl-pentanoic acid (2,3-dichloro-phenyl)-amide; 4-(5-fluoro-2-hydroxy-phenyl)-2-hydroxy-4-methyl-2-trifluoromethyl-pentanoic acid (3,5-dimethylphenyl)-amide; 4-(5-fluoro-2-hydroxy-phenyl)-2-hydroxy-4-methyl-2-trifluoromethyl-pentanoic acid (3,5-bis-trifluoromethyl-phenyl)-amide; 4-(5-fluoro-2-hydroxy-phenyl)-2-hydroxy-4-methyl-2-trifluoromethyl-pentanoic acid (2,5-dichloro-phenyl)-amide; 4-(5-fluoro-2-hydroxy-phenyl)-2-hydroxy-4-methyl-2-trifluoromethyl-pentanoic acid (3-bromo-phenyl)-amide; 4-(5-fluoro-2-hydroxy-phenyl)-2-hydroxy-4-methyl-2-trifluoromethyl-pentanoic acid (3,5-difluoro-phenyl)-amide; 4-(5-fluoro-2-hydroxyphenyl)-2-hydroxy-4-methyl-2-trifluoromethyl-pentanoic acid (3,5-dibromo-phenyl)-amide.

For example, a DIGRA can have Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino,

$C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl;

(c) $R^3$ is $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone, wherein $R^3$ cannot be trifluoromethyl;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q comprises an azaindolyl group optionally independently substituted with one to three substituent groups, wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaminosulfonyl, halogen, hydroxy, carboxy, cyano, trifluoromethyl, trifluoromethoxy, trifluoromethylthio, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone, wherein each substituent group of Q is optionally independently substituted with one to three substituent groups selected from $C_1$-$C_3$ alkyl, $C_1$-$C_3$ alkoxy, halogen, hydroxy, oxo, cyano, amino, or trifluoromethyl.

Examples of these compounds include 1,1,1-trifluoro-4-(5-fluoro-2-methoxyphenyl)-4-methyl-2-(1H-pyrrolo[2,3-c]pyridin-2-ylmethyl)pentan-2-ol; 1,1,1-trifluoro-4-(5-fluoro-2-methoxyphenyl)-4-methyl-2-(1H-pyrrolo[2,3-b]pyridin-2-ylmethyl)pentan-2-ol; 1,1,1-trifluoro-4-(5-fluoro-2-methoxyphenyl)-4-methyl-2-(1H-pyrrolo[3,2-c]pyridin-2-ylmethyl)pentan-2-ol; 1,1,1-trifluoro-4-(5-fluoro-2-methoxyphenyl)-4-methyl-2-(1H-pyrrolo[3,2-b]pyridin-2-ylmethyl)pentan-2-ol; 4-fluoro-2-[4,4,4-trifluoro-3-hydroxy-1,1-dimethyl-3-(1H-pyrrolo[2,3-c]pyridin-2-ylmethyl)butyl]phenol; 4-fluoro-2-[4,4,4-trifluoro-3-hydroxy-1,1-dimethyl-3-(1H-pyrrolo[2,3-b]pyridin-2-ylmethyl)butyl]phenol; 4-fluoro-2-[4,4,4-trifluoro-3-hydroxy-1,1-dimethyl-3-(1H-pyrrolo[3,2-c]pyridin-2-ylmethyl)butyl]phenol; 4-fluoro-2-[4,4,4-trifluoro-3-hydroxy-1,1-dimethyl-3-(1H-pyrrolo[3,2-b]pyridin-2-ylmethyl)butyl]phenol; 1,1,1-trifluoro-4-(3-fluorophenyl)-4-methyl-2-(1 H-pyrrolo[2,3-c]pyridin-2-ylmethyl)pentan-2-ol; 1,1,1-trifluoro-4-(4-fluorophenyl)-4-methyl-2-(1H-pyrrolo[2,3-c]pyridin-2-ylmethyl)pentan-2-ol; 4-(2,3-dihydrobenzofuran-7-yl)-1,1,1-trifluoro-4-methyl-2-(1H-pyrrolo[2,3-c]pyridin-2-yelmethyl)pentan-2-ol; 4-(2,3-dihydrobenzofuran-7-yl)-1,1,1-trifluoro-4-methyl-2-(1H-pyrrolo[3,2-c]pyridin-2-yelmethyl)pentan-2-ol; 1,1,1-trifluoro-4-methyl-4-phenyl-2-(1H-pyrrolo[2,3-c]pyridine-2-ylmethyl)pentan-2-ol; 1,1,1-trifluoro-4-(4-fluoro-2-methoxyphenyl)-4-methyl-2-(1H-pyrrolo[2,3-c]pyridin-2-ylmethyl)pentan-2-ol; 1,1,1-trifluoro-4-(4-fluoro-2-methoxyphenyl)-4-methyl-2-(1H-pyrrolo[3,2-c]pyridin-2-ylmethyl)pentan-2-ol; 1,1,1-trifluoro-4-methyl-4-phenyl-2-(1H-pyrrolo[3,2-c]pyridin-2-ylmethyl)pentan-2-ol; 1,1,1-trifluoro-4-(4-fluorophenyl)-4-methyl-2-(1H-pyrrolo[3,2-c]pyridin-2-ylmethyl)pentan-2-ol; 5-fluoro-2-[4,4,4-trifluoro-3-hydroxy-1,1-dimethyl-3-(1H-pyrrolo[2,3-c]pyridin-2-ylmethyl)butyl]phenol; 1,1,1-trifluoro-4-(5-fluoro-2-methylphenyl)-4-methyl-2-(1H-pyrrolo[2,3-c]pyridin-2-ylmethyl)pentan-2-ol; 1,1,1-trifluoro-4-(5-fluoro-2-methoxyphenyl)-4-methyl-2-(3-methyl-1H-pyrrolo[2,3-c]pyridin-2-ylmethyl)pentan-2-ol; 4-fluoro-2-[4,4,4-trifluoro-3-hydroxy-1,1-dimethyl-3-(3-methyl-1H-pyrrolo[2,3-c]pyridin-2-ylmethyl)butyl]phenol; 5-fluoro-2-[4,4,4-trifluoro-3-hydroxy-1,1-dimethyl-3-(1H-pyrrolo[3,2-c]pyridin-2-ylmethyl)butyl]pentan-2-ol; 1,1,1-trifluoro-4-(5-fluoro-2,3-dihydrobenzofuran-7-yl)-4-methyl-2-(1H-pyrrolo[2,3-c]pyridine-2-ylmethyl)pentan-2-ol; 4-fluoro-2-[4,4,4-trifluoro-3-hydroxy-1,1-dimethyl-3-(1H-pyrrolo[2,3-c]-[3-methylpyridin]-2-ylmethyl)butyl]phenol; 4-fluoro-2-[4,4,4-trifluoro-3-hydroxy-1,1-dimethyl-3-(1H-pyrrolo[2,3-c]-[2-fluoropyridin]-2-ylmethyl)butyl]phenol; and 4-fluoro-2-[4,4,4-trifluoro-3-hydroxy-1,1-dimethyl-3-(1H-pyrrolo[2,3-c]-[2-trifluoromethylpyridin]-2-ylmethyl)butyl]phenol.

For example, a DIGRA can have Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) $R^3$ is the trifluoromethyl group;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q comprises a heteroaryl group optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, acyl, $C_1$-$C_3$ silanyloxy, $C_1$-$C_5$ alkoxycarbonyl, carboxy, halogen, hydroxy, oxo, cyano, heteroaryl, heterocyclyl, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, or trifluoromethyl.

Examples of these compounds include 4-cyclohexyl-1,1,1-trifluoro-4-methyl-2-quinolin-4-ylmethylpentan-2-ol; 4-pyrimidin-5-yl-2-[4,4,4-trifluoro-3-hydroxy-1,1-dimethyl-3-(1H-pyrrolo[2,3-c]pyridin-2-ylmethyl)butyl]phenol; 4-pyrimidin-5-yl-2-[4,4,4-trifluoro-3-hydroxy-1,1-dimethyl-3-(1H-pyrrolo[3,2-c]pyridin-2-ylmethyl)butyl]phenol; 1,1,1-trifluoro-4-(5-fluoro-2-methoxyphenyl)-4-methyl-2-(3-methyl-1H-pyrrolo[3,2-c]pyridin-2-ylmethyl)pentan-2-ol; 1,1,1-trifluoro-4-(5-fluoro-2,3-dihydrobenzofuran-7-yl)-4-methyl-2-(1H-pyrrolo[3,2-c]pyridin-2-ylmethyl)pentan-2-ol; 1,1,1-trifluoro-4-(5-fluoro-2-methylphenyl)-4-methyl-2-(3-methyl-1H-pyrrolo[2,3-c]pyridin-2-ylmethyl)pentan-2-ol; 2-(4,6-dimethyl-1H-pyrrolo[3,2-c]pyridin-2-ylmethyl)-1,1,1-trifluoro-4-(5-fluoro-2-methoxyphenyl)-4-methylpentan-2-ol; 2-(5,7-dimethyl-1H-pyrrolo[2,3-c]pyridin-2-ylmethyl)-1,1,1-trifluoro-4-(5-fluoro-2-methoxyphenyl)-4-methylpentan-2-ol; 2-[4-(5-fluoro-2-methoxyphenyl)-2-hydroxy-4-methyl-2-trifluoromethylpentyl]-1H-pyrrolo[3,2-b]pyridine-5-carbonitrile; 1,1,1-trifluoro-4-(5-fluoro-2-methoxyphenyl)-4-methyl-2-(6-methyl-1H-pyrrolo[3,2-c]pyridin-2-ylmethyl)pentan-2-ol; 1,1,1-trifluoro-4-(5-fluoro-2-methoxyphenyl)-4-methyl-2-(4-methyl-1H-pyrrolo[3,2-c]pyridin-2-ylmethyl)pentan-2-ol; 2-[4-(5-fluoro-2-methoxyphenyl)-2-hydroxy-4-methyl-2-trifluoromethylpentyl]-4-methyl-1H-pyrrolo[3,2-c]pyridine-6-carbonitrile; 2-[4-(5-fluoro-2-methoxyphenyl)-2-hydroxy-4-methyl-2-trifluoromethylpentyl]-1H-pyrrolo[2,3-c]pyridine-5-carbonitrile; 2-[4-(5-fluoro-2-methoxyphenyl)-2-hydroxy-4-methyl-2-trifluoromethylpentyl]-1H-pyrrolo[3,2-c]pyridine-4-carbonitrile; 1,1,1-trifluoro-4-(5-fluoro-2-methoxyphenyl)-4-methyl-2-(5H-pyrrolo[3,2-d]pyrimidin-6-ylmethyl)pentan-2-ol; 1,1,1-trifluoro-4-(5-fluoro-2-methoxyphenyl)-4-methyl-2-thieno[2,3-d]pyridazin-2-ylmethylpentan-2-ol; 1,1,1-trifluoro-4-(5-fluoro-2-methoxyphenyl)-4-methyl-2-(5H-pyrrolo[3,2-c]pyridazin-6-ylmethyl)pentan-2-ol; 1,1,1-trifluoro-4-(5-fluoro-2-methoxyphenyl)-4-methyl-2-(2-methyl-5H-pyrrolo[3,2-d]pyrimidin-6-ylmethyl)pentan-2-ol; 1,1,1-trifluoro-4-(5-fluoro-2-methylphenyl)-4-methyl-2-(1H-pyrrolo[2,3-d]pyridazin-2-ylmethyl)pentan-2-ol; 2-(4,6-dimethyl-H-pyrrolo[3,2-c]pyridin-2-ylmethyl)-1,1,1-trifluoro-4-(5-fluoro-2-methylphenyl)-4-methylpentan-2-ol; 4-(5-chloro-2,3-dihydrobenzofuran-7-yl)-2-(4,6-dimethyl-1H-pyrrolo[3,2-c]pyridin-2-ytmethyl)-1,1,1-trifluoro-4-methylpentan-2-ol; 2-[4-(5-fluoro-2-methylphenyl)-2-hydroxy-4-methyl-2-trifluoromethylpentyl]-1H-pyrrolo[3,2-b]pyridine-5-carbonitrile; 4-(5-chloro-2,3-dihydrobenzofuran-7-yl)-1,1,1-trifluoro-4-methyl-2-(3-methyl-1H-pyrrolo[2,3-c]pyridin-2-ylmethyl)pentan-2-ol; 1,1,1-trifluoro-4-(5-fluoro-2-methylphenyl)-4-methyl-2-(5H-pyrrolo[3,2-c]- pyridazin-6-ylmethyl)pentan-2-ol; 4-(5-chloro-2,3-dihydrobenzofuran-7-yl)-1,1,1-trifluoro-4-methyl-2-(5H-pyrrolo[3,2-c]pyridazin-6-ylmethyl)pentan-2-ol; 4-(5-chloro-2,3-dihydrobenzofuran-7-yl)-1,1,1-trifluoro-4-methyl-2-(1-H-pyrrolo[2,3-d]pyridazin-2-ylmethyl)pentan-2-ol; 1,1,1-trifluoro-4-(5-fluoro-2-methoxyphenyl)-2-(7-fluoro-1H-pyrrolo[2,3-c]pyridin-2ylmethyl)-4-methylpentan-2-ol; 1,1,1-trifluoro-4-(5-fluoro-2-methoxyphenyl)-4-methyl-2-(4-methyl-1H-pyrrolo[2,3-c]pyridin-2-ylmethyl)pentan-2-ol; 2-(5,7-dichloro-1H-pyrrolo[2,3-c]pyridin-2-ylmethyl)-1,1,1-trifluoro-4-(5-fluoro-2-methoxyphenyl)-4-methylpentan-2-ol; 1,1,1-trifluoro-4-(5-fluoro-2-methoxyphenyl)-4-methyl-2-(5-trifluoromethyl-1H-pyrrolo[2,3-c]pyridin-2-ylmethyl)pentan-2-ol; 1,1,1-trifluoro-4-(5-fluoro-2-methoxyphenyl)-2-(5-methoxy-1H-pyrrolo[2,3-c]pyridin-2-ylmethyl)-4-methylpentan-2-ol; 1,1,1-trifluoro-4-(5-fluoro-2-methylphenyl)-4-methyl-2-(4-methyl-1H-pyrrolo[2,3-c]pyridin-2-ylmethyl)pentan-2-ol; 1,1,1-trifluoro-4-(5-fluoro-2-methylphenyl)-2-(5-isopropoxy-1H-pyrrolo[2,3-c]pyridin-2-ylmethyl)-4-methylpentan-2-ol; 1,1,1-trifluoro-4-(5-fluoro-2-methylphenyl)-2-(5-methoxy-1H-pyrrolo[2,3-c]pyridin-2-ylmethyl)-4-methylpentan-2-ol; 4-(5-chloro-2,3-dihydrobenzofuran-7-yl)-1,1,1-trifluoro-2-(5-methoxy-1H-pyrrolo[2,3-c]pyridin-2-ylmethyl)-4-methylpentan-2-ol; 1,1,1-trifluoro-4-(5-fluoro-2-methylphenyl)-2-(7-fluoro-1H-pyrrolo[2,3-c]pyridin-2-ylmethyl)-4-methylpentan-2-ol; 4-(5-chloro-2,3-dihydrobenzofuran-7-yl)-1-trifluoro-4-methyl-2-(5-trifluoromethyl-1H-pyrrolo[2,3-c]pyridin-2-ylmethyl)pentan-2-ol; 1,1,1-trifluoro-4-(5-fluoro-2-methylphenyl)-4-methyl-2-(5-trifluoromethyl-1H-pyrrolo[2,3-c]pyridin-2-ylmethyl)pentan-2-ol; 4-(5-chloro-2,3-dihydrobenzofuran-7-yl)-1,1,1-trifluoro-2-(5-isopropoxy-1H-pyrrolo[2,3-c]pyridin-2-ylmethyl)-4-methylpentan-2-ol; 4-(5-chloro-2,3-dihydrobenzofuran-7-yl)-1,1,1-trifluoro-2-(7-fluoro-1H-pyrrolo[2,3-c]pyridin-2-ylmethyl)-4-methylpentan-2-ol; 4-(5-chloro-2,3-dihydrobenzofuran-7-yl)-2-(5-dimethylamino-1H-pyrrolo[2,3-c]pyridin-2-ylmethyl)-1,1,1-trifluoro-4-methylpentan-2-ol; 4-(5-chloro-2,3-dihydrobenzofuran-7-yl)-1,1,1-trifluoro-4-methyl-2-(5-piperidin-1-yl-1H-pyrrolo[2,3-c]pyridin-2-ylmethyl)pentan-2-ol; 4-(5-chloro-2,3-dihydrobenzofuran-7-yl)-1,1,1-trifluoro-4-methyl-2-(5-morpholin-4-yl-1H-pyrrolo[2,3-c]pyridin-2-ylmethyl)pentan-2-ol; 1,1,1-trifluoro-4-(5-fluoro-2-methylphenyl)-4-methyl-2-(5-piperidin-1-yl-1H-pyrrolo[2,3-c]pyridin-2-ylmethyl)pentan-2-ol; 4-(5-chloro-2,3-dihydrobenzofuran-7-yl)-2-(5-ethoxy-1H-pyrrolo[2,3-c]pyridin-2-ylmethyl)-1,1,1-trifluoro-4-methylpentan-2-ol; 2-(5-benzyloxy-1H-pyrrolo[2,3-c]pyridin-2-ylmethyl)-1,1,1-trifluoro-4-(5-fluoro-2-methylphenyl)-4-methylpentan-2-ol; 2-(5-benzyloxy-1H-pyrrolo[2,3-c]pyridin-2-ylmethyl)-4-(5-chloro-2,3-dihydrobenzofiran-7-yl)-1,1,1-trifluoro-4-methylpentan-2-ol; 1,1,1-trifluoro-4-(5-fluoro-2-methoxyphenyl)-2-(5-chloro-1H-pyrrolo[2,3-c- ]pyridin-2-ylmethyl)-4-methylpentan-2-ol; 1,1,1-trifluoro-4-(5-fluoro-2-methoxyphenyl)-4-methyl-2-[5-(methylamino)-1H-pyrrolo[2,3-c]pyridin-2-ylmethyl]pentan-2-ol; 1,1,1-trifluoro-4-(5-fluoro-2-methoxyphenyl)-4-methyl-2-(5-amino-1H-pyrrolo [2,3-c]pyridin-2-ylmethyl)pentan-2-ol; 1,1,1-trifluoro-4-(5-fluoro-2-methylphenyl)-4-methyl-2-(6-amino-1H-pyrrol- o[2,3-c]pyridin-2-ylmethyl)pentan-2-ol; 4-(5-chloro-2,3-dihydrobenzofuran-7-yl)-1,1,1-trifluoro-2-(5-amino-1H-pyrrolo[2,3-c] pyridin-2-ylmethyl)-4-methylpentan-2-ol; 4-(5-chloro-2,3-dihydrobenzofuran-7-yl)-1,1,1-trifluoro-4-methyl-2-(5-methylamino-1H-pyrrolo[2,3-c]pyridin-2-ylmethyl)pentan-2-ol; 7-[4-(5-fluoro-2-methoxyphenyl)-2-hydroxy-4-methyl-2-trifluoromethylpentyl]-1H-pyrrolo[2,3-b]pyridin-7-ium chloride; 6-[4-(5-fluoro-2-methoxyphenyl)-2-hydroxy-4-methyl-2-trifluoromethylpentyl]-2-methyl-1H-pyrrolo[2,3-c]pyridin-6-ium chloride; 4-(5-bromo-2,3-dihydrobenzofuran-7-yl)-1,1,1-trifluoro-4-methyl-2-(1H-pyrrolo[2,3-c]pyridin-2-ylmethyl)pentan-2-ol; 1,1,1-trifluoro-4-methyl-4-(5-methyl-2,3-dihydrobenzofuran-7-yl)-2-(1H-pyrrolo[2,3-c]pyridin-2-ylmethyl)pentan-2-ol; 4-(5-chloro-2,3-dihydrobenzofuran-7-yl)-1,1,1-trifluoro-4-methyl-2-(1H-pyrrolo[2,3-c]pyridin-2-ylmethyl)pentan-2-ol; 1,1,1-trifluoro-4-(5-fluoro-2-methoxyphenyl)-4-methyl-2-pyrrolo[2,3-b]pyridin-1-ylmethylpentan-2-ol; 1,1,1-trifluoro-4-(5-fluoro-2-methoxyphenyl)-4-methyl-2-(6-oxy-1H-pyrrolo[2,3-c]pyridin-2-ylmethyl)pentan-2-ol; 1,1,1-trifluoro-4-(5-fluoro-2-methoxyphenyl)-4-methyl-2-pyrrolo[2,3-c]pyridin-1-ylmethylpentan-2-ol; 2-benzo[b]thiophen-2-ylmethyl-1,1,1-trifluoro-4-(5-fluoro-2-methoxyphenyl)-4-methylpentan-2-ol; 1,1,1-trifluoro-4-(5-fluoro-2-methoxyphenyl)-4-methyl-2-thieno[2,3-c]pyridin-2-ylmethylpentan-2-ol; 1,1,1-trifluoro-4-(5-fluoro-2-methoxyphenyl)-2-indazol-1-ylmethyl-4-methylpentan-2-ol; 1,1,1-trifluoro-4-(5-fluoro-2-methoxyphenyl)-4-methyl-2-pyrazolo[1,5-a]pyridin-2-ylmethylpentan-2-ol; 4-(5-chloro-2,3-dihydrobenzofuran-7-yl)-2,4-dimethyl-1-thieno[2,3-c]pyridin-2-ylpentan-2-ol; 4-(5-fluoro-2-methylphenyl)-2,4-dimethyl-1-thieno[2,3-c]pyridin-2-ylpentan-2-ol; 1,1,1-trifluoro-4-(5-fluoro-2-methoxyphenyl)-2-furo[2,3-c]pyridin-2-ylmethy- 1-4-methylpentan-2-ol; 4-(5-chloro-2,3-dihydrobenzofuran-7-yl)-1-furo[2,3-c]pyridin-2-yl-2,4-dimethylpentan-2-ol; 4-(5-fluoro-2-methylphenyl)-1-furo-[2,3-c]pyridin-2-yl-2,4-dimethylpentan-2-ol; 1,1,1-trifluoro-4-(5-fluoro-2-methylphenyl)-4-methyl-2-(1H-pyrrolo[3,2-c]pyridin-2-ylmethyl)pentan-2-ol- ; 1,1,1-trifluoro-4-methyl-4-(5-methyl-2,3-dihydrobenzofuran-7-yl)-2-(1H-pyrrolo[3,2-c]pyridin-2-ylmethyl)pentan-2-ol; 4-(5-chloro-2,3-dihydrobenzofuran-7-yl)-1,1,1-trifluoro-4-methyl-2-(1H-pyrrolo[3,2-c]pyridin-2-ylmethyl)pentan-2-ol; 4-(5-bromo-2,3-dihydrobenzofuran-7-yl)-1,1,1-trifluoro-4-methyl-2-(1H-pyrrolo[3,2-c]pyridin-2-ylmethyl)pentan-2-ol; 2-(3-dimethylaminomethyl-1H-pyrrolo[3,2-c]pyridin-2-ylmethyl)-1,1,1-trifluoro-4-(5-fluoro-2-methoxyphenyl)-4-methylpentan-2-ol; 1,1,1-trifluoro-4-(5-fluoro-2-methoxyphenyl)-4-methyl-2-pyrrolo[3,2-c]pyridin-1-ylmethylpentan-2-ol; 1,1,1-trifluoro-4-(5-fluoro-2-methoxyphenyl)-4-methyl-2-pyrrolo[3,2-b]pyridin-1-ylmethylpentan-2-ol; 1,1,1-trifluoro-4-(5-fluoro-2-methoxyphenyl)-2-furo[3,2-c]pyridin-2-ylmethyl-4-methylpentan-2-ol; 4-(5-chloro-2,3-dihydrobenzofuran-7-yl)-1,1,1-trifluoro-4-methyl-2-pyrrolo[3,2-b]pyridin-1-ylmethylpentan-2-ol; 1,1,1-trifluoro-4-(5-fluoro-2-methoxyphenyl)-4-methyl-2-thieno[3,2-c]pyridin-2-ylmethylpentan-2-ol; 4-(5-chloro-2,3-dihydrobenzofuran-7-yl)-1,1,1-trifluoro-4-methyl-2-thieno[3,2-c]pyridin-2-ylmethylpentan-2-ol; 1,1,1-trifluoro-4-(5-fluoro-2-methylphenyl)-4-methyl-2-pyrrolo[3,2-b]pyridin-1-ylmethylpentan-2-ol; 1,1,1-trifluoro-4-(5-fluoro-2-methylphenyl)-4-methyl-2-thieno[3,2-c]pyridin-2-ylmethylpentan-2-ol; 4-fluoro-2-(4,4,4-trifluoro-3-hydroxy-1,1-dimethyl-3-thieno[3,2-c]pyridin-2-ylmethylbutyl)phenol; 4-fluoro-2-(4,4,4-trifluoro-3-furo[3,2-c]pyridin-2-ylmethyl-3-hydroxy-1,1-dimethylbutyl)phenol; 4-fluoro-2-(4,4,4-trifluoro-3-hydroxy-1,1-dimethyl-3-pyrrolo[3,2-b]pyridin-1-ylmethylbutyl)phenol; 2-[4-(5-fluoro-2-hydroxyphenyl)-2-hydroxy-4-methyl-2-trifluoromethylpentyl]-1H-indole-6-carboxylic acid; 2-[4-(5-fluoro-2-hydroxyphenyl)-2-hydroxy-4-methyl-2-trifluoromethylpentyl]-1H-indole-6-carboxylic acid dimethylamide; {2-[4-(5-fluoro-2-hydroxyphenyl)-2-hydroxy-4-methyl-2-trifluoromethylpentyl]-1H-indol-6-yl}morpholin-4-ylmethanone; 2-[4-(5-fluoro-2-methoxyphenyl)-2-hydroxy-4-methyl-2-trifluoromethylpentyl]-1H-indole-6-carboxylic acid dimethylamide; {2-[4-(5-fluoro-2-methoxyphenyl)-2-hydroxy-4-methyl-2-trifluoromethylpentyl]-1H-indol-6-yl}morpholin-4-ylmethanone; 2-[4-(5-fluoro-2-hydroxyphenyl)-2-hydroxy-4-methyl-2-trifluoromethylpentyl]-1H-indole-6-carboxylic acid amide; 2-[4-(5-fluoro-2-methoxyphenyl)-2-hydroxy-4-methyl-2-trifluoromethylpentyl]-1H-indole-6-carboxylic acid amide; 4-fluoro-2-[4,4,4-trifluoro-3-hydroxy-1,1-dimethyl-3-(5-nitro-1H-indol-2-ylmethyl)butyl]phenol; 2-[4-(5-fluoro-2-methoxyphenyl)-2-hydroxy-4-methyl-2-trifluoromethylpentyl]-1H-indole-6-carbonitrile; 2-[4-(5-fluoro-2-hydroxyphenyl)-2-hydroxy-4-methyl-2-trifluoromethylpentyl]-1H-indole-6-carbonitrile; N-{2-[4-(5-fluoro-2-methoxyphenyl)-2-hydroxy-4-methyl-2-trifluoromethylpentyl]-1H-indol-5-yl}acetamide; 1,1,1-trifluoro-4-(4-fluoro-2-methoxyphenyl)-2-(7-fluoro-4-methyl-1H-indo-1-2-ylmethyl)-4-methylpentan-2-ol; 5-fluoro-2-[4,4,4-trifluoro-3-(7-fluoro-4-methyl-1H-indol-2-ylmethyl)-3-hydroxy-1,1-dimethylbutyl]phenol; 2-[4-(3-[1,3 ]dioxolan-2-ylphenyl)-2-hydroxy-4-methyl-2-trifluoromethylpentyl]-1H-indole-5-carbonitrile; 2-[4-(5-fluoro-2-methoxyphenyl)-2-hydroxy-4-methyl-2-trifluoromethylpentyl]-1H-indole-5-carboxylic acid-2-trimethylsilanylethyl ester; 2-[4-(5-fluoro-2-methoxyphenyl)-2-hydroxy-4-methyl-2-trifluoromethylpentyl]-1H-indole-5-carboxylic acid; 2-[4-(4-fluoro-2-hydroxyphenyl)-2-hydroxy-4-methyl-2-trifluoromethylpenty- 1]-4-methyl-1H-indole-6-carbonitrile; {2-[4-(5-Fluoro-2-methoxyphenyl)-2-hydroxy-4-methyl-2-trifluoromethylpentyl]-1H-indol-5-yl}piperidin-1-ylmethanone; 2-[4-(5-fluoro-2-methoxyphenyl)-2-hydroxy-4-methyl-2-trifluoromethylpentyl]-1H-indole-5-carboxylic acid methylamide; {2-[4-(5-fluoro-2-methoxyphenyl)-2-hydroxy-4-methyl-2-trifluoromethylpentyl]-1H-indol-5-yl}pyrrolidin-1-ylmethanone; 1-{2-[4-(5-fluoro-2-methoxyphenyl)-2-hydroxy-4-methyl-2-trifluoromethylpentyl]1H-indole-5-carbonyl}piperidin-4-one; 2-[4-(5-fluoro-2-methoxyphenyl)-2-hydroxy-4-methyl-2-trifluoromethylpentyl]-1H-indole-5-carboxylic acid (2-hydroxyethyl)amide; {2-[4-(5-fluoro-2-methoxyphenyl)-2-hydroxy-4-methyl-2-trifluoromethylpentyl]-1H-indol-5-yl}(4-hydroxypiperidin-1-yl)methanone; {2-[4-(5-fluoro-2-methoxyphenyl)-2-hydroxy-4-methyl-2-trifluoromethylpentyl]-1H-indol-5-yl}(3-hydroxypyrrolidin-1-yl)methanone; 2-[4-(5-fluoro-2-methoxyphenyl)-2-hydroxy-4-methyl-2-trifluoromethylpentyl]-1H-indole-5-carboxylic acid cyanomethylamide; 2-[4-(5-fluoro-2-methoxyphenyl)-2-hydroxy-4-methyl-2-trifluoromethylpentyl]-1 H-indo le-5-carboxylic acid (2-dimethylaminoethyl)amide; {2-[4-(5-fluoro-2-methoxyphenyl)-2-hydroxy-4-methyl-2-trifluoromethylpentyl]-1H-indol-5-yl}(4-methylpiperazin-1-yl)methanone; ({2-[4-(5-fluoro-2-methoxyphenyl)-2-hydroxy-4-methyl-2-trifluoromethylpentyl]-1H-indole-5-carbonyl}amino)acetic acid methyl ester; 2-[4-(5-fluoro-2-methoxyphenyl)-2-hydroxy-4-methyl-2-trifluoromethylpentyl]-1H-indole-5-carboxylic acid carbamoylmethylamide; 4-({2-[4-(5-fluoro-2-methoxyphenyl)-2-hydroxy-4-methyl-2-trifluoromethylpentyl]-1H-indole-5-carbonyl}amino)butyric acid methyl ester; ({2-[4-(5-fluoro-2-methoxyphenyl)-2-hydroxy-4-methyl-2-trifluoromethylpentyl]-1H-indole-5-carbonyl}amino)acetic acid; 4-({2-[4-(5-fluoro-2-methoxyphenyl)-2-hydroxy-4-methyl-2-trifluoromethylpentyl]-1H-indole-5-carbonyl}amino)butyric acid; 2-[4-(3-dimethylaminomethylphenyl)-2-hydroxy-4-methyl-2-trifluoromethylpentyl]-1H-indole-5-carbonitrile; 4-fluoro-2-[4,4,4-trifluoro-3-hydroxy-1,1-dimethyl-3-(5-trifluoromethyl-1H-indol-2-ylmethyl)butyl]phenol; 2-[4-(5-bromo-2,3-dihydrobenzofuran-7-yl)-2-hydroxy-4-methyl-2-trifluoromethylpentyl]-4-methyl-1H-indole-6-carbonitrile; 2-[2-hydroxy-4-(5-methanesulfonyl-2,3-dihydrobenzofuran-7-yl)-4-methyl-2-trifluoromethylpentyl]-4-methyl-1H-indole-6-carbonitrile; 2-[4-(5-bromo-2,3-dihydrobenzofuran-7-yl)-2-hydroxy-4-methyl-2-trifluoromethylpentyl]-1H-indole-5-carboxylic acid; 2-[4-(5-bromo-2,3-dihydrobenzofuran-7-yl)-2-hydroxy-4-methyl-2-trifluoromethylpentyl]-1H-indole-5-carboxylic acid amide; 2-[4-(5-bromo-2,3-dihydrobenzofuran-7-yl)-2-hydroxy-4-methyl-2-trifluoromethylpentyl]-1H-indole-5-carboxylic acid dimethylamide; 2-[4-(5-Bromo-2,3-dihydrobenzofuran-7-yl)-2-hydroxy-4-methyl-2-trifluoromethylpentyl]-1H-indole-5-carboxylic acid cyanomethylamide; {2-[4-(5-bromo-2,3-dihydrobenzofuran-7-yl)-2-hydroxy-4-methyl-2-trifluoromethylpentyl]-1H-indol-5-yl}pyrrolidin-1-ylmethanone; {2-[4-(5-bromo-2,3-dihydrobenzofuran-7-yl)-2-hydroxy-4-methyl-2-trifluoro-methylpentyl]-1H-indol-5-yl}morpholin-4-ylmethanone; 2-[4-(5-fluoro-2-methoxyphenyl)-2-hydroxy-4-methyl-2-trifluoromethylpentyl]-1H-indole-5-carboxylic acid amide; {2-[4-(5-fluoro-2-methoxyphenyl)-2-hydroxy-4-methyl-2-trifluoromethylpentyl]-1H-indol-5-yl}morpholin-4-ylmethanone; 2-(4-benzo[1,3]dioxol-4-yl-2-hydroxy-4-methyl-2-trifluoromethylpentyl)-4-methyl-1H-indole-6-carbonitrile; 1,1,1-trifluoro-4-methyl-4-phenyl-2-quinolin-4-ylmethylhexan-2-ol; 2-[2-hydroxy-4-methyl-4-(5-methylsulfanyl-2- ,3-dihydrobenzofuran-7-yl)-2-trifluoromethylpentyl]-1H-indole-3-carbonitrile; 7-(4,4,4-trifluoro-3-hydroxy-1,1-dimethyl-3-quinolin-4-ylmethylbutyl)-2,3-dihydrobenzofuran-5-carbonitrile; 2-[2-hydroxy-4-(5-methanesulfonyl-2,3-dihydrobenzofuran-7-yl)-4-methyl-2-trifluoromethylpentyl]-1H-indole-3-carbonitrile; 2-[2-hydroxy-4-(2-hydroxy-5-methylphenyl)-4-methyl-2-trifluoro-methylpentyl]-4-methyl-1H-indole-6-carbonitrile; 1,1,1-trifluoro-4-(5-fluoro-2,3-dihydrobenzofuran-7-yl)-4-methyl-2-(5-methylsulfanyl-1H-indol-2-ylmethyl)pentan-2-ol; 2-[2-hydroxy-4-(2-methoxy-5-methylsulfanylphenyl)-4-methyl-2-trifluoromethylpentyl]-1H-indole-3-carbonitrile; 2-[2-Hydroxy-4-(5-methanesulfonyl-2-methoxyphenyl)-4-methyl-2-trifluoromethylpentyl]-1H-indole-3-carbonitrile; 2-[4-(5-fluoro-2,3-dihydrobenzofuran-7-yl)-2-hydroxy-4-methyl-2-trifluoromethylpentyl]-1H-indole-5-sulfonic acid dimethylamide; 1,1,1-trifluoro-4-(5-fluoro-2,3-dihydrobenzofuran-7-y- 1)-4-methyl-2-(5-phenyl-1H-indol-2-ylmethyl)pentan-2-ol; 2-[4-(5-tert-butyl-2-hydroxyphenyl)-2-hydroxy-4-methyl-2-trifluoromethylpentyl]-1H-indole-3-carbonitrile; 2-[2-hydroxy-4-(2-hydroxy-5-isopropylphenyl)-4-methyl-2-trifluoromethylpentyl]-1H-indole-3-carbonitrile; 2-[2-hydroxy-4-(2-hydroxy-3,5-dimethylphenyl)-4-methyl-2-trifluoromethylpentyl]-1H-indole-3-carbonitrile; 2-[2-hydroxy-4-(5-hydroxy-2,4-dimethylphenyl)-4-methyl-2-trifluoromethylpentyl]-1H-indole-3-carbonitrile; 2-[4-(5-tert-butyl-2-methoxyphenyl)-2-hydroxy-4-methyl-2-trifluoromethylpentyl]-1H-indole-3-carbonitrile; 2-[4-(5-tert-butyl-2-methoxyphenyl)-2-hydroxy-4-methyl-2-trifluoromethylpentyl]-1-methyl-1H-indole-3-carbonitrile; 2-[2-hydroxy-4-(5-isopropyl-2-methoxyphenyl)-4-methyl-2-trifluoromethylpentyl]-1H-indole-3-carbonitrile; 2-[2-hydroxy-4-(5-isopropyl-2-methoxyphenyl)-4-methyl-2-trifluoromethylpentyl]-1-methyl-1H-indole-3-carbonitrile; 2-[2-hydroxy-4-(2-hydroxy-5-methanesulfonylphenyl)-4-methyl-2-trifluoromethylpentyl]-1H-indole-3-carbonitrile; 2-[2-hydroxy-4-(2-methoxy-5-methylphenyl)-4-methyl-2-trifluoromethylpentyl]-4-methyl-1H-indole-6-carbonitrile; 1,1,1-trifluoro-4-methyl-2-quinolin-4-ylmethyl-4-o-tolylpentan-2-ol; 1,1,1-trifluoro-4-methyl-2-quinolin-4-ylmethyl-4-m-tolylpentan-2-ol; 1,1,1-trifluoro-4-(2-fluorophenyl)-2-(1H-indol-2-ylmethyl)-4-methylpentan-2-ol; 1,1,1-trifluoro-4-(2-fluorophenyl)-4-methyl-2-quinolin-4-ylmethylpentan-2-ol; 1,1,1-trifluoro-4-(3-fluorophenyl)-2-(1H-indol-2-ylmethyl)-4-methylpentan-2-ol; 1,1,1-trifluoro-4-(3-fluorophenyl)-4-methyl-2-quinolin-4-ylmethylpentan-2-ol; 1,1,1-trifluoro-4-(4-fluorophenyl)-2-(1H-indol-2-ylmethyl)-4-methylpentan-2-ol; 1,1,1-trifluoro-4-(4-fluorophenyl)-4-methyl-2-quinolin-4-ylmethylpentan-2-ol; 3-(4,4,4-trifluoro-3-hydroxy-1,1-dimethyl-3-quinolin-4-ylmethylbutyl)phenol; 1,1,1-trifluoro-4-methyl-2-quinolin-4-ylmethyl-4-(2-trifluoromethylphenyl)pentan-2-ol; 1,1,1-trifluoro-2-(1H-indol-2-ylmethyl)-4-methyl-4-(4-trifluoromethylphenyl)pentan-2-ol; 1,1,1-trifluoro-4-methyl-2-quinolin-4-ylmethyl-4-(4-trifluoromethylphenyl)pentan-2-ol; 4-(3-chlorophenyl)-1,1,1-trifluoro-2-(1H-indol-2-ylmethyl)-4-methylpentan-2-ol; 4-(3-chlorophenyl)-1,1,1,-trifluoro-4-methyl-2-quinolin-4-ylmethylpentan-2-ol; 4-(4-dimethylaminophenyl)-1,1,1-trifluoro-2-(1H-indol-2-ylmethyl)-4-methylpentan-2-ol; 4-biphenyl-3-yl-1,1,1-trifluoro-4-methyl-2-quinolin-4-ylmethylpentan-2-ol; 4-(3-bromophenyl)-1,1,1-trifluoro-2-(1H-indol-2-ylmethyl)-4-methylpentan-2-ol; 4-(2-difluoromethoxy-5-fluorophenyl)-1,1,1-trifluoro-2-(1H-indol-2-ylmethyl)-4-methylpentan-2-ol; 4-biphenyl-3-yl-1,1,1-trifluoro-2-(1H-indol-2-ylmethyl)-4-methylpentan-2-ol; 4-(4-dimethylaminophenyl)-1,1,1-trifluoro-4-methyl-2-quinolin-4-ylmethylpentan-2-ol; 2-[4-(5-fluoro-2-methylphenyl)-2-hydroxy-4-methyl-2-trifluoromethylpentyl]-1,6-dihydropyrrolo[2,3-c]pyridin-5-one; 2-[4-(5-Fluoro-2-methylphenyl)-2-hydroxy-4-methyl-2-trifluoromethylpentyl]-6-methyl-1,6-dihydropyrrolo[2,3-c]pyridin-5-one; 2-[4-(5-fluoro-2-methyl- phenyl)-2-hydroxy-4-methyl-2-trifluoromethylpentyl]-4-methyl-1,4-dihydropyrrolo[3,2-b]pyridin-5-one; 1,1,1-trifluoro-4-(5-fluoro-2-methylphenyl)-2-(6-methoxy-1H-pyrrolo[3,2-c]pyridin-2-ylmethyl)-4-methylpentan-2-ol; 2-[4-(5-fluoro-2-methylphenyl)-2-hydroxy-4-methyl-2-trifluoromethylpentyl]-5-methyl-1,5-dihydropyrrolo[3,2-c]pyridin-6-one; 2-[4-(5-fluoro-2-methylphenyl)-2-hydroxy-4-methyl-2-trifluoromethylpentyl]-1,3a-dihydropyrrolo[3,-2-c]pyridin-6-one; 2-[4-(5-fluoro-2-methylphenyl)-2-hydroxy-4-methyl-2-trifluoromethylpentyl]-1,7-dihydropyrrolo[3,2-c]pyridine-4,6-dione; 6-[4-(5-fluoro-2-methylphenyl)-2-hydroxy-4-methyl-2-trfluoromethylpentyl]-3-methyl-1,7-dihydropyrrolo[2,3-d]pyrimidine-2,4-dione; 2-[4-(5-chloro-2,3-dihydrobenzofuran-7-yl)-2-hydroxy-4-methyl-2-trifluoro- methylpentyl]-1,6-dihydropyrrolo[2,3-c]pyridin-5-one; 2-[4-(5-chloro-2,3-dihydrobenzofuran-7-yl)-2-hydroxy-4-methyl-2-trifluoromethylpentyl]-6-methyl-1,6-dihydropyrrolo[2,3-c]pyridin-5-one; 2-[4-(5-chloro-2,3-dihydrobenzofuran-7-yl)-2-hydroxy-4-methyl-2-trifluoromethylpentyl]-1,4-dihydropyrrolo[3,2-b]pyridin-5-one; 2-[4-(5-chloro-2,3-dihydrobenzofiran-7-yl)-2-hydroxy-4-methyl-2-trifluoromethylpentyl]-4-methyl-1,4-dihydropyrrolo[3,2-b]pyridin-5-one; 2-[4-(5-chloro-2,3-dihydrobenzofuran-7-yl)-2-hydroxy-4-methyl-2-trifluoro-methylpentyl]-1,5-dihydropyrrolo[3,2-c]pyridin-6-one; 2-[4-(5-chloro-2,3-dihydrobenzofuran-7-yl)-2-hydroxy-4-methyl-2-trifluoromethylpentyl]-5-methyl-1,5-dihydropyrrolo[3,2-c]pyridin-6-one; 4-(5-chloro-2,3-dihydrobenzofuran-7-yl)-1,1,1-trifluoro-2-(6-methoxy-5,6-dihydro-1H-pyrrolo[3,2-c]pyridin-2-ylmethyl)-4-methylpentan-2-ol; 2-[4-(5-chloro-2,3-dihydrobenzofuran-7-yl)-2-hydroxy-4-methyl-2-trifluoromethylpentyl]-1,7-dihydropyrrolo[3,2-c]pyridine-4,6-dione; 6-[4-(5-chloro-2,3-dihydrobenzofuran-7-yl)-2-hydroxy-4-methyl-2-trifluoromethylpentyl]-3-methyl-1,7-dihydropyrrolo[2,3-d]pyrimidine-2,4-dione; 2-[4-(3-dimethylaminomethylphenyl)-2-hydroxy-4-methyl-2-trifluoromethylpentyl]-1H-indole-5-carbonitrile; 1,1,1-trifluoro-2-(1H-indol-2-ylmethyl)-4-methyl-4-(3-morpholin-4-ylmethylphenyl)pentan-2-ol; 1,1,1-trifluoro-4-methyl-4-(3-morpholin-4-ylmethylphenyl)-2-(1H-pyrrolo[2- ,3-d]pyridazin-2-ylmethyl)pentan-2-ol; 1,1,1-trifluoro-4-(5-fluoro-2-methylphenyl)-4-methyl-2-(5-morpholin-4-ylmethyl-1H-indol-2-ylmethyl)pentan-2-ol; 1,1,1-trifluoro-4-(5-fluoro-2-methylphenyl)-4-methyl-2-(5-morpholin-4-ylmethyl-1H-pyrrolo[2,3-c]pyridin-2-ylmethyl)pentan-2-ol; {2-[4-(5-fluoro-2-methylphenyl)-2-hydroxy-4-methyl-2-trifuoromethylpentyl]-1H-indol-5-yl}phenylmethanone; {2-[4-(5-fluoro-2-methylphenyl)-2-hydroxy-4-methyl-2-trifluoromethylpenty-1]-1H-pyrrolo[2,3-c]pyridin-5-yl}phenylmethanone; {2-[4-(5-fluoro-2-methylphenyl)-2-hydroxy-4-methyl-2-trifluoromethylpentyl]-1H-indol-5-yl}furan-2-ylmethanone;

{2-[4-(5-fluoro-2-methylphenyl)-2-hydroxy-4-methyl-2-trifluoromethylpentyl]-1H-pyrrolo[2,3-c]pyridin-5-yl}furan-2-ylmethanone; 1,1,1-trifluoro-2-(1H-indol-2-ylmethyl)-4-methyl-4-pyridin-2-ylpentan-2-ol; 1,1,1-trifluoro-4-methyl-4-pyridin-4-yl-2-quinolin-4-ylmethylpentan-2-ol; 2-(2,6-dimethylpyridin-4-ylmethyl)-1,1,1-trifluoro-4-(5-fluoro-2-methoxyphenyl)-4-methylpentan-2-ol; 2-[3-(2,6-dimethylpyridin-4-ylmethyl)-4,4,4-trifluoro-3-hydroxy-1,1-dimethylbutyl]-4-fluorophenol; 1,1,1-trifluoro-4,4-dimethyl-5-phenyl-2-quinolin-4-ylmethylpentan-2-ol; 1,1,1-trifluoro-4-(5-fluoro-2-methoxyphenyl)-4-methyl-2-pyridin-4-ylmethylpentan-2-ol; 4-fluoro-2-[4,4,4-trifluoro-3-(2-fluoropyridin-4-ylmethyl)-3-hydroxy-1,1-dimethylbutyl]phenol; 2-[3-(2-bromopyridin-4-ylmethyl)-4,4,4-trifluoro-3-hydroxy-1,1-dimethylbutyl]-4-fluorophenol; 2-(6,8-dimethylquinolin-4-ylmethyl)-1,1,1-trifluoro-4-(5-fluoro-2-methoxy- phenyl)-4-methylpentan-2-ol; 4-[4-(5-fluoro-2-methoxyphenyl)-2-hydroxy-4-methyl-2-trifluoromethylpentyl]pyridine-2-carbonitrile; 2,6-dichloro-4-[4-(5-fluoro-2-methoxyphenyl)-2-hydroxy-4-methyl-2-trifluoromethylpentyl]nicotinonitrile; 4-[4-(5-fluoro-2-methoxyphenyl)-2-hydroxy-4-methyl-2-trifluoromethylpentyl]quinolin-2-ol; 2,6-dichloro-4-[4-(5-fluoro-2-hydroxyphenyl)-2-hydroxy-4-methyl-2-trifluoromethylpentyl]nicotinonitrile; 2-(2-chloro-8-methylquinolin-4-ylmethyl)-1,1,1-trifluoro-4-(5-fluoro-2-methoxyphenyl)-4-methylpentan-2-ol; 2-(2,6-dichloroquinolin-4-ylmethyl)-1,1,1-trifluoro-4-(5-fluoro-2-methoxyphenyl)-4-methylpentan-2-ol; 2-[3-(2-chloro-8-methylquinolin-4-ylmethyl)-4,4,4-trifluoro-3-hydroxy-1,1-dimethylbutyl]-4-fluorophenol; 2-[3-(2,6-dichloroquinolin-4-ylmethyl)-4,4,4-trifluoro-3-hydroxy-1,1-dimethylbutyl]-4-fluorophenol; 4-(2,3-dihydrobenzofuran-7-yl)-2-(2,6-dimethylpyridin-4-ylmethyl)-1,1,1-trifluoro-4-methylpentan-2-ol; 2-(2,6-dimethylpyridin-4-ylmethyl)-1,1,1-trifluoro-4-(3-fluorophenyl)-4-methylpentan-2-ol; 2-(2,6-dimethylpyridin-4-ylmethyl)-1,1,1-trifluoro-4-(4-fluorophenyl)-4-methylpentan-2-ol; 1,1,1-trifluoro-4-(5-fluoro-2-methylphenyl)-4-methyl-2-quinolin-4-ylmethylpentan-2-ol; 2-(2,6-dimethylpyridin-4-ylmethyl)-1,1,1-trifluoro-4-(5-fluoro-2-methylphenyl)-4-methylpentan-2-ol; 2-(2,6-dimethylpyridin-4-ylmethyl)-1,1,1-trifluoro-4-methyl-4-m-tolylpentan-2-ol; 1,1,1-trifluoro-4-(5-fluoro-2-methoxyphenyl)-4-methyl-2-(2-methylquinolin-4-ylmethyl)pentan-2-ol; 4-fluoro-2-(4,4,4-trifluoro-3-hydroxy-1,1,1-dimethyl-3-quinolin-4-ylmethylbutyl)phenol; 4-fluoro-2-[4,4,4-trifluoro-3-hydroxy-1,1-dimethyl-3-(2-methylquinolin-4-ylmethyl)butyl]phenol; 2-(2,6-dimethylpyridin-4-ylmethyl)-1,1,1-trifluoro-4-(4-fluoro-2-methoxyphenyl)-4-methylpentan-2-ol; 1,1,1-trifluoro-4-(5-fluoro-2-methoxyphenyl)-4-methyl-2-(7-methylquinolin-4-ylmethyl)pentan-2-ol; 2-[3-(2,6-dimethylpyridin-4-ylmethyl)-4,4,4-trifluoro-3-hydroxy-1,1-dimethylbutyl]-5-fluorophenol; and 2-(5,7-dimethylquinolin-4-ylmethyl)-1,1,1-trifluoro-4-(5-fluoro-2-methoxyphenyl)-4-methylpentan-2-ol.

For example, a DIGRA can have Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl;

(c) $R^3$ is hydrogen, $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein $R^3$ cannot be trifluoromethyl;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q comprises a heteroaryl group optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$

alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, acyl, $C_1$-$C_3$ silanyloxy, $C_1$-$C_5$ alkoxycarbonyl, carboxy, halogen, hydroxy, oxo, cyano, heteroaryl, heterocyclyl, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, or trifluoromethyl.

Examples of these compounds include 2-cyclopropyl-4-(5-fluoro-2-methoxyphenyl)-4-methyl-1-(1H-pyrrolo[3,2-c]pyridin-2-yl)pentan-2-ol; 4-(5-fluoro-2-methoxyphenyl)-2-hydroxy-4-methyl-2-(1H-pyrrolo[2,3-c]pyridin-2-ylmethyl)pentanoic acid; 4-(5-fluoro-2-methoxyphenyl)-2-hydroxy-4-methyl-2-(1H-pyrrolo[2,3-c]pyridin-2-ylmethyl)pentanoic acid methyl ester; 2-cyclopropyl-4-(5-fluoro-2-methylphenyl)-4-methyl-1-(1H-pyrrolo[2,3-c]pyridin-2-yl)pentan-2-ol; 4-(5-chloro-2,3-dihydrobenzofuran-7-yl)-2-cyclopropyl-4-methyl-1-(1H-pyrrolo[2,3-c]pyridin-2-yl)pentan-2-ol; 2-cyclopropyl-4-(5-fluoro-2-methylphenyl)-4-methyl-1-(1H-pyrrolo[3,2-c]pyridin-2-yl)pentan-2-ol; 4-(5-chloro-2,3-dihydrobenzofuran-7-yl)-2-cyclopropyl-4-methyl-1-(1H-pyrrolo[3,2-c]pyridin-2-yl)pentan-2-ol; 4-(5-fluoro-2-methoxyphenyl)-2,4-dimethyl-1-(1H-pyrrolo[2,3-c]pyridin-2-yl)pentan-2-ol; 5-(5-fluoro-2-methoxyphenyl)-2,5-dimethyl-3-(1H-pyrrolo[2,3-c]pyridin-2-ylmethyl)hexan-3-ol; 5-(5-fluoro-2-methoxyphenyl)-2,2,5-trimethyl-3-(1H-pyrrolo[2,3-c]pyridin-2-ylmethyl)hexan-3-ol; 2-cyclohexyl-4-(5-fluoro-2-methoxyphenyl)-4-methyl-1-(1H-pyrrolo[2,3-c]pyridin-2-yl)pentan-2-ol; 2-cyclopentyl-4-(5-fluoro-2-methoxyphenyl)-4-methyl-1-(1H-pyrrolo[2,3-c]pyridin-2-yl)pentan-2-ol; 5-(5-fluoro-2-methoxyphenyl)-5-methyl-3-(1H-pyrrolo[2,3-c]pyridin-2-ylmethyl)hexan-3-ol; 2-(5-fluoro-2-methoxyphenyl)-2,6-dimethyl-4-(1H-pyrrolo[2,3-c]pyridin-2-ylmethyl)heptan-4-ol; 2-(5-fluoro-2-methoxyphenyl)-2,5,5-trimethyl-4-(1H-pyrrolo[2,3-c]pyridin-2-ylmethyl)heptan-4-ol; 1,1-difluoro-4-(5-fluoro-2-methoxyphenyl)-4-methyl-2-(1H-pyrrolo[2,3-c]pyridin-2-ylmethyl)pentan-2-ol; 1-cyclohexyl-4-(5-fluoro-2-methoxyphenyl)-4-methyl-2-(1H-pyrrolo[2,3-c]pyridin-2-ylmethyl)pentan-2-ol; 5-(5-fluoro-2-methylphenyl)-2,5-dimethyl-3-(1H-pyrrolo[2,3-c]pyridin-2-ylmethyl)hexan-3-ol; 5-(5-fuoro-2-methylphenyl- )-2,2,5-trimethyl-3-(1H-pyrrolo[2,3-c]pyridin-2-ylmethyl)hexan-3-ol; 5-(5-chloro-2,3-dihydrobenzofuran-7-yl)-2,5-dimethyl-3-(1 H-pyrrolo[2,3-c]pyridin-2-ylmethyl)hexan-3-ol; 2-cyclobutyl-4-(5-fluoro-2-methoxyphenyl)-4-methyl-1-(1H-pyrrolo[2,3-c]pyridin-2-yl)pentan-2-ol; 2-(5-fluoro-2-methoxyphenyl)-2,6,6-trimethyl-4-(1H-pyrrolo[2,3-c]pyridin-2-ylmethyl)heptan-4-ol; 5-(5-fluoro-2-methoxyphenyl)-5-methyl-3-(1H-pyrrolo[2,3-c]pyridin-2-ylmethyl)hex-1-en-3-ol; 5-(5-fluoro-2-methoxyphenyl)-5-methyl-3-(1H-pyrrolo[2,3-c]pyridin-2-ylmethyl)hex-1-yn-3-ol; 1-fluoro-4-(5-fluoro-2-methoxyphenyl)-4-methyl-2-(1H-pyrrolo[2,3-c]pyridin-2-ylmethyl)pentan-2-ol; 2,2-difluoro-5-(5-fluoro-2-methoxyphenyl)-5-methyl-3-(1H-pyrrolo[2,3-c]pyridin-2-ylmethyl)hexan-3-ol; 2-fluoro-5-(5-fluoro-2-methoxyphenyl)-2,5-dimethyl-3-(1H-pyrrolo[2,3-c]pyridin-2-ylmethyl)hexan-3-ol; 2-fluoro-5-(5-fluoro-2-methoxyphenyl)-5-methyl-3-(1H-pyrrolo[2,3-c]pyridin-2-ylmethyl)hexan-3-ol; 5-(5-fluoro-2-methoxyphenyl)-2,5-dimethyl-3-(1H-pyrrolo[2,3-c]pyridin-2-ylmethyl)hex-1-en-3-ol; 1,1,1-trifluoro-5-(5-fluoro-2-methoxyphenyl)-5-methyl-3-(1H-pyrrolo[2,3-c]pyridin-2-ylmethyl)hexan-3-ol; 4-(5-fluoro-2-methoxyphenyl)-4-methyl-2-phenyl-1-(1H-pyrrolo[2,3-c]pyridin-2-yl)pentan-2-ol; 5-(5-chloro-2,3-dihydrobenzofuran-7-yl)-2,2,5-trimethyl-3-(1H-pyrrolo[2,3-c]pyridin-2-ylmethyl)hexan-3-ol; 5-(5-fluoro-2-methylphenyl)-2,2,5-trimethyl-3-thieno[2,3-c]pyridin-2-ylmethylhexan-3-ol; 1,1-difluoro-4-(5-fluoro-2-methoxyphenyl)-4-methyl-2-(1H-pyrrolo[3,2-c]pyridin-2-ylmethyl)pentan-2-ol; 5-(5-fluoro-2-methoxyphenyl)-2,5-dimethyl-3-(1H-pyrrolo[3,2-c]pyridin-2-ylmethyl)hexan-3-ol; 5-(5-fluoro-2-methoxyphenyl)-2,2,5-trimethyl-3-(1H-pyrrolo[3,2-c]pyridin-2-ylmethyl)hexan-3-ol; 2-(1-fluorocyclopropyl)-4-(5-fluoro-2-methoxyphenyl)-4-methyl-1-(1H-pyrrolo[2,3-c]pyridin-2-yl)pentan-2-ol; 2-(1-fluorocyclopropyl)-4-(4-fluorophenyl)-4-methyl-1-quinolin-4-ylpentan-2-ol; 2-[4,4-difluoro-3-hydroxy-1,1-dimethyl-3-(1H-pyrrolo[3,2-c]pyridin-2-ylmethyl)butyl]-4-fluorophenol; 5-(5-chloro-2,3-dihydrobenzofuran-7-yl)-2,5-dimethyl-3-(1H-pyrrolo [3,2-c]pyridin-2-ylmethyl)hexan-3-ol; 5-(5-fluoro-2-methylphenyl)-2,5-dimethyl-3-(1H-pyrrolo[3,2-c]pyridin-2-ylmethyl)hexan-3-ol; 5-(5-fluoro-2-methylphenyl)-2,2,5-trimethyl-3-(1H-pyrrolo[3,2-c]pyridin-2-ylmethyl)hexan-3-ol; 4-(5-chloro-2,3-dihydrobenzofuran-7-yl)-1,1-difluoro-4-methyl-2-(1H-pyrrolo[3,2-c]pyridin-2-ylmethyl)pentan-2-ol; 4-(5-chloro-2,3-dihydrobenzofuran-7-yl)-1,1-difluoro-4-methyl-2-pyrrolo[3,2-b]pyridin-1-ylmethylpentan-2-ol; 5-(5-chloro-2,3-dihydrobenzofuran-7-yl)-2,2,5-trimethyl-3-(1H-pyrrolo[3,2-c]pyridin-2-ylmethyl)hexan-3-ol; 5-(5-fluoro-2-methylphenyl)-2,2,5-trimethyl-3-(3-methyl-1H-pyrrolo[2,3-c]pyridin-2-ylmethyl)hexan-3-ol; 5-(5-chloro-2,3-dihydrobenzofuran-7-yl)-2,5-dimethyl-3-(3-methyl-1H-pyrrolo[2,3-c]pyridin-2-ylmethyl)hexan-3-ol; 5-(5-chloro-2,3-dihydrobenzofuran-7-yl)-2,5-dimethyl-3-(5-phenyl-1H-pyrrolo[2,3-c]pyridin-2-ylmethyl)hexan-3-ol; 5-(5-fluoro-2-methylphenyl)-2,2,5-trimethyl-3-(5-phenyl-1H-pyrrolo[2,3-c]pyridin-2-ylmethyl)hexan-3-ol; 5-(5-fluoro-2-methylphenyl)-2,5-dimethyl-3-(5-phenyl-1H-pyrrolo[2,3-c]pyridin-2-ylmethyl)hexan-3-ol; 5-(5-fluoro-2-methylphenyl)-5-methyl-3-(5-phenyl-1H-pyrrolo[2,3-c]pyridin-2-ylmethyl)hexan-3-ol; 4-(5-fluoro-2-methylphenyl)-2,4-dimethyl-1-(5-phenyl-1H-pyrrolo[2,3-c]pyridin-2-yl)pentan-2-ol; 4-(5-chloro-2,3-dihydrobenzofuran-7-yl)-1,1-difluoro-4-methyl-2-(6-methyl-1H-pyrrolo[3,2-c]pyridin-2-ylmethyl)pentan-2-ol; 5-(5-fluoro-2-methylphenyl)-2,5-dimethyl-3-(5-pyridin-3-yl-1H-pyrrolo[2,3-c]pyridin-2-ylmethyl)hexan-3-ol; 5-(5-chloro-2,3-dihydrobenzofuran-7-yl)-5-methyl-3-(5-phenyl-1H-pyrrolo[2,3-c]pyridin-2-ylmethyl)hexan-3-ol; 4-(5-chloro-2,3-dihydrobenzofuran-7-yl)-2,4-dimethyl-1-(5-phenyl-1H-pyrrolo[2,3-c]pyridin-2-yl)pentan-2-ol; 1,1-difluoro-4-(5-methanesulfonyl-2,3-dihydrobenzofuran-7-yl)-4-methyl-2-(1H-pyrrolo[2,3-c]pyridin-2-ylmethyl)pentan-2-ol; 5-(5-chloro-2,3-dihydrobenzofuran-7-yl)-2,5-dimethyl-3-(5-pyridin-3-yl-1H-pyrrolo[2,3-c]pyridin-2-ylmethyl)hexan-3-ol; 2-(5-bromo-1H-indol-2-ylmethyl)-1,1-difluoro-4-(5-methanesulfonyl-2,3-dihydrobenzofuran-7-yl)-4-methylpentan-2-ol; and 2-[2-difluoromethyl-2-hydroxy-4-(5-methanesulfonyl-2,3-dihydrobenzofuran-7-yl)-4-methylpentyl]-4-methyl-1H-indole-6-carbonitrile.

For example, a DIGRA can have Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_8$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently $C_1$-$C_5$ alkyl, wherein one or both are independently substituted with hydroxy, $C_1$-$C_5$ alkoxy, $C_1$-$C_5$ alkylthio wherein the sulfur atom is optionally oxidized to a sulfoxide or sulfone, amino wherein the nitrogen atom is optionally independently mono- or di-substituted by $C_1$-$C_5$ alkyl or aryl;

(c) $R^3$ is hydrogen, $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q comprises a heteroaryl group optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein each substituent group of Q is optionally independently substituted with one to three substituent groups selected

from the group consisting of $C_1$-$C_3$ alkyl, $C_1$-$C_3$ alkoxy, acyl, $C_1$-$C_3$ silanyloxy, $C_1$-$C_5$ alkoxycarbonyl, carboxy, halogen, hydroxy, oxo, cyano, heteroaryl, heterocyclyl, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, or trifluoromethyl.

For example, a DIGRA can have Formula I, wherein

(a) A is an aryl, heteroaryl, heterocyclyl, or $C_3$-$C_8$ cycloalkyl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen, $C_1$-$C_5$ alkyl, $C_5$-$C_{15}$ arylalkyl, or $R^1$ and $R^2$ together with the carbon atom they are commonly attached to form a $C_3$-$C_8$ spiro cycloalkyl ring;

(c) B is the carbonyl group or methylene group, which is optionally independently substituted with one or two substituent groups selected from the group consisting of $C_1$-$C_3$ alkyl, hydroxy, and halogen;

(d) $R^3$ is the trifluoromethyl group;

(e) D is absent;

(f) E is the hydroxy group or amino group wherein the nitrogen atom is optionally independently mono- or di-substituted by $C_1$-$C_5$ alkyl; and

(g) Q comprises a 5- to 7-membered heterocyclyl ring fused to a 5- to 7-membered heteroaryl or heterocyclyl ring, each optionally independently substituted with one to three substituent groups, wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, oxo, cyano, trifluoromethyl, trifluoromethoxy, trifluoromethylthio, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, $C_1$-$C_3$ alkoxycarbonyl, acyl, aryl, benzyl, heteroaryl, heterocyclyl, halogen, hydroxy, oxo, cyano, amino wherein the nitrogen atom is optionally independently mono- or di-substituted by $C_1$-$C_5$ alkyl, and ureido wherein either nitrogen atom is optionally independently substituted with $C_1$-$C_5$ alkyl or trifluoromethyl, wherein Q cannot be 1H-[1,5]naphthyridin-4-one.

Examples of these compounds include 4-[4-(5-fluoro-2-methoxyphenyl)-2-hydroxy-4-methyl-2-trifluoromethylpentyl]-4H-thieno[3,2-b]pyridin-7-one; 4-[4-(5-fluoro-2-hydroxyphenyl)-2-hydroxy-4-methyl-2-trifluoromethylpenty-1]-4H-thieno[3,2-b]pyridin-7-one; 4-[4-(2,3-dihydrobenzofuran-7-yl)-2-hydroxy-4-methyl-2-trifluoromethylpentyl]-4H-thieno[3,2-b]pyridin-7-one; 1-[4-(5-fluoro-2-methoxyphenyl)-2-hydroxy-4-methyl-2-trifluoromethylpentyl]-1H-[1,6]naphthyridin-4-one; 1-[4-(5-fluoro-2-hydroxyphenyl)-2-hydroxy-4-methyl-2-trifluoromethylpentyl]-1H-[1,6]naphthyridin-4-one; 4-[4-(5-fluoro-2-methylphenyl)-2-hydroxy-4-methyl-2-trifluoromethylpentyl]-4H-thieno[3,2-b]pyridin-7-one; 4-[2-hydroxy-4-(5-methanesulfonyl-2,3-dihydrobenzofuran-7-yl)-4-methyl-2-trifluoromethylpentyl]-4H-thieno[3,2-b]pyridin-7-one; 1-[2-hydroxy-4-(5-methanesulfonyl-2,3-dihydrobenzofuran-7-yl)-4-methyl-2-trifluoromethylpentyl]-1H-[1,6]naphthyridin-4-one; 1-[4-(5-fluoro-2-methylphenyl)-2-hydroxy-4-methyl-2-trifluoromethylpentyl]-1H-[1,6]naphthyridin-4-one; 4-[2-hydroxy-4-(2-methoxy-3-methylphenyl)-4-methyl-2-trifluoromethylpentyl]-4H-thieno[3,2-b]pyridin-7-one; 4-[2-hydroxy-4-(2-methoxyphenyl)-4-methyl-2-trifluoromethylpentyl]-4H-thieno[3,2-b]pyridin-7-one; 4-[4-(3-bromo-2-methoxy-

phenyl)-2-hydroxy-4-methyl-2-trifluoromethylpentyl]-4H-thieno[3,2-b]pyridin-7-one; 4-[2-hydroxy-4-(2-hydroxy-3-methylphenyl)-4-methyl-2-trifluoromethylpentyl]-4H-thieno[3,2-b]pyridin-7-one; 4-[4-(3-bromo-2-hydroxyphenyl)-2-hydroxy-4-methyl-2-trifluoromethylpentyl]-4H-thieno[3,2-b]pyridin-7-one; 3-bromo-1-[4-(5-chloro-2,3-dihydrobenzofuran-7-yl)-2-hydroxy-4-methyl-2-trifluoromethylpentyl]-1H-[1,6]naphthyridin-4-one; 6-chloro-4-[4-(2,3-dihydrobenzofuran-7-yl)-2-hydroxy-4-methyl-2-trifluoromethylpentyl]-4H-thieno[3,2-b]pyridin-7-one; 6-bromo-4-[4-(2,3-dihydrobenzofuran-7-yl)-2-hydroxy-4-methyl-2-trifluoromethylpentyl]-4H-thieno[3,2-b]pyridin-7-one; 3-chloro-1-[4-(5-fluoro-2-hydroxyphenyl)-2-hydroxy-4-methyl-2-trifluoromethylpentyl]-1H-[1,6]naphthyridin-4-one; 1-[4-(5-chloro-2,3-dihydrobenzofuran-7-yl)-2-hydroxy-4-methyl-2-trifluoromethylpentyl]-3-methyl-1H-[1,6]naphthyridin-4-one; 1-[4-(5-Chloro-2,3-dihydrobenzofuran-7-yl)-2-hydroxy-4-methyl-2-trifluoromethylpentyl]-3-methyl-1H-[1,7]naphthyridin-4-one; 1-[2-hydroxy-4-(2-methoxy-3,5-dimethylphenyl)-4-methyl-2-trifluoromethylpentyl]-3-methyl-1H-[1,6]naphthyridin-4-one; 1-[2-hydroxy-4-(2-methoxy-3,5-dimethylphenyl)-4-methyl-2-trifluoromethylpentyl]-3-methyl-1H-[1,7]naphthyridin-4-one; 1-[2-hydroxy-4-(2-hydroxy-3,5-dimethylphenyl)-4-methyl-2-trifluoromethylpentyl]-3-methyl-1H-[1,6]naphthyridin-4-one; 1-[4-(5-fluoro-2-methylphenyl)-2-hydroxy-4-methyl-2-trifluoromethylpentyl]-1H-[1,8]naphthyridin-4-one; 1-[4-(5-fluoro-2-methylphenyl)-2-hydroxy-4-methyl-2-trifluoromethylpentyl]-1H-[1,7]naphthyridin-4-one; 4-[4-(5-fluoro-2-hydroxyphenyl)-2-hydroxy-4-methyl-2-trifluoromethylpenty- 1]-4H-thiazolo[4,5-b]pyridin-7-one; 4-[4-(5-fluoro-2-hydroxyphenyl)-2-hydroxy-4-methyl-2-trifluoromethylpentyl]-4H-oxazolo[4,5-b]pyridin-7-one; 4-[4-(5-fluoro-2-methylphenyl)-2-hydroxy-4-methyl-2-trifluoromethylpentyl]-4H-furo[3,2-b]pyridin-7-one; 7-[4-(5-fluoro-2-methylphenyl)-2-hydroxy-4-methyl-2-trifluoromethylpentyl]-7H-thieno[2,3-b]pyridin-4-one; 4-[4-(5-fluoro-2-hydroxyphenyl)-2-hydroxy-4-methyl-2-trifluoromethylpentyl]-4H-oxazolo[5,4-b]pyridin-7-one; 4-[4-(5-fluoro-2-hydroxyphenyl)-2-hydroxy-4-methyl-2-trifluoromethylpentyl]-4H-thiazolo [5,4-b]pyridin-7-one; 7-[4-(5-fluoro-2-methylphenyl)-2-hydroxy-4-methyl-2-trifluoromethylpentyl]-7H-furo[2,3-b]pyridin-4-one; 4-[4-(5-fluoro-2-methylphenyl)-2-hydroxy-4-methyl-2-trifluoromethylpentyl]-1,4-dihydropyrrolo[3,2-b]pyridin-7-one; 1-[4-(5-fluoro-2-hydroxyphenyl)-2-hydroxy-4-methyl-2-trifluoromethylpentyl]-5,6,7,8-tetrahydro-1H-[1,6]naphthyridin-4-one; 1-[4-(5-fluoro-2-methylphenyl)-2-hydroxy-4-methyl-2-trifluoromethylpentyl]-6-methyl-5,6,7,8-tetrahydro-1H-[1,6]naphthyridin-4-one; 1-[4-(2,3-dihydrobenzofuran-7-yl)-2-hydroxy-4-methyl-2-trifluoromethylpentyl]-1H-[1,8] naphthyridin-4-one; 1-[2-hydroxy-4-(5-methanesulfonyl-2,3-dihydrobenzofuran-7-yl)-4-methyl-2-trifluoromethylpentyl]-1H-[1,7]naphthyridin-4-one; 4-[2-hydroxy-4-(5-methanesulfonyl-2,3-dihydrobenzofuran-7-yl)-4-methyl-2-trifluoromethylpentyl]-4- H-thiazolo[4,5-b]pyridin-7-one; 4-[4-(2,3-dihydrobenzofuran-7-yl)-2-hydroxy-4-methyl-2-trifluoromethylpentyl]-4H-oxazolo[4,5-b]pyridin-7-one; 4-[2-hydroxy-4-(5-methanesulfonyl-2,3-dihydrobenzofuran-7-yl)-4-methyl-2-trifluoromethylpentyl]-4H-furo[3,2-b]pyridin-7-one; 7-[4-(2,3-dihydrobenzofuran-7-yl)-2-hydroxy-4-methyl-2-trifluoromethylpentyl]-7H-thieno[2,3-b]pyridin-4-one; 4-[2-hydroxy-4-(5-methanesulfonyl-2,3-dihydrobenzofuran-7-yl)-4-methyl-2-trifluoromethylpentyl]-4H-oxazolo[5,4-b]pyridin-7-one; 4-[2-hydroxy-4-(5-methanesulfonyl-2,3-dihydrobenzofuran-7-yl)-4-methyl-2-trifluoromethylpentyl]-4H-thiazolo[5,4-b]pyridin-7-one; 7-[4-(2,3-dihydrobenzofuran-7-yl)-2-hydroxy-4-methyl-2-trifluoromethylpentyl]-7H-furo[2,3-b]pyridin-4-one; 4-[4-(2,3-dihydrobenzofuran-7-yl)-2-hydroxy-4-methyl-2-trifluoromethylpentyl]-1,4-dihydropyrrolo[3,2-b]pyridin-7-one; 1-[2-hydroxy-4-(5-methanesulfonyl-2,3-dihydrobenzofuran-7-yl)-4-methyl-2-trifluoromethylpentyl]-5,6,7,8-tetrahydro-1H-[1,6]naphthyridin-4-one; 1-[4-(2,3-dihydrobenzofuran-7-yl)-2-hydroxy-4-methyl-2-trifluoromethylpentyl]-6-methyl-5,6,7,8-tetrahydro-1H-[1,6]naphthyridin-4-one; 1-[4-(2,3-dihydrobenzofuran-7-yl)-2-hydroxy-4-methyl-2-trifluoromethylpentyl]-5-methyl-5,6,7,8-tetrahydro-1H-[1,5]naphthyridin-4-one; 1-[4-(2,3-dihydrobenzofuran-7-yl)-2-hydroxy-4-methyl-2-trifluoromethylpentyl]-5-methyl-5,6,7,8-tetrahydro-1H-[1,5]naphthyridin-4-one; 4-[2-hydroxy-4-(4-methoxybiphenyl-3-yl)-4-methyl-2-trifluoromethylpentyl]-4H-thieno[3,2-b]pyridin-7-one; 4-[2-hydroxy-4-(2-methoxy-5-pyridin-3-ylphenyl)-4-methyl-2-trifluoromethylpentyl]-4H-thieno[3,2-b]pyridin-7-one; 4-[2-hydroxy-4-(2-methoxy-5-pyrimidin-5-ylphenyl)-4-methyl-2-trifluoromethylpentyl]-4H-thieno[3,2-b]pyridin-7-one; 4-[2-hydroxy-4-(2-methoxy-5-thiophen-3-ylphenyl)-4-methyl-2-trifluoromethylpentyl]-4H-thieno[3,2-b]pyridin-7-one; 4-[2-hydroxy-4-(4-hydroxybiphenyl-3-yl)-4-methyl-2-trifluoromethylpentyl]-4H-thieno[3,2-b]pyridin-7-one; 4-[2-hydroxy-4-(2-hydroxy-5-pyridin-3-ylphenyl)-4-methyl-2-trifluoromethylpentyl]-4H-thieno[3,2-b]pyridin-7-one; 4-[2-hydroxy-4-(2-hydroxy-5-pyrimidin-5-ylphenyl)-4-methyl-2-trifluoromethylpentyl]-4H-thieno[3,2-b]pyridin-7-one; 4-[2-Hydroxy-4-(2-hydroxy-5-thiophen-3-ylphenyl)-4-methyl-2-trifluoromethylpentyl]-4H-thieno[3,2-b]pyridin-7-one; 1-[2-hydroxy-4-(4-methoxybiphenyl-3-yl)-4-methyl-2-trifluoromethylpentyl]-1H-[1,6]naphthyridin-4-one; 1-[2-hydroxy-4-(2-methoxy-5-pyridin-3-ylphenyl)-4-methyl-2-trifluoromethylpentyl]-1H-[1,6]naphthyridin-4-one; 1-[2-hydroxy-4-(2-methoxy-5-pyrimidin-5-ylphenyl)-4-methyl-2-trifluoromethylpentyl]-1H-[1,6]naphthyridin-4-one; 1-[2-hydroxy-4-(2-methoxy-5-thiophen-3-ylphenyl)-4-methyl-2-trifluoromethylpentyl]-1H-[1,6]naphthyridin-4-one- ; 1-[2-hydroxy-4-(2-methoxy-5-thiophen-3-ylphenyl)-4-methyl-2-trifluoromethylpentyl]-1H-[1,6]naphthyridin-4-one; 1-[2-hydroxy-4-(2-hydroxy-5-pyridin-3-ylphenyl)-4-methyl-2-trifluoromethylpentyl]-1H-[1,6]naphthyridin-4-one; 1-[2-hydroxy-4-(2-hydroxy-5-pyrimidin-5-ylphenyl)-4-methyl-2-trifluoromethylpentyl]-1H-[1,6]naphthyridin-4-one; 1-[2-hydroxy-4-(2-hydroxy-5-thiophen-3-yphenyl)-4-methyl-2-trifluoromethylpentyl]-1H-[1,6]naphthyridin-4-one; 5-[4-(5-fluoro-2-methoxyphenyl)-2-hydroxy-4-methyl-2-trifluoromethylpentyl]-5H-pyrido[3,2-d]pyrimidin-8-one; 1-[4-(5-fluoro-2-methoxyphenyl)-2-hydroxy-4-methyl-2-trifluoromethylpentyl]-1H-pyrido[2,3-d]pyridazin-4-one; 5-[4-(5-fluoro-2-methoxyphenyl)-2-hydroxy-4-methyl-2-trifluoromethylpenty-1]-5H-pyrido[3,2-c]pyridazin-8-one; 4-[4-(2-fifluoromethoxy-3-methylphenyl- )-2-hydroxy-4-methyl-2-trifluoromethylpentyl]-4H-thieno[3,2-b]pyridin-7-one; 3-chloro-1-[4-(2,3-dihydrobenzofuran-7-yl)-2-hydroxy-4-methyl-2-trifluoromethylpentyl]-1H-[1,6]naphthyridin-4-one; 4-(4-benzo[1,3]dioxol-4-yl-2-hydroxy-4-methyl-2-trifluoromethylpentyl)-6-bromo-4H-thieno[3,2-b]pyridin-7-one; 4-(4-benzo[1,3]dioxol-4-yl-2-hydroxy-4-methyl-2-trifluoromethylpentyl)-6-chloro-4H-thieno[3,2-b]pyridin-7-one; 6-chloro-4-[2-hydroxy-4-methyl-4-(5-pyridin-3-yl-2,3-dihydrobenzofuran-7-yl)-2-trifluoromethylpentyl]-4H-thieno[3,2-b]pyridin-7-one; 1-(4-benzo[1,3]dioxol-4-yl-2-hydroxy-4-methyl-2-trifluoromethylpentyl)-3-chloro-1H-[1,6]naphthyridin-4-one; 6-chloro-4-[2-hydroxy-4-methyl-4-(5-pyrimidin-5-yl-2,3-dihydrobenzofuran-7-yl)-2-trifluoromethylpentyl]-4H-thieno[3,2-b]pyridin-7-one; 3-chloro-1-[2-hydroxy-4-methyl-4-(5-pyrimidin-5-yl-2,3-dihydrobenzofuran-7-yl)-2-trifluoromethylpentyl]-1H-[1,6]naphthyridin-4-one; 3-chloro-1-[2-hydroxy-4-methyl-4-(5-pyridin-3-yl-2,3-dihydrobenzofuran-7-yl)-2-trifluoromethylpentyl]-1H-[1,6]naphthyridin-4-one; 4-[2-hydroxy-4-methyl-4-(5-pyrimidin-5-yl-2,3-dihydrobenzofuran-7-yl)-2-trifluoromethylpentyl]-4H-thieno[3,2-b]pyridin-7-one; 1-[2-hydroxy-4-methyl-4-(5-pyrimidin-5-yl-2,3-dihydrobenzofuran-7-yl)-2-trifluoromethylpentyl]-1H-[1,6]naphthyridin-4-one; 6-chloro-4-[2-hydroxy-4-(2-methoxy-5-pyridin-3-ylphenyl)-4-methyl-2-trifluoromethylpentyl]-4H-thieno[3,2-b]pyridin-7-one; 6-chloro-4-[2-hydroxy-4-(2-methoxy-5-pyrimidin-5-ylphenyl)-4-methyl-2-trifluoromethylpentyl]-4H-thieno[3,2-b]pyridin-7-one; 6-chloro-4-[2-hydroxy-4-(2-hydroxy-5-pyridin-3-ylphenyl)-4-methyl-2-trifluoromethylpentyl]-4H-thieno[3,2-b]pyridin-7-one; 6-chloro-4-[2-hydroxy-4-(-2-hydroxy-5-pyrimidin-5-ylphenyl)-4-methyl-2-trifluoromethylpentyl]-4H-thieno[3,2-b]pyridin-7-one; 4-(4-biphenyl-3-yl-2-hydroxy-4-methyl-2-trifluoro-methylpentyl)-6-chloro-4H-thieno[3,2-b]pyridin-7-one; 4-(4-biphenyl-3-yl-2-hydroxy-4-methyl-2-trifluoromethylpentyl)-4H-thieno[3,2-b]pyridin-7-one; 3-chloro-1-{4-[5-(5-chloropyridin-3-yl)-2,3-dihydrobenzofuran-7-yl]-2-hydroxy-4-methyl-2-trifluoromethylpentyl}-1H-[1,6]naphthyridin-4-one; 6-chloro-4-{4-[5-(2,6-dimethylpyridin-4-yl)-2-methoxyphenyl]-2-hydroxy-4-methyl-2-trifluoromethylpentyl}-4H-thieno[3,2-b]pyridin-7-one- ; 4-[2-hydroxy-4-(2-hydroxy-5-pyridin-2-ylphenyl)-4-methyl-2-trifluoromethylpentyl]-4H-thieno[3,2-b]pyridin-7-one; 6-chloro-4-[2-hydroxy-4-methyl-4-(5-pyrazin-2-yl-2,3-dihydrobenzofuran-7-yl)-2-trifluoromethylpentyl]-4H-thieno[3,2-b]pyridin-7-one; 3-chloro-1-[2-hydroxy-4-methyl-4-(5-pyrimidin-2-yl-2,3-dihydrobenzofuran-7-yl)-2-trifluoromethylpentyl]-1H-[1,6]naphthyridin-4-one; 5-{7-[3-(6-chloro-7-oxo-7H-thieno[3,2-b]pyridin-4-ylmethyl)-4,4,- 4-trifluoro-3-hydroxy-1,1-dimethylbutyl]-2,3-dihydrobenzofuran-5-yl}nicotinonitrile; 4-{4-Methoxy-3-[4,4,4-trifluoro-3-hydroxy-l,1-dimethyl-3-(7-oxo-7H-thieno[3,2-b]pyridin-4-ylmethyl)butyl]phenyl}pyridine-2-carbonitrile; 6-chloro-4-{4-[5-(2-fluoro-6-methylpyridin-4-yl)-2-methoxyphenyl]-2-hydroxy-4-methyl-2-trifluoromethylpentyl}-4H-thieno[3,2-b]pyridin-7-one; 3-chloro-1-{2-hydroxy-4-[5-(1H-imidazol-4-yl)-2,3-dihydrobenzofuran-7-yl]-4-methyl-2-trifluoromethylpentyl}-1H-[1,6]naphthyridin-4-one; 6-chloro-4-[2-hydroxy-4-methyl-4-(5-morpholin-4-yl-2,3-dihydrobenzofuran-7-yl)-2-trifluoromethylpentyl]-4H-thieno[3,2-b]pyridin-7-one; and 1-[2-hydroxy-4-methyl-4-(5-piperidin-1-yl-2,3-dihydrobenzofuran-7-yl)-2-trifluoromethylpentyl]-1H-[1,6]naphthyridin-4-one.

For example, a DIGRA can have Formula I, wherein A, B, D, E, $R^1$, and $R^2$ have the meanings disclosed immediately above, and $R^3$ is hydrogen, $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein $R^3$ cannot be trifluoromethyl.

For example, a DIGRA can have Formula I, wherein

(a) A is an aryl, heteroaryl, heterocyclyl, or $C_3$-$C_8$ cycloalkyl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaminosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl;

(c) $R^3$ is the trifluoromethyl group;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q comprises an indolyl group optionally substituted with one to three substituent groups, wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaminosulfonyl, halogen, hydroxy, carboxy, cyano, trifluoromethyl, trifluoromethoxy, trifluoromethylthio, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_{1-}$ $C_3$ alkyl, $C_1$-$C_3$ alkoxy, halogen, hydroxy, oxo, cyano, amino, and trifluoromethyl.

Examples of these compounds include 4-(5-bromo-2,3-dihydrobenzofuran-7-yl)-1,1,1-trifluoro-2-(1 H-indol-2-ylmethyl)-4-methylpentan-2-ol; 1,1,1-trifluoro-2-(1H-indol-2-ylmethyl)-4-methyl-4-pyridin-2-ylpentan-2-ol; 4-(2,3-dihydro-5-cyanobenzofuran-7-yl)-1,1,1-trifluoro-2-(1H-indol-2-yl-methyl)-4-methylpentan-2-ol; 4-(2,3-dihydrobenzofuran-7-yl)-1,1,1-trifluoro-2-(1H-indol-2-ylmethyl)-4-methylpentan-2-ol; 1,1,1-trifluoro-4-(5-fluoro-2,3-dihydrobenzofuran-7-yl)-2-(1H-indol-2-ylmethyl)-4-methylpentan-2-ol; 1,1,1-trifluoro-2-(1H-indol-2-ylmethyl)-4-methyl-4-(5-methyl-2,3-dihydrobenzofuran-7-yl)pentan-2-ol; 4-(2,3-dihydrobenzofuran-5-yl)-1,1,1-trifluoro-2-(1H-indol-2-ylmethyl)-4-methylpentan-2-ol; 2-[4-(2,3-dihydrobenzofuran-7-yl)-2-hydroxy-4-methyl-2-trifluoromethylpentyl]-1H-indole-3-carbonitrile; 2-[4-(5-fluoro-2,3-dihydrobenzofuran-7-yl)-2-hydroxy-4-methyl-2-trifluoromethylpentyl]-1H-indole-3-carbonitrile; 2-[4-(5-bromo-2,3-dihydrobenzofuran-7-yl)-2-hydroxy-4-methyl-2-trifluoromethylpentyl]-1H-indole-3-carbonitrile; 2-[4-(2,3-dihydrobenzofuran-7-yl)-2-hydroxy-4-methyl-2-trifluoromethylpentyl]-4-methyl-1H-indole-6-carbonitrile; 2-[4-(2,3-dihydrobenzofuran-7-yl)-2-hydroxy-4-methyl-2-trifluoromethylpentyl]-1H-indole-5-carbonitrile; 4-(2,3-dihydrobenzofuran-7-yl)-1,1,1-trifluoro-2-(7-fluoro-1H-indol-2-ylmethyl)4-methylpentan-2-ol; 1-[4-(2,3-dihydrobenzofuran-7-yl)-2-hydroxy-4-methyl-2-trifluoromethylpentyl]-1H-indo le-3-carbonitrile; 4-(2,3-dihydrobenzofuran-7-yl)-1,1,1-trifluoro-4-methyl-2-(5-trifluoromethyl-1H-indol-2-ylmethyl)pentan-2-ol; and 1,1,1-trifluoro-2-(1H-indol-2-ylmethyl)-4-methyl-4-thiophen-3-ylpentan-2-ol.

For example, a DIGRA can have Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) $R^3$ is carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl,

nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(d) B is the methylene or carbonyl group;

(e) D is the -NH- group;

(f) E is the hydroxy group; and

(g) Q comprises the group

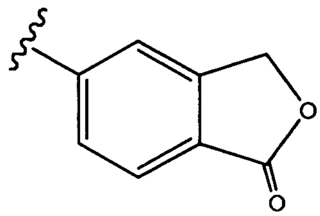

.

Examples of these compounds include 2-benzyl-2-hydroxy-4-methyl-4-phenylpentanoic acid (1-oxo-1,3-dihydroisobenzofuran-5-yl)amide; 2-hydroxy-4-methyl-2,4-diphenylpentanoic acid (1-oxo-1,3-dihydroisobenzofuran-5-yl) amide; 2-hydroxy-4-methyl-2-phenethyl-4-phenylpentanoic acid (1-oxo-1,3-dihydroisobenzofuran-5-yl)amide; 2-hydroxy-2-(3-methoxybenzyl)$_4$-methyl-4-phenylpentanoic acid (1-oxo-1,3-dihydroisobenzofuran-5-yl)amide; 2-hydroxy-2-(4-methoxybenzyl)-4-methyl-4-phenylpentanoic acid (1-oxo-1,3-dihydroisobenzofuran-5-yl)amide; 2-hydroxy-2-[2-(4-methoxyphenyl)ethyl]4-methyl-4-phenylpentanoic acid (1-oxo-1,3-dihydroisobenzofuran-5-yl)amide; 2-cyclohexylmethyl-2-hydroxy-4-methyl-4-phenylpentanoic acid (1-oxo-1,3-dihydroisobenzofuran-5-yl)amide; 2-(4-tert-butylbenzyl)-2-hydroxy-4-methyl-4-phenylpentanoic acid (1-oxo-1,3-dihydroisobenzofuran-5-yl)amide; 2-biphenyl-4-ylmethyl-2-hydroxy-4-methyl-4-phenylpentanoic acid (1-oxo-1,3-dihydroisobenzofuran-5-yl)amide; 2-hydroxy-4-methyl-2-naphthalen-2-ylmethyl-4-phenylpentanoic acid (1-oxo-1,3-dihydroisobenzofuran-5-yl)amide; 2-hydroxy-2-(3-hydroxybenzyl)-4-methyl-4-phenylpentanoic acid (1-oxo-1,3-dihydroisobenzofuran-5-yl)amide; 2-hydroxy-4-methyl-2-(2-methyl-2-phenylpropyl)-4-phenylpentanoic acid (1-oxo-1,3-dihydroisobenzofuran-5-yl)amide; 2-benzyl-4-(5-fluoro-2-methoxyphenyl)-2-hydroxy-4-methylpentanoic acid (1-oxo-1,3-dihydroisobenzofuran-5-yl)amide; 2-cyclohexylmethyl-4-(5-fluoro-2-methoxyphenyl)-2-hydroxy-4-methylpentanoic acid (1-oxo-1,3-dihydroisobenzofuran-5-yl)amide; 2-benzyl-4-(5-fluoro-2-hydroxyphenyl)-2-hydroxy-4-methylpentanoic acid (1-oxo-1,3-dihydroisobenzofuran-5-yl)amide; 2-cyclohexylmethyl-4-(5-fluoro-2-hydroxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-4-methyl-2-(2-methyl-2-phenylpropyl)pentanoic acid (1-oxo-1,3-dihydroisobenzofuran-5-yl) amide; 2-(2-chloro-6-fluorobenzyl)-4-(5-fluoro-2-methoxyphenyl)-2-hydroxy-4-methylpentanoic acid (1-oxo-1,3-dihydroisobenzofuran-5-yl)amide; 2-(3-fluorobenzyl)-4-(5-fluoro-2-methoxyphenyl)-2-hydroxy-4-methylpentanoic acid (1-oxo-1,3-dihydroisobenzofuran-5-yl)amide; 2-(2-fluorobenzyl)-4-(5-fluoro-2-methoxyphenyl)-2-hydroxy-4-methylpentanoic acid (1-oxo-1,3-dihydroisobenzofuran-5-yl)amide; 2-(3,4-difluorobenzyl)-4-(5-fluoro-2-methoxyphenyl)-2-hydroxy-4-methylpentanoic acid (1-oxo-1,3-dihydroisobenzofuran-5-yl)amide; 2-(2-chloro-6-fluorobenzyl)-4-(5-fluoro-2-hydroxyphenyl)-2-hydroxy-4-methylpentanoic acid (1-oxo-1,3-dihydroisobenzofuran-5-yl)amide; 2-(3-fluorobenzyl)-4-(5-fluoro-2-hydroxyphenyl)-2-hydroxy-4-methylpentanoic acid (1-oxo-1,3-dihydroisobenzofuran-5-yl)amide; 2-(2-fluorobenzyl)-4-(5-fluoro-2-hydroxyphenyl)-2-hydroxy-4-methylpentanoic acid (1-oxo-1,3-dihydroisobenzofuran-5-yl)amide; 2-(3,4-difluorobenzyl)-4-(5-fluoro-2-hydroxyphenyl)-2-hydroxy-4-methylpentanoic acid (1-oxo-1,3-dihydroisobenzofuran-5-yl) amide; 2-(4-fluorobenzyl)-4-(5-fluoro-2-methoxyphenyl)-2-hydroxy-4-methylpentanoic acid (1-oxo-1,3-dihydroisobenzofuran-5-yl)amide; 4-(5-fluoro-2-methoxyphenyl)-2-hydroxy-4-methyl-2-(3-methylbenzyl)pentanoic acid (1-oxo-1,3-dihydroisobenzofuran-5-yl)amide; 2-(4-fluorobenzyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-4-methyl-2-(3-methylbenzyl)pentanoic acid (1-oxo-1,3-dihydroisobenzofuran-5-yl)amide; 2-(3,5-difluorophenyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-methoxyphenyl)-2-hydroxy-4-methyl-2-(2-methylbenzyl)pentanoic acid (1-oxo-1,3-dihydroisobenzofuran-5-yl)amide; 2-(3,5-dimethylbenzyl)-4-(5-fluoro-2-methoxyphenyl)-2-hydroxy-4-methylpentanoic acid (1-oxo-1,3-dihydroisobenzofuran-5-yl)amide; 2-(2,5-difluorobenzyl)-4-(5-fluoro-2-methoxyphenyl)-2-hydroxy-4-methylpentanoic acid (1-oxo-1,3-dihydroisobenzofuran-5-yl)amide; 2-(2,5-difluorobenzyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-4-methyl-2-(2-methylbenzyl)pentanoic acid (1-oxo-1,3-dihydroisobenzofuran-5-yl)amide; 2-(3,5-dimethylbenzyl)-4-(5-fluoro-2-hydroxyphenyl)-2-hydroxy-4-methylpentanoic acid (1-oxo-1,3-dihydroisobenzofuran-5-yl)amide; 2-(3-chlorobenzyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-methoxyphenyl)-2-hydroxy-2-[2-(4-methoxyphenyl)ethyl]-4-methylpentanoic acid (1-oxo-1,3-dihydroisobenzofuran-5-yl)amide; 4-(5-fluoro-2-methoxyphenyl)-2-hydroxy-2-(2-methoxybenzyl)$_4$-methylpentanoic acid (1-oxo-1,3-dihydroisobenzofuran-5-yl)amide; 4-(5-fluoro-2-methoxyphenyl)-2-hydroxy-4-methyl-2-phenethylpentanoic acid (1-oxo-1,3-dihydroisobenzofuran-5-yl)amide; 2-(2-chlorobenzyl)-4-(5-fluoro-2-methoxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-4-methyl-2-phenethylpentanoic acid (1-oxo-1,3-dihydroisobenzofuran-5-yl) amide; 4-(5-fluoro-2-hydroxyphenyl)-2-hydroxy-2-[2-(4-hydroxyphenyl)ethyl]-4-methylpentanoic acid (1-oxo-1,3-dihydroisobenzofuran-5-yl)amide; 2-(2-chlorobenzyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-2-(2-hydroxybenzyl)-4-methylpentanoic acid (1-oxo-1,3-dihydroisobenzofuran-5-yl)amide; 2-(2-bromobenzyl)-4-(5-fluoro-2-methoxyphenyl)-2-hydroxy-4-methylpentanoic acid (1-oxo-1,3-dihydroisobenzofuran-5-yl)amide; 2-(2-bromobenzyl)-4-(5-fluoro-2-hydroxyphenyl)-2-hydroxy-4-methylpentanoic acid (1-oxo-1,3-dihydroisobenzofuran-5-yl)amide; 2-(5-fluoro-2-methoxybenzyl)-2-hydroxy-4-methyl-4-phenylpentanoic acid (1-oxo-1,3-dihydroisobenzofuran-5-yl)amide; 2-(5-fluoro-2-hydroxybenzyl)-2-hydroxy-4-methyl-4-phenylpentanoic acid (1-oxo-1,3-dihydroisobenzofuran-5-yl)amide; 2-(5-fluoro-2-methoxybenzyl)-4-(5-fluoro-2-methoxyphenyl)-2-hydroxy-4-methylpentanoic acid (1-oxo-1,3-dihydroisobenzofuran-5-yl)amide; 2-(5-fluoro-2-hydroxybenzyl)-4-(5-fluoro-2-hydroxyphenyl)-2-hydroxy-4-methylpentanoic acid (1-oxo-1,3-dihydroisobenzofuran-5-yl) amide; 2-(3,5-dimethoxybenzyl)-2-hydroxy-4-methyl-4-phenylpentanoic acid (1-oxo-1,3-dihydroisobenzofuran-5-yl) amide; 2-(3,5-dihydroxybenzyl)-2-hydroxy-4-methyl-4-phenylpentanoic acid (1-oxo-1,3-dihydroisobenzofuran-5-yl)-amide; 2-hydroxy-2-(2-methoxybenzyl)$_4$-methyl-4-phenylpentanoic acid (1-oxo-1,3-dihydroisobenzofuran-5-yl)amide; 12-hydroxy-2-(2-hydroxybenzyl)-4-methyl-4-phenylpentanoic acid (1-oxo-1,3-dihydroisobenzofuran-5-yl)amide; 2-hydroxy-2-[2-(4-hydroxyphenyl)ethyl]-4-methyl-4-phenylpentanoic acid (1-oxo-1,3-dihydroisobenzofuran-5-yl)amide; 15-[2-benzyl-4-(5-fluoro-2-methoxyphenyl)-2-hydroxy-4-methylpentylamino]-3H-isobenzofuran-1-one; 4-(5-fluoro-2-methoxyphenyl)-2-hydroxy-4-methyl-2-(1-phenylvinyl)pentanoic acid (1-oxo-1,3-dihydroisobenzofuran-5-yl)amide; 2-hydroxy-4-methyl-4-phenyl-2-pyridin-2-ylmethylpentanoic acid(1-oxo-1,3-dihydroisobenzofuran-5-yl)amide; 4-(5-fluoro-2-methoxyphenyl)-2-hydroxy-4-methyl-2-(1-phenylethyl-)pentanoic acid(1-oxo-1,3-dihydroisobenzofuran-5-yl)amide; 4-(5-fluoro-2-hydroxyphenyl)-2-hydroxy-4-methyl-2-(1-phenylethyl)pentanoic acid(1-oxo-1,3-dihydroisobenzofuran-5-yl)amide; 2-cyclopentyl-4-(5-fluoro-2-methoxyphenyl)-2-hydroxy-4-methylpentanoic acid(1-oxo-1,3-dihydroisobenzofuran-5-yl)amide; 2-cyclopentyl-4-(5-fluoro-2-hydroxyphenyl)-2-hydroxy-4-methylpentanoic acid(1-oxo-1,3-dihydroisobenzofuran-5-yl)amide; 2-cyclopentylmethyl-4-(5-fluoro-2-hydroxyphenyl)-2-hydroxy-4-methylpentanoic acid(1-oxo-1,3-dihydroisobenzofuran-5-yl)amide; and 2-benzyl-2-hydroxy-N-(1-oxo-1,3-dihydroisobenzofuran-5-yl)$_4$-phenyl-butyramide.

For example, a DIGRA can have Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) $R^3$ is the trifluoromethyl group;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is $-NR^6R^7$, wherein $R^6$ and $R^7$ are each independently hydrogen, $C_1$-$C_8$ alkyl, $C_2$-$C_8$ alkenyl, $C_2$-$C_8$ alkynyl,

$C_1$-$C_8$ alkoxy, $C_2$-$C_8$ alkenyloxy, $C_2$-$C_8$ alkynyloxy, hydroxy, carbocyclyl, heterocyclyl, aryl, aryloxy, acyl, heteroaryl, carbocycle-$C_1$-$C_8$ alkyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, heteroaryl-$C_2$-$C_8$ alkenyl, or $C_1$-$C_5$ alkylthio wherein the sulfur atom is oxidized to a sulfoxide or sulfone, each optionally independently substituted with one to three substituent groups, wherein each substituent group of $R^6$ and $R^7$ are independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyl, $C_1$-$C_5$ alkylaminocarbonyl, $C_1$-$C_5$ dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaminosulfonyl, halogen, hydroxy, carboxy, cyano, oxo, trifluoromethyl, trifluoromethoxy, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone; and

(g) Q comprises a heteroaryl group optionally independently substituted with one to three substituent groups, wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, $C_1$-$C_5$ alkylaminocarbonyl, $C_1$-$C_5$ dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaminosulfonyl, halogen, hydroxy, carboxy, cyano, trifluoromethyl, trifluoromethoxy, trifluoromethylthio, nitro, or amino wherein the nitrogen atom is optionally independently mono- or di-substituted by $C_1$-$C_5$ alkyl; or ureido wherein either nitrogen atom is optionally independently substituted with $C_1$-$C_5$ alkyl; or $C_1$-$C_5$ alkylthio wherein the sulfur atom is optionally oxidized to a sulfoxide or sulfone, wherein each substituent group of Q is optionally independently substituted with one to three substituent groups selected from $C_1$-$C_3$ alkyl, $C_1$-$C_3$ alkoxy, halogen, hydroxy, oxo, cyano, amino, or trifluoromethyl.

Examples of these compounds include 3-(5-fluoro-2-methoxyphenyl)-3-methyl-1-(pyridin-2-ylmethyl)-1-trifluoromethyl-butylamine; 3-(5-fluoro-2-methoxy-phenyl)-1-(1H-indol-2-ylmethyl)-3-methyl-1-trifluoromethyl-butylamine; 1-(2,6-dichloro-pyridin-4-ylmethyl)-3-(5-fluoro-2-methoxy-phenyl)-3-methyl-1-trifluoromethyl-butylamine; 1-(4,6-dimethyl-pyridin-2-ylmethyl)-3-(5-fluoro-2-methoxy-phenyl)-3-methyl-1-trifluoromethyl-butylamine; 1-(2-chloro-pyridin-4-ylmethyl)-3-(5-fluoro-2-methoxy-phenyl)-3-methyl-1-trifluoromethyl-butylamine; 3-(5-fluoro-2-methyl-phenyl)-3-methyl-1-(3-methyl-1H-indol-2-ylmethyl)-1-trifluoromethyl-butylamine; 3-(5-fluoro-2-methoxy-phenyl)-3-methyl-1-(3-methyl-1H-indol-2-ylmethyl)-1-trifluoromethyl-butylamine; 1-(6-fluoro-1H-indol-2-ylmethyl)-3-(5-fluoro-2-methoxy-phenyl)-3-methyl-1-trifluoromethyl-butylamine; 3-(4-fluoro-phenyl)-3-methyl-1-(3-methyl-1H-indol-2-ylmethyl)-1-trifluoro-methyl-butylamine; 3-benzofuran-7-yl-1-(2,6-dichloro-pyridin-4-ylmethyl)-3-methyl-1-trifluoromethyl-butylamine; 3-(2,3-dihydro-benzofuran-7-yl)-1-(6-fluoro-1H-indol-2-ylmethyl)-3-methyl-1-trifluoromethyl-butylamine; 3-(5-fluoro-2-methoxy-phenyl)-3-methyl-1-quinolin-4-ylmethyl-1-trifluoromethyl-butylamine; 1-(2-chloro-quinolin-4-ylmethyl)-3-(5-fluoro-2-methylphenyl)-3-methyl-1-trifluoromethyl-butylamine; 3-(4-fluoro-phenyl)-3-methyl-1-quinolin-4-ylmethyl-1-trifluoromethyl-butylamine; 7-[3-amino-3-(1H-benzoimidazol-2-ylmethyl)-4,4,4-trifluoro-1,1-dimethyl-butyl]-2,3-dihydrobenzofuran-5-carbonitrile; 1-(6-fluoro-1H-benzoimidazol-2-ylmethyl)-3-(5-fluoro-2-methyl-phenyl)-3-methyl-1-trifluoromethyl-butylamine; 2-[3-amino-3-(1H-benzoimidazol-2-ylmethyl)-4,4,4-trifluoro-1,1-dimethyl-butyl]4-fluoro-phenol; 1-(1H-benzoimidazol-2-ylmethyl)-3-(4-fluoro-phenyl)-3-methyl-1-trifluoromethyl-butylamine; 1-(1H-indol-2-ylmethyl)-3-methyl-3-pyridin-3-yl-1-trifluoromethyl-butylamine; 1-(1H-benzoimidazol-2-ylmethyl)-3-methyl-3-pyridin-4-yl-1-trifluoromethyl-butylamine; 3-methyl-1-(3-methyl-1H-indol-2-ylmethyl)-3-pyridin-3-yl-1-trifluoromethyl-butylamine; 1-(6-fluoro-1H-indol-2-ylmethyl)-3-methyl-3-pyridin-3-yl-1-trifluoromethyl-butylamine; 3-(2,3-dihydrobenzofuran-7-yl)-1-(1H-indol-2-ylmethyl)-3-methyl-1-trifluoromethyl-butylamine; [3-(5-fluoro-2-methoxy-phenyl)-3-methyl-1-quinolin-4-ylmethyl-1-trifluoromethyl-butyl]-methyl-amine; ethyl-[3-(5-fluoro-2-methoxy-phenyl)-3-methyl-1-quinolin-4-ylmethyl-1-trifluoromethyl-butyl]-amine; [3-(5-fluoro-2-methoxy-phenyl)-3-methyl-1-quinolin-4-ylmethyl-1-trifluoromethyl-butyl]-propylamine; [3-(5-fluoro-2-methoxy-phenyl)-3-methyl-1-quinolin-4-ylmethyl-1-trifluoromethyl-butyl]-isobutylamine; butyl-[3-(5-fluoro-2-methoxy-phenyl)-3-methyl-1-quinolin-4-ylmethyl-1-trifluoromethyl-butyl]-amine; [3-(5-fluoro-2-methoxy-phenyl)-3-methyl-1-quinolin-4-ylmethyl-1-trifluoromethyl-butyl]-dimethylamine; N-[3-(5-fluoro-2-methoxy-phenyl)-3-methyl-1-quinolin-4-ylmethyl-1-trifluoromethyl-butyl]-acetamide; N-[3-(5-fluoro-2-methoxy-phenyl)-3-methyl-1-quinolin-4-ylmethyl-1-trifluoromethyl-butyl]-formamide; N-[3-(5-fluoro-2-methoxy-phenyl)-3-methyl-1-quinolin-4-ylmethyl-1-trifluoromethyl-butyl]-methanesulfonamide; 1-(2,6-dimethyl-pyridin-4-ylmethyl)-3-(5-fluoro-2-methoxyphenyl)-3-methyl-1-trifluoromethyl-butylamine; 3-(5-fluoro-2-methoxy-phenyl)-3-methyl-1-(1H-pyrrolo[2,3-c]pyridin-2-ylmethyl)-1-trifluoromethyl-butylamine; 2-[2-amino-4-(5-fluoro-2-methoxy-phenyl)-4-methyl-2-trifluoromethyl-pentyl]-4-methyl-1 H-indole-6-carbonitrile; N-[3-(5-fluoro-2-methoxy-phenyl)-3-methyl-1-quinolin-4-ylmethyl-1-trifluoromethyl-butyl]-hydroxylamine; and 2-(3-amino-4,4,4-trifluoro-1,1-dimethyl-3-quino lin-4-ylmethyl-butyl)-4-fluoro-phenol.

For example, a DIGRA can have Formula I, wherein A, B, D, E, $R^1$, $R^2$, $R^6$, and $R^7$ have the meanings disclosed

immediately above, and $R^3$ is $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein $R^3$ cannot be trifluoromethyl.

Examples of these compounds include 1-(2,6-dichloro-pyridin-4-ylmethyl)-3-(5-fluoro-2-methoxy-phenyl)-1,3-dimethyl-butylamine; 1-ethyl-3-(5-fluoro-2-methoxy-phenyl)-3-methyl-1-quinolin-4-ylmethyl-butylamine; 1-cyclohexylmethyl-3-(5-fluoro-2-methoxy-phenyl)-1-(1H-indol-2-ylmethyl)-3-methylbutylamine; 1-(2-chloro-quinolin-4-ylmethyl)-1-cyclopentyl-3-(5-fluoro-2-methoxyphenyl)-3-methyl-butylamine; 1-(2-chloro-pyridin-4-ylmethyl)-1-cyclopentylmethyl-3-(5-fluoro-2-methoxy-phenyl)-3-methyl-butylamine; 3-(5-fluoro-2-methoxy-phenyl)-1,3-dimethyl-1-quinolin-4-ylmethyl-butylamine; 1-cyclopropyl-3-(5-fluoro-2-methoxyphenyl)-3-methyl-1-quinolin-4-ylmethyl-butylamine; 3-(5-fluoro-2-methoxy-phenyl)-1,3-dimethyl-1-(1H-pyrrolo[2,3-c]pyridin-2-ylmethyl)-butylamine; 1-cyclopropyl-3-(5-fluoro-2-methoxy-phenyl)-3-methyl-1-(1H-pyrrolo[2,3-c]-pyridin-2-ylmethyl)-butylamine; 2-[3-amino-1,1,3-trimethyl-4-(1H-pyrrolo [2,3-c]pyridin-2-yl)-butyl]-4-fluoro-phenol; 2-[2-amino-4-(5-fluoro-2-methoxy-phenyl)-2,4-dimethyl-pentyl]-4-methyl-1H-indo le-6-carbonitrile.

Other compounds that can function as DIGRAs and methods for their manufacture are disclosed, for example, in U.S. Patent Application Publications 2004/0029932, 2004/0162321, 2004/0224992, 2005/0059714, 2005/0176706, 2005/0203128, 2005/0234091, 2005/0282881, 2006/0014787, 2006/0030561, 2006/0116396, 2006/0189646, and 2006/0189647.

In another aspect of the present disclosure an ophthalmic pharmaceutical composition for treating or controlling an anterior-segment infection and its inflammatory sequelae is provided. In one embodiment, such inflammatory sequelae comprise acute inflammation. In another embodiment of the present disclosure, such inflammatory sequelae comprise chronic inflammation of the anterior segment. The ophthalmic pharmaceutical compositions can comprises: (a) at least a DIGRA, a prodrug thereof, or a pharmaceutically acceptable salt thereof; and (b) an anti-infective agent. In one aspect of the present disclosure, the pharmaceutical composition further comprises a pharmaceutically acceptable carrier.

The concentration of a DIGRA, a prodrug thereof, or a pharmaceutically acceptable salt thereof in such an ophthalmic composition can be in the range from about 0.0001 to about 1000 mg/ml (or, alternatively, from about 0.001 to about 500 mg/ml, or from about 0.001 to about 300 mg/ml, or from about 0.001 to about 250 mg/ml, or from about 0.001 to about 100 mg/ml, or from about 0.001 to about 50 mg/ml, or from about 0.01 to about 300 mg/ml, or from about 0.01 to about 250 mg/ml, or from about 0.01 to about 100 mg/ml, or from about 0.1 to about 100 mg/ml, or from about 0.1 to about 50 mg/ml).

In one embodiment, a composition of the present invention is in a form of a suspension, dispersion, gel, or ointment. In another embodiment, the suspension or dispersion is based on an aqueous solution. For example, a composition of the present invention can comprise sterile saline solution. In still another embodiment, micrometer-or nanometer-sized particles of a DIGRA, or a pharmaceutically acceptable salt thereof and an anti-infective agent can be coated with a physiologically acceptable surfactant (non-limiting examples are disclosed below), then the coated particles are dispersed in a liquid medium. The coating can keep the particles in a suspension. Such a liquid medium can be selected to produce a sustained-release suspension. For example, the liquid medium can be one that is sparingly soluble in the ocular environment into which the suspension is administered.

An anti-infective agent suitable for a composition of the present invention is selected from the group consisting of antibacterial, antiviral, antifungal, antiprotozoal, and combinations thereof.

Non-limiting examples of biologically-derived antibacterial agents include aminoglycosides (e.g., amikacin, apramycin, arbekacin, bambermycins, butirosin, dibekacin, dihydrostreptomycin, fortimicin(s), gentamicin, isepamicin, kanamycin, micronomicin, neomycin, neomycin undecylenate, netilmicin, paromomycin, ribostamycin, sisomicin, spectinomycin, streptomycin, tobramycin, trospectomycin), amphenicols (e.g., azidamfenicol, chloramphenicol, florfenicol, thiamphenicol), ansamycins (e.g., rifamide, rifampin, rifamycin sv, rifapentine, rifaximin), β-lactams (e.g., carbacephems (e.g., loracarbef), carbapenems (e.g., biapenem, imipenem, meropenem, panipenem), cephalosporins (e.g., cefaclor, cefadroxil, cefamandole, cefatrizine, cefazedone, cefazolin, cefcapene pivoxil, cefclidin, cefdinir, cefditoren, cefepime, cefetamet, cefixime, cefinenoxime, cefodizime, cefonicid, cefoperazone, ceforanide, cefotaxime, cefotiam, cefozopran, cefpimizole, cefpiramide, cefpirome, cefpodoxime proxetil, cefprozil, cefroxadine, cefsulodin, ceftazidime, cefteram, ceftezole, ceftibuten, ceftizoxime, ceftriaxone, cefuroxime, cefuzonam, cephacetrile sodium, cephalexin, cephaloglycin, cephaloridine, cephalosporin, cephalothin, cephapirin sodium, cephradine, pivcefalexin), cephamycins (e.g., cefbuperazone, cefinetazole, cefininox, cefotetan, cefoxitin), monobactams (e.g., aztreonam, carumonam, tigemonam), oxacephems, flomoxef, moxalactam), penicillins (e.g., amdinocillin, amdinocillin pivoxil, amoxicillin, ampicillin, apalcillin, aspoxicillin, azidocillin, azlocillin, bacampicillin, benzylpenicillinic acid, benzylpenicillin sodium, carbenicillin, carindacillin, clometocillin, cloxacillin, cyclacillin, dicloxacillin, epicillin, fenbenicillin, floxacillin, hetacillin, lenampicillin, metampicillin, methicillin sodium, mezlocillin, nafcillin sodium, oxacillin, penamecillin, penethamate hydriodide, penicillin G benethamine, penicillin G benzathine, penicillin G benzhydrylamine, penicillin G calcium, penicillin G hydrabamine, penicillin G potassium, penicillin G procaine, penicillin N, penicillin O, penicillin V, penicillin V benzathine, penicillin V hydrabamine, penimepicycline, phenethicillin potassium, piperacillin, pivampicillin, propicillin, quinacillin, sulbenicillin, sultamicillin, talampicillin, temocillin, ticarcillin), ritipenem, lincosamides (e.g., clindamycin, lincomycin), macrolides (e.g., azithromycin, carbomycin, clarithromycin, dirithromycin, erythromycin, erythromycin acistrate, erythromycin estolate, erythromycin glucoheptonate, erythromycin lactobionate, erythromycin propionate, erythromycin stearate, josamycin, leucomycins, midecamycins, miokamycin, oleandomycin, primycin, rokitamycin, rosaramicin, roxithromycin, spiramycin, troleandomycin), polypeptides (e.g., amphomycin, bacitracin, capreomycin, colistin, enduracidin, enviomycin, fusafungine, gramicidin s, gramicidin(s), mikamycin, polymyxin, pristinamycin, ristocetin, teicoplanin, thiostrepton, tuberactinomycin, tyrocidine, tyrothricin, vancomycin, viomycin, virginiamycin, zinc bacitracin), tetracyclines (e.g., apicycline, chlortetracycline, clomocycline, demeclocycline, doxycycline, guamecycline, lymecycline, meclocycline, methacycline, minocycline, oxytetracycline, penimepicycline, pipacycline, rolitetracycline, sancycline, tetracycline), cycloserine, mupirocin, and tuberin.

Non-limiting examples of synthetic antibacterial agents include 2,4-diaminopyrimidines (e.g., brodimoprim, tetroxoprim, trimethoprim), nitrofurans (e.g., furaltadone, furazolium chloride, nifuradene, nifuratel, nifurfoline, nifurpirinol, nifurprazine, nifurtoinol, nitrofuirantoin), quinolones and analogs (e.g., cinoxacin, ciprofloxacin, clinafloxacin, difloxacin, enoxacin, fleroxacin, flumequine, gatifloxacin, grepafloxacin, levofloxacin, lomefloxacin, miloxacin, moxifloxacin, nadifloxacin, nalidixic acid, norfloxacin, ofloxacin, oxolinic acid, pazufloxacin, pefloxacin, pipemidic acid, piromidic acid, rosoxacin, rufloxacin, sparfloxacin, temafloxacin, tosufloxacin, trovafloxacin, or a fluoroquinolone having the chemical name of 7-[(3R)-3-aminohexahydro-1H-azepin-1-yl]-8-chloro-1-cyclopropyl-6-fluoro-1,4-dihydro-4-oxo-3-quinolinecarboxylic acid monohydrochloride), sulfonamides (e.g., acetyl sulfamethoxypyrazine, benzylsulfamide, chloramines B, chloramines T, dichloramine T, $n^2$-formylsulfisomidine, $n^4$-β-D-glucosylsulfanilamide, mafenide, 4'-(methylsulfamoyl) sulfanilanilide, noprylsulfamide, phthalylsulfacetamide, phthalylsulfathiazole, salazosulfadimidine, succinylsulfathiazole, sulfabenzamide, sulfacetamide, sulfachlorpyridazine, sulfachrysoidine, sulfacytine, sulfadiazine, sulfadicramide, sulfadimethoxine, sulfadoxine, sulfaethidole, sulfaguanidine, sulfaguanol, sulfalene, sulfaloxic acid, sulfamerazine, sulfameter, sulfamethazine, sulfamethizole, sulfamethomidine, sulfamethoxazole, sulfamethoxypyridazine, sulfametrole, sulfamidochrysoidine, sulfamoxole, sulfanilamide, 4-sulfanilamidosalicylic acid, $n^4$-sulfanilylsulfanilamide, sulfanilylurea, N-sulfanilyl-3,4-xylamide, sulfanitran, sulfaperine, sulfaphenazole, sulfaproxyline, sulfapyrazine, sulfapyridine, sulfasomizole, sulfasymazine, sulfathiazole, sulfathiourea, sulfatolamide, sulfisomidine, sulfisoxazole) sulfones (e.g., acedapsone, acediasulfone, acetosulfone sodium, dapsone, diathymosulfone, glucosulfone sodium, solasulfone, succisulfone, sulfanilic acid, p-sulfanilylbenzylamine, sulfoxone sodium, thiazolsulfone), clofoctol, hexedine, methenamine, methenamine anhydromethylene citrate, methenamine hippurate, methenamine mandelate, methenamine sulfosalicylate, nitroxoline, taurolidine, and xibomol. In one embodiment, a composition of the present invention comprises an anti-infective agent selected from the group consisting of cinoxacin, ciprofloxacin, clinafloxacin, difloxacin, enoxacin, fleroxacin, flumequine, gatifloxacin, grepafloxacin, levofloxacin, lomefloxacin, miloxacin, moxifloxacin, nadifloxacin, nalidixic acid, norfloxacin, ofloxacin, oxolinic acid, pazufloxacin, pefloxacin, pipemidic acid, piromidic acid, rosoxacin, rufloxacin, sparfloxacin, temafloxacin, tosufloxacin, trovafloxacin, and a fluoroquinolone having the chemical name of 7-[(3R)-3-aminohexahydro-1H-azepin-1-yl]-8-chloro-1-cyclopropyl-6-fluoro-1,4-dihydro-4-oxo-3-quinolinecarboxylic acid monohydrochloride (as a species of the family of compounds disclosed in U.S. Patents 5,385,900 and 5,447,926.

Non-limiting examples of antiviral agents include Rifampin, Ribavirin, Pleconaryl, Cidofovir, Acyclovir, Pencyclovir, Gancyclovir, Valacyclovir, Famciclovir, Foscarnet, Vidarabine, Amantadine, Zanamivir, Oseltamivir, Resquimod, antiproteases, PEGylated interferon (Pegasys™), anti HIV proteases (e.g. lopinivir, saquinivir, amprenavir, HIV fusion inhibitors, nucleotide HIV RT inhibitors (e.g., AZT, Lamivudine, Abacavir), non-nucleotide HIV RT inhibitors, Doconosol, interferons, butylated hydroxytoluene (BHT), and Hypericin.

Non-limiting examples of biologically-derived antifungal agents include polyenes (e.g., amphotericin B, candicidin, dermostatin, filipin, fungichromin, hachimycin, hamycin, lucensomycin, mepartricin, natamycin, nystatin, pecilocin, perimycin), azaserine, griseofulvin, oligomycins, neomycin undecylenate, pyrrolnitrin, siccanin, tubercidin, and viridin.

Non-limiting examples of synthetic antifungal agents include allylamines (e.g., butenafine, naftifine, terbinafine), imidazoles (e.g., bifonazole, butoconazole, chlordantoin, chlormidazole, cloconazole, clotrimazole, econazole, enilconazole, fenticonazole, flutrimazole, isoconazole, ketoconazole, lanoconazole, miconazole, omoconazole, oxiconazole nitrate, sertaconazole, sulconazole, tioconazole), thiocarbamates (e.g., tolciclate, tolindate, tolnaftate), triazoles (e.g., fluconazole, itraconazole, saperconazole, terconazole), acrisorcin, amorolfine, biphenamine, bromosalicylchloranilide, buclosamide, calcium propionate, chlorphenesin, ciclopirox, cloxyquin, coparaffinate, diamthazole dihydrochloride, exalamide, flucytosine, halethazole, hexetidine, loflucarban, nifuratel, potassium iodide, propionic acid, pyrithione, salicylanilide, sodium propionate, sulbentine, tenonitrozole, triacetin, ujothion, undecylenic acid, and zinc propionate.

Non-limiting examples of antiprotozoal agents include polymycin B sulfate, bacitracin zinc, neomycine sulfate (e.g., Neosporin), imidazoles (e.g., clotrimazole, miconazole, ketoconazole), aromatic diamidines (e.g., propamidine isethionate, Brolene), polyhexamethylene biguanide ("PHMB"), chlorhexidine, pyrimethamine (Daraprim®), sulfadiazine, folinic acid (leucovorin), clindamycin, and trimethoprim-sulfamethoxazole.

In one aspect, the anti-infective agent is selected from the group consisting of bacitracin zinc, chloramphenicol, ciprofloxacin hydrochloride, erythromycin, gatifloxacin, gentamycin sulfate, levofloxacin, moxifloxacin, ofloxacin, sulfacetamide sodium, polymyxin B, tobramycin sulfate, trifluridine, vidarabine, acyclovir, valacyclovir, famcyclovir, foscarnet, ganciclovir, formivirsen, cidofovir, amphotericin B, natamycin, fluconazole, itraconazole, ketoconazole, miconazole, polymyxin B sulfate, neomycin sulfate, clotrimazole, propamidine isethionate, polyhexamethylene biguanide, chlorhexidine, pyrimethamine, sulfadiazine,folinic acid (leucovorin), clindamycin, trimethoprim-sulfamethoxazole, and combinations thereof.

The concentration of an anti-infective agent in such an ophthalmic composition can be in the range from about 0.0001 to about 1000 mg/ml (or, alternatively, from about 0.001 to about 500 mg/ml, or from about 0.001 to about 300 mg/ml, or from about 0.001 to about 250 mg/ml, or from about 0.001 to about 100 mg/ml, or from about 0.001 to about 50 mg/ml, or from about 0.01 to about 300 mg/ml, or from about 0.01 to about 250 mg/ml, or from about 0.01 to about 100 mg/ml, or from about 0.1 to about 100 mg/ml, or from about 0.1 to about 50 mg/ml).

In another aspect, a composition of the present invention can further comprise a non-ionic surfactant, such as polysorbates (such as polysorbate 80 (polyoxyethylene sorbitan monooleate), polysorbate 60 (polyoxyethylene sorbitan monostearate), polysorbate 20 (polyoxyethylene sorbitan monolaurate), commonly known by their trade names of Tween® 80, Tween® 60, Tween® 20), poloxamers (synthetic block polymers of ethylene oxide and propylene oxide, such as those commonly known by their trade names of Pluronic®; e.g., Pluronic® F127 or Pluronic® F108) ), or poloxamines (synthetic block polymers of ethylene oxide and propylene oxide attached to ethylene diamine, such as those commonly known by their trade names of Tetronic®; e.g., Tetronic® 1508 or Tetronic® 908, etc., other nonionic surfactants such as Brim®, Myrj®, and long chain fatty alcohols (i.e., oleyl alcohol, stearyl alcohol, myristyl alcohol, docosohexanoyl alcohol, etc.) with carbon chains having about 12 or more carbon atoms (e.g., such as from about 12 to about 24 carbon atoms). Such compounds are delineated in Martindale, 34th ed., pp. 1411-1416 (Martindale, "The Complete Drug Reference," S. C. Sweetman (Ed.), Pharmaceutical Press, London, 2005) and in Remington, "The Science and Practice of Pharmacy," 21st Ed., p. 291 and the contents of chapter 22, Lippincott Williams & Wilkins, New York, 2006) . The concentration of a non-ionic surfactant, when present, in a composition of the present invention can be in the range from about 0.001 to about 5 weight percent (or alternatively, from about 0.01 to about 4, or from about 0.01 to about 2, or from about 0.01 to about 1, or from about 0.01 to about 0.5 weight percent).

In addition, a composition of the present invention can include additives such as buffers, diluents, carriers, adjuvants, or other excipients. Any pharmacologically acceptable buffer suitable for application to the eye may be used. Other agents may be employed in the composition for a variety of purposes. For example, buffering agents, preservatives, co-solvents, oils, humectants, emollients, stabilizers, or antioxidants may be employed. Water-soluble preservatives which may be employed include sodium bisulfite, sodium bisulfate, sodium thiosulfate, benzalkonium chloride, chlorobutanol, thimerosal, ethyl alcohol, methylparaben, polyvinyl alcohol, benzyl alcohol, and phenylethyl alcohol. These agents may be present in individual amounts of from about 0.001 to about 5% by weight (preferably, about 0.01 % to about 2% by weight). Suitable water-soluble buffering agents that may be employed are sodium carbonate, sodium borate, sodium phosphate, sodium acetate, sodium bicarbonate, etc., as approved by the United States Food and Drug Administration ("US FDA") for the desired route of administration. These agents may be present in amounts sufficient to maintain a pH of the system of between about 2 and about 11. As such, the buffering agent may be as much as about 5% on a weight to weight basis of the total composition. Electrolytes such as, but not limited to, sodium chloride and potassium chloride may also be included in the formulation.

In one aspect, the pH of the composition is in the range from about 4 to about 11. Alternatively, the pH of the composition is in the range from about 5 to about 9, from about 6 to about 9, or from about 6.5 to about 8. In another aspect, the composition comprises a buffer having a pH in one of said pH ranges.

In another aspect, the composition has a pH of about 7. Alternatively, the composition has a pH in a range from about 7 to about 7.5.

In still another aspect, the composition has a pH of about 7.4.

In yet another aspect, a composition also can comprise a viscosity-modifying compound designed to facilitate the administration of the composition into the subject or to promote the bioavailability in the subject. In still another aspect, the viscosity-modifying compound may be chosen so that the composition is not readily dispersed after being administered into the vistreous. Such compounds may enhance the viscosity of the composition, and include, but are not limited to: monomeric polyols, such as, glycerol, propylene glycol, ethylene glycol; polymeric polyols, such as, polyethylene glycol; various polymers of the cellulose family, such as hydroxypropylmethyl cellulose ("HPMC"), carboxymethyl cellulose ("CMC") sodium, hydroxypropyl cellulose ("HPC"); polysaccharides, such as hyaluronic acid and its salts, chondroitin sulfate and its salts, dextrans, such as, dextran 70; water soluble proteins, such as gelatin; vinyl polymers, such as, polyvinyl alcohol, polyvinylpyrrolidone, povidone; carbomers, such as carbomer 934P, carbomer 941, carbomer 940, or carbomer 974P; and acrylic acid polymers. In general, a desired viscosity can be in the range from about 1 to about 400 centipoises ("cps") or mPa.s.

In yet another aspect, the present invention provides a composition for treating or controlling an ophthalmic (e.g., anterior-segment) inflammatory disease, condition, or disorder. In one embodiment, the composition comprises: (a) at least a DIGRA, or a pharmaceutically acceptable salt thereof; and (b) an anti-inflammatory agent other than said DIGRA, prodrug thereof, and pharmaceutically acceptable salt thereof.

In still another aspect, such an anti-inflammatory agent comprises a compound that inhibits or blocks a cyclooxygenase inflammatory pathway, a lipoxygenase inflammatory pathway, or both.

In still another aspect, such an anti-inflammatory agent comprises a compound that inhibits or blocks production of a prostaglandin, thromboxane, or leukotriene.

In yet another aspect, the present invention provides a composition for treating or controlling an ophthalmic (e.g., anterior-segment) inflammatory disease, condition, or disorder. In one embodiment, the composition comprises: (a) at least a DIGRA, or a pharmaceutically acceptable salt thereof; (b) an anti-infective agent; and (c) an anti-inflammatory agent other than said DIGRA, and pharmaceutically acceptable salt thereof. The DIGRA, anti-infective agent, and anti-inflammatory agent other than said DIGRA, and pharmaceutically acceptable salt thereof are present in amounts effective to treat or control the disease, condition, or disorder. In one embodiment, such an anti-inflammatory agent is selected from the group consisting of non-steroidal anti-inflammatory drugs ("NSAIDs"); peroxisome proliferator-activated receptor ("PPAR") ligands, such as PPAR$\alpha$, PPAR$\delta$, or PPAR$\gamma$ ligands; combinations thereof; and mixtures thereof.

Non-limiting examples of the NSAIDs are: aminoarylcarboxylic acid derivatives (e.g., enfenamic acid, etofenamate, flufenamic acid, isonixin, meclofenamic acid, mefenamic acid, niflumic acid, talniflumate, terofenamate, tolfenamic acid), arylacetic acid derivatives (e.g., aceclofenac, acemetacin, alclofenac, amfenac, amtolmetin guacil, bromfenac, bufexamac, cinmetacin, clopirac, diclofenac sodium, etodolac, felbinac, fenclozic acid, fentiazac, glucametacin, ibufenac, indomethacin, isofezolac, isoxepac, lonazolac, metiazinic acid, mofezolac, oxametacine, pirazolac, proglumetacin, sulindac, tiaramide, tolmetin, tropesin, zomepirac), arylbutyric acid derivatives (e.g., bumadizon, butibufen, fenbufen, xenbucin), arylcarboxylic acids (e.g., clidanac, ketorolac, tinoridine), arylpropionic acid derivatives (e.g., alminoprofen, benoxaprofen, bermoprofen, bucloxic acid, carprofen, fenoprofen, flunoxaprofen, flurbiprofen, ibuprofen, ibuproxam, indoprofen, ketoprofen, loxoprofen, naproxen, oxaprozin, piketoprolen, pirprofen, pranoprofen, protizinic acid, suprofen, tiaprofenic acid, ximoprofen, zaltoprofen), pyrazoles (e.g., difenamizole, epirizole), pyrazolones (e.g., apazone, benzpiperylon, feprazone, mofebutazone, morazone, oxyphenbutazone, phenylbutazone, pipebuzone, propyphenazone, ramifenazone, suxibuzone, thiazolinobutazone), salicylic acid derivatives (e.g., acetaminosalol, aspirin, benorylate, bromosaligenin, calcium acetylsalicylate, diflunisal, etersalate, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salacetamide, salicylamide o-acetic acid, salicylsulfuric acid, salsalate, sulfasalazine), thiazinecarboxamides (e.g., ampiroxicam, droxicam, isoxicam, lornoxicam, piroxicam, tenoxicam), $\varepsilon$-acetamidocaproic acid, S-(5'-adenosyl)-L-methionine, 3-amino-4-hydroxybutyric acid, amixetrine, bendazac, benzydamine, $\alpha$-bisabolol, bucolome, difenpiramide, ditazol, emorfazone, fepradinol, guaiazulene, nabumetone, nimesulide, oxaceprol, paranyline, perisoxal, proquazone, superoxide dismutase, tenidap, zileuton, their physiologically acceptable salts, combinations thereof, and mixtures thereof.

In certain embodiments, said anti-inflammatory agent other than said DIGRA, and pharmaceutically acceptable salt thereof is selected from the group consisting of flurbiprofen, suprofen, bromfenac, diclofenac, indomethacin, ketorolac, salts thereof, and combinations thereof.

In another aspect of the present invention, an anti-inflammatory agent is a PPAR-binding molecule. In one embodiment, such a PPAR-binding molecule is a PPAR$\alpha$-, PPAR$\delta$-, or PPAR$\gamma$-binding molecule. In another embodiment, such a PPAR-binding molecule is a PPAR$\alpha$, PPAR$\delta$, or PPAR$\gamma$ agonist. Such a PPAR ligand binds to and activates PPAR to modulate the expression of genes containing the appropriate peroxisome proliferator response element in its promoter region.

PPAR$\gamma$ agonists can inhibit the production of TNF-$\alpha$ and other inflammatory cytokines by human macrophages (C-Y. Jiang et al., Nature, Vol. 391, 82-86 (1998)) and T lymphocytes (A.E. Giorgini et al., Horm. Metab. Res. Vol. 31, 1-4 (1999)). More recently, the natural PPAR$\gamma$ agonist 15-deoxy-$\Delta$-12,14-prostaglandin J2 (or "15-deoxy-$\Delta$-12,14-PG J2"), has been shown to inhibit neovascularization and angiogenesis (X. Xin et al., J. Biol. Chem. Vol. 274:9116-9121 (1999)) in the rat cornea. Spiegelman et al., in U.S. Patent 6,242,196, disclose methods for inhibiting proliferation of PPAR$\gamma$-responsive hyperproliferative cells by using PPAR$\gamma$ agonists; numerous synthetic PPAR$\gamma$ agonists are disclosed by Spiegelman et al., as well as methods for diagnosing PPAR$\gamma$-responsive hyperproliferative cells. All documents referred to herein are incorporated by reference. PPARs are differentially expressed in diseased versus normal cells. PPAR$\gamma$ is expressed to different degrees in the various tissues of the eye, such as some layers of the retina and the

cornea, the choriocapillaris, uveal tract, conjunctival epidermis, and intraocular muscles (see, e.g., U.S. Patent 6,316,465).

In one aspect, a PPARγ agonist used in a composition or a method of the present invention is a thiazolidinedione, a derivative thereof, or an analog thereof. Non-limiting examples of thiazolidinedione-based PPARγ agonists include pioglitazone, troglitazone, ciglitazone, englitazone, rosiglitazone, and chemical derivatives thereof. Other PPARγ agonists include Clofibrate (ethyl 2-(4-chlorophenoxy)-2-methylpropionate), clofibric acid (2-(4-chlorophenoxy)-2-methylpropanoic acid), GW 1929 (N-(2-benzoylphenyl)-O-{2-(methyl-2-pyridinylamino)ethyl} -L-tyrosine), GW 7647 (2- {{4-{2-{{(cyclohexylamino)carbonyl}(4-cyclohexylbutyl)amino}ethyl}phenyl}thio}-2-methylpropanoic acid), and WY 14643 ({{4-chloro-6-{(2,3-dimethylphenyl)amino}-2-pyrimidinyl}thio} acetic acid). GW 1929, GW 7647, and WY 14643 are commercially available, for example, from Koma Biotechnology, Inc. (Seoul, Korea). In one embodiment, the PPARγ agonist is 15-deoxy-Δ-12, 14-PG J2.

Non-limiting examples of PPAR-α agonists include the fibrates, such as fenofibrate and gemfibrozil. A non-limiting example of PPAR-δ agonist is GW501516 (available from Axxora LLC, San Diego, California or EMD Biosciences, Inc., San Diego, California).

The concentration of an-y foregoing additional active ingredient in such an ophthalmic composition can be in the range from about 0.0001 to about 1000 mg/ml (or, alternatively, from about 0.001 to about 500 mg/ml, or from about 0.001 to about 300 mg/ml, or from about 0.001 to about 250 mg/ml, or from about 0.001 to about 100 mg/ml, or from about 0.001 to about 50 mg/ml, or from about 0.01 to about 300 mg/ml, or from about 0.01 to about 250 mg/ml, or from about 0.01 to about 100 mg/ml, or from about 0.1 to about 100 mg/ml, or from about 0.1 to about 50 mg/ml).

In still another aspect, a method for preparing a composition of the present invention comprises combining: (a) at least a DIGRA, or a pharmaceutically acceptable salt thereof; and (b) a pharmaceutically acceptable carrier; In one embodiment, such a carrier can be a sterile saline solution or a physiologically acceptable buffer. In another embodiment, such a carrier comprises a hydrophobic medium, such as a pharmaceutically acceptable oil. In still another embodiment, such as carrier comprises an emulsion of a hydrophobic material and water.

Physiologically acceptable buffers include, but are not limited to, a phosphate buffer or a Tris-HCl buffer (comprising tris(hydroxymethyl)aminomethane and HCl). For example, a Tris-HCl buffer having pH of 7.4 comprises 3 g/l of tris(hydroxymethyl)aminomethane and 0.76 g/l of HCl. In yet another aspect, the buffer is 10X phosphate buffer saline ("PBS") or 5X PBS solution.

Other buffers also may be found suitable or desirable in some circumstances, such as buffers based on HEPES (N-{2-hydroxyethyl}peperazine-N'-{2-ethanesulfonic acid}) having $pK_a$ of 7.5 at 25 °C and pH in the range of about 6.8-8.2; BES (N,N-bis{2-hydroxyethyl}2-aminoethanesulfonic acid) having $pK_a$ of 7.1 at 25°C and pH in the range of about 6.4-7.8; MOPS (3-{N-morpholino}propanesulfonic acid) having $pK_a$ of 7.2 at 25°C and pH in the range of about 6.5-7.9; TES (N-tris{hydroxymethyl}-methyl-2-aminoethanesulfonic acid) having $pK_a$ of 7.4 at 25°C and pH in the range of about 6.8-8.2; MOBS (4-{N-morpholino}butanesulfonic acid) having $pK_a$ of 7.6 at 25°C and pH in the range of about 6.9-8.3; DIPSO (3-(N,N-bis {2-hydroxyethyl}amino)-2-hydroxypropane)) having $pK_a$ of 7.52 at 25°C and pH in the range of about 7-8.2; TAPSO (2-hydroxy-3{tris(hydroxymethyl)methylamino}-1-propanesulfonic acid)) having $pK_a$ of 7.61 at 25°C and pH in the range of about 7-8.2; TAPS ({(2-hydroxy-1,1-bis(hydroxymethyl)ethyl)amino}-1-propanesulfonic acid)) having $pK_a$ of 8.4 at 25°C and pH in the range of about 7.7-9.1; TABS (N-tris(hydroxymethyl)methyl-4-aminobutanesulfonic acid) having $pK_a$ of 8.9 at 25°C and pH in the range of about 8.2-9.6; AMPSO (N-(1,1-dimethyl-2-hydroxyethyl)-3-amino-2-hydroxypropanesulfonic acid)) having $pK_a$ of 9.0 at 25°C and pH in the range of about 8.3-9.7; CHES (2-cyclohexylamino)ethanesulfonic acid) having $pK_a$ of 9.5 at 25°C and pH in the range of about 8.6-10.0; CAPSO (3-(cyclohexylamino)-2-hydroxy-1-propanesulfonic acid) having $pK_a$ of 9.6 at 25°C and pH in the range of about 8.9-10.3; or CAPS (3-(cyclohexylamino)-1-propane sulfonic acid) having $pK_a$ of 10.4 at 25°C and pH in the range of about 9.7-11.1.

In certain embodiments, a composition of the present invention is formulated in a buffer having an acidic pH, such as from about 4 to about 6.8, or alternatively, from about 5 to about 6.8. In such embodiments, the buffer capacity of the composition desirably allows the composition to come rapidly to a physiological pH after being administered into the patient.

It should be understood that the proportions of the various components or mixtures in the following examples may be adjusted for the appropriate circumstances.

EXAMPLE 1

Two mixtures I and II are made separately by mixing the ingredients listed in Table 1. Five parts (by weight) of mixture I are mixed with twenty parts (by weight) of mixture II for 15 minutes or more. The pH of the combined mixture is adjusted to 6.2-6.4 using 1 N NaOH or 1 N HCl solution to yield a composition of the present invention.

Table 1

| Ingredient | Amount |
|---|---|
| Mixture I | |
| ciprofloxacin HCl | 0.2g |
| Carbopol 934P NF | 0.25 g |
| Purified water | 99.55 g |
| Mixture II | |
| Propylene glycol | 5 g |
| EDTA | 0.1 mg |
| Compound of Formula IV | 50 g |

EXAMPLE 2:

Two mixtures I and II are made separately by mixing the ingredients listed in Table 2. Five parts (by weight) of mixture I are mixed with twenty parts (by weight) of mixture II for 15 minutes or more. The pH of the combined mixture is adjusted to 6.2-6.4 using 1 N NaOH or 1 N HCl solution to yield a composition of the present invention.

Table 2

| Ingredient | Amount |
|---|---|
| Mixture I | |
| moxifloxacin | 0.2g |
| diclofenac | 0.3g |
| Carbopol 934P NF | 0.25 g |
| Purified water | 99.25 g |
| Mixture II | |
| Propylene glycol | 5 g |
| EDTA | 0.1 mg |
| Compound of Formula IV | 50 g |

EXAMPLE 3:

Two mixtures I and II are made separately by mixing the ingredients listed in Table 3. Five parts (by weight) of mixture I are mixed with twenty parts (by weight) of mixture II for 15 minutes or more. The pH of the combined mixture is adjusted to 6.2-6.4 using 1 N NaOH or 1 N HCl solution to yield a composition of the present invention.

Table 3

| Ingredient | Amount |
|---|---|
| Mixture I | |
| gatifloxacin | 0.2g |
| ciglitazone | 0.2g |
| Carbopol 934P NF | 0.25 g |
| Purified water | 99.35 g |
| Mixture II | |
| Propylene glycol | 3 g |

(continued)

| Ingredient | Amount |
|---|---|
| Triacetin | 7g |
| Compound of Formula II | 50 g |
| EDTA | 0.1 mg |

EXAMPLE 4:

Two mixtures I and II are made separately by mixing the ingredients listed in Table 4. Five parts (by weight) of mixture I are mixed with twenty parts (by weight) of mixture II for 15 minutes or more. The pH of the combined mixture is adjusted to 6.2-7.5 using 1 N NaOH or 1 N HCl solution to yield a composition of the present invention.

Table 4

| Ingredient | Amount |
|---|---|
| Mixture I | |
| tobramycin sulfate | 0.3g |
| gemfibrozil | 0.3g |
| Carbopol 934P NF | 0.25 g |
| Olive oil | 99.15 g |
| Mixture II | |
| Propylene glycol | 7 g |
| Glycerin | 3 g |
| Compound of Formula III | 50 g |
| Cyclosporine A | 5 g |
| HAP (30%) | 0.5 mg |
| Alexidine 2HCl | 1-2 ppm |
| Note: "HAP" denotes hydroxyalkyl phosphonates, such as those known under the trade name Dequest®. | |

EXAMPLE 5:

The ingredients listed in Table 5 are mixed together for at least 15 minutes. The pH of the mixture is adjusted to 6.2-7.5 using 1 N NaOH or 1 N HCl solution to yield a composition of the present invention.

Table 5

| Ingredient | Amount (% by weight) |
|---|---|
| Povidone | 1 |
| HAP (30%) | 0.05 |
| Glycerin | 3 |
| Propylene glycol | 3 |
| Compound of Formula IV | 0.5 |
| Trifluridine | 0.1 |
| Tyloxapol | 0.25 |
| BAK | 10-100 ppm |

(continued)

| Ingredient | Amount (% by weight) |
|---|---|
| Purified water | q.s. to 100 |
| Note: "BAK" denotes benzalkonium chloride. | |

EXAMPLE 6:

The ingredients listed in Table 6 are mixed together for at least 15 minutes. The pH of the mixture is adjusted to 7-7.5 using 1 N NaOH or 1 N HCl solution to yield a composition of the present invention.

Table 6

| Ingredient | Amount (% by weight) |
|---|---|
| Povidone | 1.5 |
| HAP (30%) | 0.05 |
| Glycerin | 3 |
| Propylene glycol | 3 |
| Compound of Formula IV | 0.75 |
| Foscavir | 0.1 |
| Tyloxapol | 0.25 |
| Alexidine 2HCl | 1-2 ppm |
| Purified water | q.s. to 100 |

EXAMPLE 7:

The ingredients listed in Table 7 are mixed together for at least 15 minutes. The pH of the mixture is adjusted to 6.5-7.8 using 1 N NaOH or 1 N HCl solution to yield a composition of the present invention.

Table 7

| Ingredient | Amount (% by weight) |
|---|---|
| CMC (MV) | 0.5 |
| HAP (30%) | 0.05 |
| Glycerin | 3 |
| Propylene glycol | 3 |
| Compound of Formula IV | 0.75 |
| Amphotericin B | 0.1 |
| Ketorolac | 0.3 |
| Tyloxapol (a surfactant) | 0.25 |
| Alexidine 2HCl | 1-2 ppm |
| Purified water | q.s. to 100 |

EXAMPLE 8:

The ingredients listed in Table 8 are mixed together for at least 15 minutes. The pH of the mixture is adjusted to 6.2-7.4 using 1 N NaOH or 1 N HCl solution to yield a composition of the present invention.

Table 8

| Ingredient | Amount (% by weight) |
|---|---|
| CMC (MV) | 0.5 |
| HAP (30%) | 0.05 |
| Glycerin | 3 |
| Propylene glycol | 3 |
| Compound of Formula IV | 0.75 |
| Miconazole | 0.2 |
| 15-deoxy-Δ-12,14-prostaglandin J2 | 0.3 |
| Tyloxapol (a surfactant) | 0.25 |
| Alexidine 2HCl | 1-2 ppm |
| Purified water | q.s. to 100 |

EXAMPLE 9:

The ingredients listed in Table 9 are mixed together for at least 15 minutes. The pH of the mixture is adjusted to 6.2-6.8 using 1 N NaOH or 1 N HCl solution to yield a composition of the present invention.

Table 9

| Ingredient | Amount (% by weight) |
|---|---|
| CMC (MV) | 0.5 |
| HAP (30%) | 0.05 |
| Glycerin | 3 |
| Propylene glycol | 3 |
| Compound of Formula IV | 0.75 |
| Bacitracin zinc | 0.2 |
| Flurbiprofen | 0.2 |
| Levofloxacin | 0.3 |
| Tyloxapol (a surfactant) | 0.25 |
| Alexidine 2HCl | 1-2 ppm |
| Purified water | q.s. to 100 |

EXAMPLE 10:

The ingredients listed in Table 10 are mixed together for at least 15 minutes. The pH of the mixture is adjusted to 6.2-6.8 using 1 N NaOH or 1 N HCl solution to yield a composition of the present invention.

Table 10

| Ingredient | Amount (% by weight) |
|---|---|
| CMC (MV) | 0.5 |
| HAP (30%) | 0.05 |
| Glycerin | 3 |
| Propylene glycol | 3 |

(continued)

| Ingredient | Amount (% by weight) |
|---|---|
| Compound of Formula IV | 0.75 |
| Moxifloxacin | 0.2 |
| 15-deoxy-Δ-12,14-prostaglandin J2 | 0.3 |
| clotrimazole | 0.2 |
| Tyloxapol (a surfactant) | 0.25 |
| Alexidine 2HCl | 1-2 ppm |
| Purified water | q.s. to 100 |

EXAMPLE 11:

The ingredients listed in Table 11 are mixed together for at least 15 minutes. The pH of the mixture is adjusted to 6.2-7 using 1 N NaOH or 1 N HCl solution to yield a composition of the present invention.

Table 11

| Ingredient | Amount |
|---|---|
| Ketorolac | 0.4 g |
| Compound having Formula IV | 0.2 g |
| Carbopol 934P NF | 0.25 g |
| Propylene glycol | 5 g |
| EDTA | 0.5 mg |
| Purified water | 98.65 g |

In another aspect of the present disclosure, a DIGRA, a prodrug thereof, or a pharmaceutically acceptable salt thereof, and a material selected from the group consisting of: (i) anti-infective agents, (ii) anti-inflammatory agents other than said DIGRA, prodrug thereof, and pharmaceutically acceptable salts thereof, and (iii) combinations thereof, are incorporated into a formulation for topical administration or periocular injection to a portion of the anterior segment. An injectable formulation can desirably comprise a carrier that provides a sustained-release of the active ingredients, such as for a period longer than about 1 week (or longer than about 1, 2, 3, 4, 5, or 6 months). In certain embodiments, the sustained-release formulation desirably comprises a carrier that is insoluble or only sparingly soluble in the anterior-segment environment. Such a carrier can be an oil-based liquid, emulsion, gel, or semisolid. Non-limiting examples of oil-based liquids include castor oil, peanut oil, olive oil, coconut oil, sesame oil, cottonseed oil, corn oil, sunflower oil, fish-liver oil, arachis oil, and liquid paraffin.

A compound or composition of the present invention can be injected with a fine-gauge needle, such as 25-35 gauge. Typically, an amount from about 25 μl to about 100 μl of a composition comprising a DIGRA, a prodrug thereof, or a pharmaceutically acceptable salt thereof is administered into a patient. A concentration of such DIGRA, prodrug thereof, or pharmaceutically acceptable salt thereof is selected from the ranges disclosed above.

In still another aspect of the present disclosure, a DIGRA, a prodrug thereof, or a pharmaceutically acceptable salt thereof is incorporated into an ophthalmic device that comprises a biodegradable material, and the device is implanted into an anterior-segment tissue of a subject to provide a long-term (e.g., longer than about 1 week, or longer than about 1, 2, 3, 4, 5, or 6 months) treatment or control of an anterior-segment inflammatory disease, condition, or disorder. Such a device may be implanted by a skilled physician in the subject's ocular or periocular tissue.

In still another aspect of the present dislosure, a method for treating or controlling an anterior-segment inflammatory disease, condition, or disorder comprises:

(a) providing a composition comprising a DIGRA, a prodrug thereof, or a pharmaceutically acceptable salt thereof; and (b) administering to a subject an amount of the composition at a frequency sufficient to treat or control said anterior-segment disease, condition, or disorder in a subject.

A method for treating or controlling a post-operative inflammation of the anterior segment comprises: (a) providing a composition comprising a DIGRA, a prodrug thereof, or a pharmaceutically acceptable salt thereof; and (b) administering to a subject an amount of the composition at a frequency sufficient to treat or control said post-operative inflammation.

In still another aspect of the present disclosure, a method for treating or controlling an anterior-segment inflammatory disease, condition, or disorder comprises:

(a) providing a composition comprising: (i) a DIGRA, a prodrug thereof, or a pharmaceutically acceptable salt thereof; and (ii) an anti-inflammatory agent other than said DIGRA, prodrug thereof, and pharmaceutically acceptable salt thereof; and (b) administering to a subject an amount of the composition at a frequency sufficient to treat or control an anterior-segment disease, condition, or disorder in a subject.

In still another aspect of the present disclosure, a method for treating or controlling a post-operative inflammation of the anterior segment comprises: (a) providing a composition comprising: (i) a DIGRA, a prodrug thereof, or a pharmaceutically acceptable salt thereof; and (ii) an anti-inflammatory agent other than said DIGRA, prodrug thereof, and pharmaceutically acceptable salt thereof; and (b) administering to a subject an amount of the composition at a frequency sufficient to treat or control said post-operative inflammation.

In still another aspect of the present disclosure, a method for treating or controlling an anterior-segment inflammatory disease, condition, or disorder comprises:

(a) providing a composition comprising: (i) a DIGRA, a prodrug thereof, or a pharmaceutically acceptable salt thereof; (ii) an anti-inflammatory agent other than said DIGRA, prodrug thereof, and pharmaceutically acceptable salt thereof; and (iii) an anti-infective agent; and (b) administering to a subject an amount of the composition at a frequency sufficient to treat or control an anterior-segment disease, condition, or disorder in a subject.

In still another aspect of the present disclosure, a method for treating or controlling a post-operative inflammation of the anterior segment comprises: (a) providing a composition comprising: (i) a DIGRA, a prodrug thereof, or a pharmaceutically acceptable salt thereof; (ii) an anti-inflammatory agent other than said DIGRA, prodrug thereof, and pharmaceutically acceptable salt thereof; and (iii) an anti-infective agent; and (b) administering to a subject an amount of the composition at a frequency sufficient to treat or control said post-operative inflammation.

In certain embodiments, the DIGRA is selected from among those disclosed above.

In other embodiments, the anti-inflammatory agent is selected from among those disclosed above. In some embodiments, the anti-inflammatory agent is selected from the group consisting of flurbiprofen, suprofen, bromfenac, diclofenac, indomethacin, ketorolac, salts thereof, and combinations thereof.

In another embodiment, such inflammation is a long-term inflammation. In still another embodiment, such inflammation requires at least two weeks for resolution, if untreated.

In still another embodiment, such inflammatory anterior-segment disease, condition, or disorder results from ophthalmic infection that is caused by a virus, bacteria, fungus, or protozoa.

In another aspect, a composition of the present invention is administered periocularly or in the anterior chamber. In still another aspect, a composition of the present invention is incorporated into an ophthalmic implant system or device, and the implant system or device is surgically implanted periocularly or in a tissue adjacent to the anterior portion of the eye of the patient for the sustained release of the active ingredient or ingredients. A typical implant system or device suitable for use in a method of the present invention comprises a biodegradable matrix with the active ingredient or ingredients impregnated or dispersed therein. Non-limiting examples of ophthalmic implant systems or devices for the sustained-release of an active ingredient are disclosed in U.S. Patents 5,378,475; 5,773,019; 5,902,598; 6,001,386; 6,051,576; and 6,726,918.

In yet another aspect, a composition of the present invention is administered once a week, once a month, once a year, twice a year, four times a year, or at a suitable frequency that is determined to be appropriate for treating or controlling an anterior-segment inflammatory disease, condition, or disorder.

COMPARISON OF GLUCOCORTICOIDS AND DIGRAS

One of the most frequent undesirable actions of a glucocorticoid therapy is steroid diabetes. The reason for this undesirable condition is the stimulation of gluconeogenesis in the liver by the induction of the transcription of hepatic enzymes involved in gluconeogenesis and metabolism of free amino acids that are produced from the degradation of proteins (catabolic action of glucocorticoids). A key enzyme of the catabolic metabolism in the liver is the tyrosine aminotransferase ("TAT"). The activity of this enzyme can be determined photometrically from cell cultures of treated rat hepatoma cells. Thus, the gluconeogenesis by a glucocorticoid can be compared to that of a DIGRA by measuring

the activity of this enzyme. For example, in one procedure, the cells are treated for 24 hours with the test substance (a DIGRA or glucocorticoid), and then the TAT activity is measured. The TAT activities for the selected DIGRA and glucocorticoid are then compared. Other hepatic enzymes can be used in place of TAT, such as phosphoenolpyruvate carboxykinase, glucose-6-phosphatase, or fructose-2,6-biphosphatase. Alternatively, the levels of blood glucose in an animal model may be measured directly and compared for individual subjects that are treated with a glucocorticoid for a selected condition and those that are treated with a DIGRA for the same condition.

Another undesirable result of glucocorticoid therapy is GC-induced cataract. The cataractogenic potential of a compound or composition may be determined by quantifying the effect of the compound or composition on the flux of potassium ions through the membrane of lens cells (such as mammalian lens epithelial cells) in vitro. Such an ion flux may be determined by, for example, electrophysiological techniques or ion-flux imaging techniques (such as with the use of fluorescent dyes). An exemplary in-vitro method for determining the cataractogenic potential of a compound or composition is disclosed in U.S. Patent Application Publication 2004/0219512,

Still another undesirable result of glucocorticoid therapy is hypertension. Blood pressure of similarly matched subjects treated with glucocorticoid and DIGRA for an inflammatory condition may be measured directly and compared.

Yet another undesirable result of glucocorticoid therapy is increased IOP in the subject. IOP of similarly matched subjects treated with glucocorticoid and DIGRA for a condition may be measured directly and compared.

TESTING: Comparison of the DIGRA Having Formula IV With Two Corticosteroids and One NSAID in Treating Anterior-Segment Inflammation

1. INTRODUCTION

Inflammatory processes are multidimensional in origin, and are characterized by complex cellular and molecular events involving numerous components all of which have not been identified. Prostaglandins are among these mediators and play an important role in certain forms of ocular inflammation. Paracentesis of the anterior chamber in the rabbit eye induces inflammatory reaction due to the disruption of the blood-aqueous barrier ("BAB"), which is mediated, at least in part, by prostaglandin $E_2$ [References 1 -3 below]. Intraocular or topical administration of $PGE_2$ disrupts the BAB. [Reference 4, below] The treatment schedule adopted in this study was similar to the clinical NSAIDs (Ocufen) treatment schedule used by surgeons for patients before cataract surgery. We investigated a dissociated glucocorticoid receptor agonist ("BOL-303242-X", compound having Formula IV above) at different doses on rabbit paracentesis model evaluating aqueous biomarkers levels, and iris-ciliary body MPO activity in comparison with vehicle, dexamethasone, loteprednol and flurbiprofen.

2. METHODS

2.1 Drugs and Materials

2.1.1. Test articles

BOL-303242-X (0.1%, 0.5% and 1% topical formulations), lot 2676-MLC-107, Bausch & Lomb Incorporated ("B&L") Rochester, USA.

Vehicle (10% PEG 3350; 1% Tween 80; phosphate buffer pH 7.00), lot 2676-MLC-107, B&L Rochester, USA.

Visumetazone® (0.1% Dexamethasone topical formulation), lot T253, Visufarma, Rome, Italy.

Lotemax® (0.5% Loteprednol topical formulation), lot 078061, B&L IOM, Macherio, Italy.

Ocufen® (0.03% Flurbiprofen topical formulation), lot E45324, Allergan, Westport, Ireland.

2.2 Animals

Species: Rabbit

Breed: New Zealand

Source: Morini (Reggio Emila, Italy)

Sex: Male

Age at Experimental Start: 10 weeks.

Weight Range at Experimental Start: 2.0-2.4 Kg

Total Number of Animals: 28

Identification: Ear tagged with an alphanumeric code (i.e. A1 means test article A and animal 1).

Justification: The rabbit is a standard non-rodent species used in pharmacodynamic studies. The number of animals used in this study is, in judgment of the investigators involved, the minimum number necessary to properly perform this type of study and it is consistent with world wide regulatory guidelines.

Acclimation/Quarantine: Following arrival, a member of the veterinary staff assessed animals as to their general health. Seven days elapsed between animal receipt and the start of experiment in order to acclimate animals to the laboratory environment and to observe them for the development of infection disease.

Animal Husbandry: All the animals were housed in a cleaned and disinfected room, with a constant temperature (22$\pm$1 °C), humidity (relative, 30%) and under a constant light-dark cycle (light on between 8.00 and 20.00). Commercial food and tap water were available *ad libitum.* Their body weights were measured just before the experiment (Table T-1). All the animals had a body weight inside the central part of the body weight distribution curve (10%). Four rabbits were replaced with animals of similar age and weight from the same vendor because three of them showed signs of ocular inflammation and one was dead upon arrival.

Animals Welfare Provisions: All experiments were carried out according to the ARVO (Association for Research in Vision and Ophthalmology) guidelines on the use of animals in research. No alternative test system exists which have been adequately validated to permit replacement of the use of live animals in this study. Every effort has been made to obtain the maximum amount of information while reducing to a minimum the number of animals required for this study. To the best of our knowledge, this study is not unnecessary or duplicative. The study protocol was reviewed and approved by the Institutional Animal Care and Use Committee (IACUC) of the University of Catania and complies with the acceptable standards of animal welfare care.

2.3 Experimental Preparations

2.3.1 Study design and randomization

Twenty-eight rabbits were randomly allocated into 7 groups (4 animals/each) as shown in the table below.

Table 8

<table>
<tr><th>Group</th><th>No of rabbits</th><th colspan="2">Treatment</th><th>Observations and measurements</th><th>Termination and assays</th></tr>
<tr><td>I</td><td>4</td><td>CTR</td><td rowspan="7">50 μl drops at 180, 120, 90, and 30 min prior to first paracentesis, and at 15, 30, 90 min after the first paracentesis.</td><td rowspan="7">Clinical observations and pupillary diameter at 180 and 5 min before the first paracentesis, and at 5 min before the second paracentesis.<br>Paracentesis at 0 and 2 hours.</td><td rowspan="7">Termination immediately after the second paracentesis.<br>Aqueous humor collected for $PGE_2$, protein, leukocytes and $LTB_4$ measurements.<br>Iris-ciliary body collected for MPO activity measurement.</td></tr>
<tr><td>II</td><td>4</td><td>1% BOL</td></tr>
<tr><td>III</td><td>4</td><td>0.5% BOL</td></tr>
<tr><td>IV</td><td>4</td><td>0.1% BOL</td></tr>
<tr><td>V</td><td>4</td><td>0.5% LE</td></tr>
<tr><td>VI</td><td>4</td><td>0.1% Dex</td></tr>
<tr><td>VII</td><td>4</td><td>0.03% F</td></tr>
<tr><td colspan="6">CTR=vehicle; BOL=BOL-303242-X; LE=loteprednol etabonate; Dex=dexamethasone; F=flurbiprofen</td></tr>
</table>

To each test article was randomly assigned a letter from A to G
A = vehicle (10% PEG3350/1% Tween 80/PB pH 7.00)
B = Ocufen (Flurbiprofen 0.03%)
C = Visumetazone (Dexamethasone 0.1%)
D = Lotemax (Loteprednol etabonate 0.5%)
E = BOL-303242-X 0.1% (1 mg/g)
F = BOL-303242-X 0.5% (5 mg/g)
G = BOL-303242-X 1% (10 mg/g)

2.3.2 Reagent preparation for MPO assay

2.3.2.1 Phosphate buffer (50mM; pH=6)

3.9g of $NaH_2PO_4$ $2H_2O$ were dissolved in a volumetric flask to 500ml with water. The pH was adjusted to pH=6 with 3N NaOH.

2.3.2.2 Hexa-decyl-trimethyl-ammonium bromide (0.5%)

0.5g of hexa-decyl-trimethyl-ammonium bromide was dissolved in 100ml phosphate buffer.

2.3.2.3 o-dianisidine 2HCl (0.0167%) / $H_2O_2$ (0.0005%) solution

The solution was prepared freshly. Ten microliters of $H_2O_2$ (30 wt.%) were diluted to 1ml with water (solution A). 7.5mg o-dianisidine 2HCl was dissolved in 45ml of phosphate buffer and 75 μl of solution A were added.

2.4 Experimental Protocols

2.4.1 Animals treatment and sample collection

Each rabbit was placed in a restraint device and tagged with the alphanumeric code. The formulations were instilled (50 μl) into the conjunctival sac of both eyes 180, 120, 90 and 30 min before the first paracentesis; then 15, 30, 90 min after the first paracentesis. To perform the first paracentesis the animals were anaesthetized by intravenous injection of 5mg/kg Zoletil® (Virbac; 2.5mg/kg tiletamine HCl and 2.5mg/kg zolazepam HCl) and one drop of local anesthetic (Novesina®, Novartis) was administered to the eye. Anterior chamber paracentesis was performed with a 26 G needle attached to a tuberculin syringe; the needle was introduced into the anterior chamber through the cornea, taking care not to damage the tissues. Two hours after the first paracentesis, the animals were sacrificed with 0.4 ml Tanax® (Intervet International B.V.) and the second paracentesis was performed. About 100 μl of aqueous humor were removed

at the second paracentesis. Aqueous humor was immediately split in four aliquots and stored at -80 °C until analysis. Then both eyes were enucleated and the iris-ciliary body was carefully excised, placed in polypropylene tubes, and stored at -80 °C until analysis.

2.4.2 Pupillary diameter measurement

The pupillary diameter of both eyes was measured with a Castroviejo caliper 180 min and 5 min before the first paracentesis and 5 min before the second paracentesis.

2.4.3 Clinical evaluation

The clinical evaluation of both eyes was performed by a slit lamp (4179-T; Sbisà, Italy) at 180 min and 5 min before the first paracentesis and 5 min before the second paracentesis. The clinical score was assigned according to the following scheme:

0 = normal
1 = discrete dilatation of iris and conjunctival vessels
2 = moderate dilatation of iris and conjunctival vessels
3 = intense iridal hyperemia with flare in the anterior chamber
4 = intense iridal hyperemia with flare in the anterior chamber and presence of fibrinous exudates.

2.4.4 Prostaglandin $E_2$ ($PGE_2$) measurement

For the quantitative determination of $PGE_2$ in the aqueous humor we used the $PGE_2$ Immunoassay kit (R&D Systems; Cat.No. KGE004; Lot.No. 240010). Eleven microliters or 16μl of aqueous humor were diluted to 110μl or 160μl with the calibrator diluent solution provided with the kit. One hundred microliters of samples and of standards were load into a 96-well plate and recorded in a plate layout. Samples were treated following the assay procedure described in the kit. A microplate reader (GDV, Italy; model DV 990 B/V6) set at 450 nm (wavelength correction at 540 nm) was used for making the calibration and analyzing the samples.

2.4.5 Protein measurement

For protein concentration determination in the aqueous humor we used the Protein Quantification Kit (Fluka; Cat.No. 77371; Lot.No. 1303129). Five microliters of aqueous humor were diluted to 100μl with water. Twenty microliters of samples and of standards were load into a 96-well plate and recorded in a plate layout. Samples were treated following the assay procedure described in the kit. A microplate reader (GDV, Italy; model DV 990 B/V6) set at 670 nm was used for making the calibration and analyzing the samples.

2.4.6 Leukocytes (PMN) measurement

For the determination of the number of leukocytes we used a haemocytometer (Improved Neubauer Chamber; Bright-line, Hausser Scientific) and a Polyvar 2 microscope (Reichert-Jung).

2.4.7 Leukotriene $B_4$ ($LTB_4$) measurement

For the quantitative determination of $LTB_4$ concentration in the aqueous humor we used the $LTB_4$ Immunoassay kit (R&D Systems; Cat.No. KGE006; Lot.No. 243623). 11μl of aqueous humor were diluted to 110μl with the calibrator diluent solution provided with the kit. 100 μl of samples and of standards were load into a 96-well plate and recorded in a plate layout. Samples were treated following the assay procedure described in the kit. A microplate reader (GDV, Italy; model DV 990 B/V6) set at 450 nm (wavelength correction at 540 nm) was used for making the calibration and analyzing the samples.

2.4.8 Myeloperoxidase (MPO) measurement

The activity of MPO was measured as previously described by Williams et al.[5] The iris-ciliary bodies were carefully dried, weighed and immersed in 1ml of hexa-decyl-trimethyl-ammonium bromide solution. Then, the samples were sonicated for 10 sec on ice by a ultrasound homogenizer (HD 2070, Bandelin electronic), freeze-thawed three times, sonicated for 10 sec and centrifuged at 14,000 g for 10 min to remove cellular debris. An aliquot of the supernatant

(40-200μl) was diluted to 3ml with the *o*-dianisidine 2HCl / $H_2O_2$ solution. The change in absorbance at 460nm was continuously monitored for 5 min by a spectrophotometer (UV/Vis Spectrometer Lambda EZ 201; Perkin Elmer). The slope of the line (Δ/min) was determined for each sample and used to calculate the number of units of MPO in the tissue as follows:

$$MPOunit/g = \frac{(\Delta/\min)\cdot 10^6}{\varepsilon \cdot \mu l \cdot mg}$$

were $\varepsilon$ = 11.3 $mM^{-1}$.

Values were expressed as units of MPO/g of tissue.

2.5 Data Analysis

Pupillary diameter, $PGE_2$, protein, PMN, and MPO were expressed as mean ± SEM. Statistical analysis was performed using one way ANOVA followed by a Newman-Keuls post hoc test. Clinical score was expressed as % of eyes and the statistical analysis was performed using Kruskal-Wallis followed by a Dunn post hoc test. P<0.05 was considered statistically significant in both cases. Prism 4 software (GraphPad Software, Inc.) was used for the analysis and graphs.

3. RESULTS

3.1 Pupillary diameter measurement

The raw data are displayed in Tables T-2 and T-3. No statistical significance was found between the CRT and all the treatments.

3.2 Clinical evaluation

The raw data are displayed in Tables T-4 and T-5. Only the 0.5% LE group showed a significant difference versus CTR (p<0.05).

3.3 Prostaglandin $E_2$ ($PGE_2$) measurement

The raw data are displayed in Tables T-6 and T-7. The treatments 0.03% F, 0.5% LE, 0.1% BOL, and 0.5% BOL were statistically significant versus CTR (p<0.05).

3.4 Protein measurement

The raw data are displayed in Tables T-8 and T-9. It has been found a statistical significance for the treatments 0.03% F and 1% BOL vs CTR with p<0.001, and 0.5% BOL vs CTR with p<0.05.

3.5 Leukocytes (PMN) measurement

The raw data are displayed in Tables T-10 and T-11. All the treatments were statistically significant vs CTR (p<0.001).

3.6 Leukotriene $B_4$ ($LTB_4$) measurement

All samples were under the limit of quantification (about 0.2 ng/ml) of the assay.

3.7 Myeloperoxidase (MPO) measurement

The raw data are displayed in Tables T-12 and T-13. It has been found a statistical significance for the all the treatments vs CTR with p<0.01 for 0.03% F, and p<0.001 for 0.1% Dex, 0.5% LE, 0.1% BOL, 0.5% BOL and 1% BOL.

4. DISCUSSION

The preliminary conclusions from the data generated are:

- BOL-303242-X is active in this model.
- There was not a large difference between these concentrations of BOL-303242-X and NSAID and steroid positive controls.

There was not a profound dose-response for BOL-303242-X, perhaps because we are at either maximal efficacy or maximal drug exposure at these doses. However, the results show that BOL-303242-X is as effective an anti-inflammatory drug as some of the commonly accepted prior-art steroids or NSAID. Some other very preliminary data (not shown) suggest that BOL-303242-X does not have some of the side effects of corticosteroids.

5. REFERENCES

1. Eakins KE (1977). Prostaglandin and non prostaglandin-mediated breakdown of the blood-aqueous barrier. Exp. Eye Res., Vol. 25, 483-498.
2. Neufeld AH, Sears ML (1973). The site of action of prostaglandin E2 on the disruption of the blood-aqueous barrier in the rabbit eye. Exp. Eye Res., Vol. 17, 445-448.
3. Unger WG, Cole DP, Hammond B (1975). Disruption of the blood-aqueous barrier following paracentesis in the rabbit. Exp. Eye Res., Vol. 20, 255-270.
4. Stjemschantz J (1984). Autacoids and Neuropeptides. In: Sears, ML (ed.) Pharmacology of the Eye. Springer-Verlag, New York, pp. 311-365.
5. Williams RN, Paterson CA, Eakins KE, Bhattacherjee P (1983) Quantification of ocular inflammation: evaluation of polymorphonuclear leukocyte infiltration by measuring myeloperoxidase activity. Curr. Eye Res., Vol. 2, 465-469.

Table T-1: Rabbit body weight measured just before the experiment

| Rabbit ID | Sex | Body weight (g) |
|---|---|---|
| A1 | M | 2090 |
| A2 | M | 2140 |
| A3 | M | 2100 |
| A4 | M | 2320 |
| B1 | M | 2270 |
| B2 | M | 2190 |
| B3 | M | 2340 |
| B4 | M | 2300 |
| C1 | M | 2160 |
| C2 | M | 2160 |
| C3 | M | 2280 |
| C4 | M | 2400 |
| D1 | M | 2220 |
| D2 | M | 2200 |
| D3 | M | 2180 |
| D4 | M | 2260 |
| E1 | M | 2170 |
| E2 | M | 2330 |
| E3 | M | 2350 |
| E4 | M | 2300 |
| F1 | M | 2190 |
| F2 | M | 2240 |
| F3 | M | 2120 |
| F4 | M | 2200 |

(continued)

| Rabbit ID | Sex | Body weight (g) |
|---|---|---|
| G1 | M | 2410 |
| G2 | M | 2270 |
| G3 | M | 2310 |
| G4 | M | 2130 |
| Mean ± S.D. | | 2236.8 ± 89.2 |

Table T-2 Raw data of pupillary diameter at -180 min (basal), -5 min (5 min before the first paracentesis) and at +115 min (5 min before the second paracentesis), and calculated difference between the value at +115 min and the value at -180 min.

| Treatment | Rabbit ID | Eye | Diameter (mm) | | | |
|---|---|---|---|---|---|---|
| | | | T1: -180 min | T2: -5 min | T3: +115 min | Δ(T3 - T1) |
| CTR | A1 | DX | 6.0 | 5.5 | 4.0 | -2.0 |
| | | SX | 5.5 | 5.5 | 4.0 | -1.5 |
| | A2 | DX | 6.0 | 6.5 | 4.5 | -1.5 |
| | | SX | 6.0 | 6.5 | 5.0 | -1.0 |
| | A3 | DX | 6.5 | 6.5 | 5.0 | -1.5 |
| | | SX | 6.5 | 6.5 | 5.0 | -1.5 |
| | A4 | DX | 6.0 | 6.5 | 5.0 | -1.0 |
| | | SX | 6.0 | 6.5 | 5.0 | -1.0 |
| | | | | | | |
| 0.03% F | B1 | DX | 5.0 | 6.0 | 4.0 | -1.0 |
| | | SX | 5.0 | 6.0 | 3.5 | -1.5 |
| | B2 | DX | 7.0 | 6.5 | 5.5 | -1.5 |
| | | SX | 6.0 | 7.0 | 5.0 | -1.0 |
| | B3 | DX | 6.0 | 6.5 | 4.5 | -1.5 |
| | | SX | 6.0 | 6.5 | 6.0 | 0.0 |
| | B4 | DX | 5.5 | 6.0 | 5.5 | 0.0 |
| | | SX | 6.0 | 5.5 | 5.0 | -1.0 |
| 0.1% Dex | C1 | DX | 6.0 | 5.5 | 5.5 | -0.5 |
| | | SX | 7.0 | 6.5 | 5.5 | -1.5 |
| | C2 | DX | 5.5 | 6.5 | 6.0 | 0.5 |
| | | SX | 5.5 | 6.0 | 5.5 | 0.0 |
| | C3 | DX | 6.5 | 6.0 | 4.5 | -2.0 |
| | | SX | 6.5 | 6.5 | 5.0 | -1.5 |
| | C4 | DX | 6.5 | 7.0 | 6.0 | -0.5 |
| | | SX | 7.0 | 7.5 | 6.5 | -0.5 |
| | | | | | | |

(continued)

| Treatment | Rabbit ID | Eye | Diameter (mm) | | | |
|---|---|---|---|---|---|---|
| | | | T1: -180 min | T2: -5 min | T3: +115 min | Δ(T3 - T1) |
| 0.5% LE | D1 | DX | 6.0 | 6.0 | 4.5 | -1.5 |
| | | SX | 6.0 | 6.0 | 5.0 | -1.0 |
| | D2 | DX | 6.5 | 6.5 | 5.5 | -1.0 |
| | | SX | 6.5 | 6.5 | 5.5 | -1.0 |
| | D3 | DX | 6.0 | 6.0 | 6.0 | 0.0 |
| | | SX | 6.5 | 6.5 | 6.0 | -0.5 |
| | D4 | DX | 6.5 | 6.5 | 6.0 | -0.5 |
| | | SX | 6.5 | 6.5 | 5.0 | -1.5 |
| | | | | | | |
| 0.1% BOL | E1 | DX | 6.5 | 6.5 | 5.0 | -1.5 |
| | | SX | 6.5 | 6.5 | 6.0 | -0.5 |
| | E2 | DX | 6.5 | 7.0 | 5.0 | -1.5 |
| | | SX | 6.5 | 7.0 | 6.0 | -0.5 |
| | E3 | DX | 7.0 | 7.0 | 6.0 | -1.0 |
| | | SX | 7.5 | 7.5 | 6.5 | -1.0 |
| | E4 | DX | 7.0 | 6.5 | 5.5 | -1.5 |
| | | SX | 7.0 | 7.0 | 5.5 | -1.5 |
| | | | | | | |
| 0.5% BOL | F1 | DX | 8.0 | 8.0 | 6.5 | -1.5 |
| | | SX | 8.0 | 8.0 | 6.5 | -1.5 |
| | F2 | DX | 7.0 | 7.0 | 6.5 | -0.5 |
| | | SX | 7.0 | 7.0 | 6.0 | -1.0 |
| | F3 | DX | 7.5 | 7.5 | 7.0 | -0.5 |
| | | sx | 8.0 | 8.0 | 7.0 | -1.0 |
| | F4 | DX | 7.0 | 7.0 | 6.0 | -1.0 |
| | | SX | 7.5 | 7.0 | 6.5 | -1.0 |
| | | | | | | |
| 1% BOL | G1 | DX | 6.0 | 6.0 | 5.5 | -0.5 |
| | | SX | 6.5 | 6.5 | 5.0 | -1.5 |
| | G2 | DX | 6.0 | 6.5 | 5.0 | -1.0 |
| | | SX | 6.0 | 6.5 | 5.0 | -1.0 |
| | G3 | DX | 6.5 | 7.0 | 5.5 | -1.0 |
| | | SX | 6.5 | 7.0 | 5.0 | -1.5 |
| | G4 | DX | 6.5 | 6.5 | 6.0 | -0.5 |
| | | SX | 6.5 | 6.0 | 6.0 | -0.5 |

Table T-3 Difference between the value of pupillary diameter at T3=+115 min (5 min before the second paracentesis) and the value at T1=-180 min (basal) (Mean ± SEM).

| Treatment | Rabbit Group ID | Mean (mm) Δ(T3 - T1) | SEM | n |
|---|---|---|---|---|
| CTR | A | -1.4 | 0.12 | 8 |
| 0.03% F | B | -0.9 | 0.22 | 8 |
| 0.1% Dex | C | -0.8 | 0.30 | 8 |
| 0.5% LE | D | -0.9 | 0.18 | 8 |
| 0.1% BOL | E | -1.1 | 0.16 | 8 |
| 0.5% BOL | F | -1.0 | 0.13 | 8 |
| 1 % BOL | G | -0.9 | 0.15 | 8 |

Table T-4 Raw data of clinical score at -180 min (basal), -5 min (5 min before the first paracentesis) and at +115 min (5 min before the second paracentesis).

| Treatment | Rabbit ID | Eye | Clinical Score | | |
|---|---|---|---|---|---|
| | | | -180 min | -5 min | +115 min |
| CTR | A1 | DX | 0 | 1 | 3 |
| | | SX | 0 | 1 | 3 |
| | A2 | DX | 0 | 0 | 2 |
| | | SX | 0 | 0 | 2 |
| | A3 | DX | 0 | 0 | 3 |
| | | SX | 0 | 0 | 3 |
| | A4 | DX | 0 | 0 | 3 |
| | | SX | 0 | 0 | 3 |
| | | | | | |
| 0.03% F | B1 | DX | 0 | 0 | 2 |
| | | SX | 0 | 0 | 2 |
| | B2 | DX | 0 | 0 | 2 |
| | | SX | 0 | 0 | 2 |
| | B3 | DX | 0 | 0 | 2 |
| | | SX | 0 | 0 | 2 |
| | B4 | DX | 0 | 0 | 2 |
| | | SX | 0 | 0 | 2 |

(continued)

| Treatment | Rabbit ID | Eye | Clinical Score | | |
|---|---|---|---|---|---|
| | | | -180 min | -5 min | +115 min |
| 0.1% Dex | C1 | DX | 0 | 0 | 1 |
| | | SX | 0 | 0 | 1 |
| | C2 | DX | 0 | 0 | 1 |
| | | SX | 0 | 0 | 1 |
| | C3 | DX | 0 | 1 | 3 |
| | | SX | 0 | 1 | 3 |
| | C4 | DX | 0 | 0 | 1 |
| | | SX | 0 | 0 | 1 |
| | | | | | |
| 0.5% LE | D1 | DX | 0 | 0 | 2 |
| | | SX | 0 | 0 | 2 |
| | D2 | DX | 0 | 0 | 1 |
| | | SX | 0 | 0 | 1 |
| | D3 | DX | 0 | 0 | 1 |
| | | SX | 0 | 0 | 1 |
| | D4 | DX | 0 | 0 | 1 |
| | | SX | 0 | 0 | 1 |
| | | | | | |
| 0.1% BOL | E1 | DX | 0 | 0 | 2 |
| | | SX | 0 | 0 | 2 |
| | E2 | DX | 0 | 0 | 2 |
| | | SX | 0 | 0 | 2 |
| | E3 | DX | 0 | 0 | 2 |
| | | SX | 0 | 0 | 2 |
| | E4 | DX | 0 | 0 | 3 |
| | | SX | 0 | 0 | 3 |
| | | | | | |
| 0.5% BOL | F1 | DX | 0 | 0 | 2 |
| | | SX | 0 | 0 | 2 |
| | F2 | DX | 0 | 0 | 1 |
| | | SX | 0 | 0 | 2 |
| | F3 | DX | 0 | 0 | 1 |
| | | SX | 0 | 0 | 1 |
| | F4 | DX | 0 | 0 | 2 |
| | | SX | 0 | 0 | 2 |
| | | | | | |

(continued)

| Treatment | Rabbit ID | Eye | Clinical Score | | |
|---|---|---|---|---|---|
| | | | -180 min | -5 min | +115 min |
| 1% BOL | G1 | DX | 0 | 0 | 2 |
| | | SX | 0 | 0 | 2 |
| | G2 | DX | 0 | 0 | 2 |
| | | SX | 0 | 0 | 2 |
| | G3 | DX | 0 | 0 | 2 |
| | | SX | 0 | 0 | 2 |
| | G4 | DX | 0 | 0 | 2 |
| | | SX | 0 | 0 | 2 |

Table T-5 Clinical score expressed as percentage of eyes at -180 min (basal), -5 min (5 min before the first paracentesis) and at +115 min (5 min before the second paracentesis).

| Treatment | Rabbit Group ID | N (eyes) | Score (%) | | | | |
|---|---|---|---|---|---|---|---|
| | | | 0 | 1 | 2 | 3 | 4 |
| -180 min | | | | | | | |
| CTR | A | 8 | 100 | -- | -- | -- | -- |
| 0.03% F | B | 8 | 100 | -- | -- | -- | -- |
| 0.1% Dex | C | 8 | 100 | -- | -- | -- | -- |
| 0.5% LE | D | 8 | 100 | -- | -- | -- | -- |
| 0.1% BOL | E | 8 | 100 | -- | -- | -- | -- |
| 0.5% BOL | F | 8 | 100 | -- | -- | -- | -- |
| 1% BOL | G | 8 | 100 | -- | -- | -- | -- |
| -5 min | | | | | | | |
| CTR | A | 8 | 75 | 25 | -- | -- | -- |
| 0.03% F | B | 8 | 100 | -- | -- | -- | -- |
| 0.1% Dex | C | 8 | 75 | 25 | -- | -- | -- |
| 0.5% LE | D | 8 | 100 | -- | -- | -- | -- |
| 0.1% BOL | E | 8 | 100 | -- | -- | -- | -- |
| 0.5% BOL | F | 8 | 100 | -- | -- | -- | -- |
| 1% BOL | G | 8 | 100 | -- | -- | -- | -- |
| +115 min | | | | | | | |
| CTR | A | 8 | -- | -- | 25 | 75 | -- |
| 0.03% F | B | 8 | -- | -- | 100 | -- | -- |
| 0.1% Dex | C | 8 | -- | 75 | -- | 25 | -- |
| 0.5% LE | D | 8 | -- | 75 | 25 | -- | -- |
| 0.1% BOL | E | 8 | -- | -- | 75 | 25 | -- |
| 0.5% BOL | F | 8 | -- | 37.5 | 62.5 | -- | -- |
| 1% BOL | G | 8 | -- | -- | 100 | -- | -- |

Table T-6 Raw data of $PGE_2$ levels in aqueous humor samples collected at the second paracentesis

| Treatment | Sample | $PGE_2$ (ng/ml) |
|---|---|---|
| CTR | 2-A1-DX | 3.81 |
| | 2-A1-SX | 2.91 |
| | 2-A2-DX | 4.77 |
| | 2-A2-SX | [1]N/A |
| | 2-A3-DX | 1.46 |
| | 2-A3-SX | 3.00 |
| | 2-A4-DX | 1.87 |
| | 2-A4-SX | 1.88 |
| | | |
| 0.03% F | 2-B1-DX | 1.04 |
| | 2-B1-SX | 0.75 |
| | 2-B2-DX | 0.85 |
| | 2-B2-SX | 1.11 |
| | 2-B3-DX | 2.11 |
| | 2-B3-SX | 0.93 |
| | 2-B4-DX | 0.61 |
| | 2-B4-SX | 2.11 |
| | | |
| 0.1% Dex | 2-C1-DX | 2.51 |
| | 2-C1-SX | N/A |
| | 2-C2-DX | 2.32 |
| | 2-C2-SX | N/A |
| | 2-C3-DX | 2.10 |
| | 2-C3-SX | 3.03 |
| | 2-C4-DX | 2.32 |
| | 2-C4-SX | 1.30 |
| | | |
| 0.5% LE | 2-D1-DX | [2]N/D |
| | 2-D1-SX | N/D |
| | 2-D2-DX | N/D |
| | 2-D2-SX | 0.23 |
| | 2-D3-DX | N/D |
| | 2-D3-SX | 0.68 |
| | 2-D4-DX | N/D |
| | 2-D4-SX | 1.10 |

(continued)

| Treatment | Sample | $PGE_2$ (ng/ml) |
|---|---|---|
| 0.1% BOL | 2-E1-DX | 1.62 |
| | 2-E1-SX | 1.88 |
| | 2-E2-DX | 2.15 |
| | 2-E2-SX | 0.70 |
| | 2-E3-DX | 1.34 |
| | 2-E3-SX | 1.03 |
| | 2-E4-DX | N/D |
| | 2-E4-SX | N/D |
| | | |
| 0.5% BOL | 2-F1-DX | 2.31 |
| | 2-F1-SX | 2.59 |
| | 2-F2-DX | N/D |
| | 2-F2-SX | 0.53 |
| | 2-F3-DX | 0.75 |
| | 2-F3-SX | 0.80 |
| | 2-F4-DX | 1.62 |
| | 2-F4-SX | 1.09 |
| | | |
| 1% BOL | 2-G1-DX | 0.50 |
| | 2-G1-SX | 1.87 |
| | 2-G2-DX | 1.71 |
| | 2-G2-SX | 4.04 |
| | 2-G3-DX | 1.11 |
| | 2-G3-SX | 3.78 |
| | 2-G4-DX | N/D |
| | 2-G4-SX | N/D |

[1]N/A = not available
[2]N/D = not detectable, under the limit of quantification

Table T-7 Levels of $PGE_2$ in aqueous humor samples collected at the second paracentesis (Mean $\pm$ SEM).

| Treatment | Sample Group | Mean (ng/ml) | SEM | n |
|---|---|---|---|---|
| CTR | A | 2.815 | 0.449 | 7 |
| 0.03% F | B | 1.189 | 0.209 | 8 |
| 0.1% Dex | C | 2.263 | 0.232 | 6 |
| 0.5% LE | D | 0.672 | 0.250 | 3 |
| 0.1% BOL | E | 1.452 | 0.221 | 6 |
| 0.5% BOL | F | 1.384 | 0.306 | 7 |
| 1% BOL | G | 2.168 | 0.586 | 6 |

Table T-8 Raw data of protein levels in aqueous humor samples collected at the second paracentesis

| Treatment | Sample | Protein (mg/ml) |
|---|---|---|
| CTR | 2-A1-DX | 50.24 |
| | 2-A1-SX | 53.51 |
| | 2-A2-DX | 28.73 |
| | 2-A2-SX | [1]N/A |
| | 2-A3-DX | 40.09 |
| | 2-A3-SX | 30.84 |
| | 2-A4-DX | 41.79 |
| | 2-A4-SX | 30.35 |
| | | |
| 0.03% F | 2-B1-DX | 20.78 |
| | 2-B1-SX | 28.80 |
| | 2-B2-DX | N/A |
| | 2-B2-SX | 23.41 |
| | 2-B3-DX | 20.21 |
| | 2-B3-SX | 17.53 |
| | 2-B4-DX | 15.12 |
| | 2-B4-SX | 20.52 |
| | | |
| 0.1% Dex | 2-C1-DX | 31.31 |
| | 2-C1-SX | N/A |
| | 2-C2-DX | 31.81 |
| | 2-C2-SX | N/A |
| | 2-C3-DX | 35.95 |
| | 2-C3-SX | 37.15 |
| | 2-C4-DX | 32.12 |
| | 2-C4-SX | 32.40 |
| | | |
| 0.5% LE | 2-D1-DX | 36.14 |
| | 2-D1-SX | 39.10 |
| | 2-D2-DX | 34.69 |
| | 2-D2-SX | 26.10 |
| | 2-D3-DX | 26.30 |
| | 2-D3-SX | 28.16 |
| | 2-D4-DX | 40.90 |
| | 2-D4-SX | 39.85 |

(continued)

| Treatment | Sample | Protein (mg/ml) |
|---|---|---|
| 0.1% BOL | 2-E1-DX | 34.87 |
| | 2-E1-SX | 34.41 |
| | 2-E2-DX | 31.14 |
| | 2-E2-SX | 22.82 |
| | 2-E3-DX | 29.46 |
| | 2-E3-SX | 31.69 |
| | 2-E4-DX | 35.70 |
| | 2-E4-SX | 49.25 |
| | | |
| 0.5% BOL | 2-F1-DX | 33.98 |
| | 2-F1-SX | 33.65 |
| | 2-F2-DX | 19.99 |
| | 2-F2-SX | 27.11 |
| | 2-F3-DX | 19.72 |
| | 2-F3-SX | 36.35 |
| | 2-F4-DX | 27.71 |
| | 2-F4-SX | 32.24 |
| | | |
| 1% BOL | 2-G1-DX | 20.99 |
| | 2-G1-SX | 21.48 |
| | 2-G2-DX | 15.11 |
| | 2-G2-SX | 20.28 |
| | 2-G3-DX | 20.94 |
| | 2-G3-SX | 21.89 |
| | 2-G4-DX | 20.03 |
| | 2-G4-SX | 30.76 |
| [1]N/A = not available | | |

Table T-9 Protein levels in aqueous humor samples collected at the second paracentesis (Mean ± SEM).

| Treatment | Sample Group | Mean (mg/ml) | SEM | n |
|---|---|---|---|---|
| CTR | A | 39.364 | 3.754 | 7 |
| 0.03% F | B | 20.910 | 1.648 | 7 |
| 0.1% Dex | C | 33.457 | 1.001 | 6 |
| 0.5% LE | D | 33.905 | 2.190 | 8 |
| 0.1% BOL | E | 33.667 | 2.655 | 8 |
| 0.5% BOL | F | 28.844 | 2.249 | 8 |
| 1% BOL | G | 21.435 | 1.529 | 8 |

Table T-10 Raw data of PMN numbers in aqueous humor samples collected at the second paracentesis

| Treatment | Sample | PMN (number/$\mu$l) |
|---|---|---|
| CTR | 2-A1-DX | 90 |
| | 2-A1-SX | 80 |
| | 2-A2-DX | 70 |
| | 2-A2-SX | [1]N/A |
| | 2-A3-DX | 70 |
| | 2-A3-SX | 80 |
| | 2-A4-DX | 50 |
| | 2-A4-SX | 40 |
| | | |
| 0.03% F | 2-B1-DX | 50 |
| | 2-B1-SX | 40 |
| | 2-B2-DX | N/A |
| | 2-B2-SX | 20 |
| | 2-B3-DX | 10 |
| | 2-B3-SX | 40 |
| | 2-B4-DX | 30 |
| | 2-B4-SX | 20 |
| | | |
| 0.1% Dex | 2-C1-DX | 20 |
| | 2-C1-SX | N/A |
| | 2-C2-DX | 20 |
| | 2-C2-SX | N/A |
| | 2-C3-DX | 50 |
| | 2-C3-SX | 40 |
| | 2-C4-DX | 20 |
| | 2-C4-SX | 30 |
| | | |
| 0.5% LE | 2-D1-DX | N/A |
| | 2-D1-SX | N/A |
| | 2-D2-DX | 40 |
| | 2-D2-SX | 20 |
| | 2-D3-DX | 20 |
| | 2-D3-SX | 30 |
| | 2-D4-DX | 40 |
| | 2-D4-SX | 20 |

(continued)

| Treatment | Sample | PMN (number/μl) |
|---|---|---|
| 0.1% BOL | 2-E1-DX | N/A |
| | 2-E1-SX | 20 |
| | 2-E2-DX | 40 |
| | 2-E2-SX | 50 |
| | 2-E3-DX | 20 |
| | 2-E3-SX | 20 |
| | 2-E4-DX | 20 |
| | 2-E4-SX | N/A |
| | | |
| 0.5% BOL | 2-F1-DX | 40 |
| | 2-F1-SX | 20 |
| | 2-F2-DX | 20 |
| | 2-F2-SX | 10 |
| | 2-F3-DX | 10 |
| | 2-F3-SX | 10 |
| | 2-F4-DX | 20 |
| | 2-F4-SX | 40 |
| | | |
| 1% BOL | 2-G1-DX | 30 |
| | 2-G1-SX | 20 |
| | 2-G2-DX | 30 |
| | 2-G2-SX | 40 |
| | 2-G3-DX | 20 |
| | 2-G3-SX | 30 |
| | 2-G4-DX | 40 |
| | 2-G4-SX | 20 |
| [1]N/A = not available | | |

Table T-11 PMN numbers in aqueous humor samples collected at the second paracentesis (Mean ± SEM).

| Treatment | Sample Group | Mean (number/μl) | SEM | n |
|---|---|---|---|---|
| CTR | A | 68.571 | 6.701 | 7 |
| 0.03% F | B | 30.000 | 5.345 | 7 |
| 0.1% Dex | C | 30.000 | 5.164 | 6 |
| 0.5% LE | D | 28.333 | 4.014 | 6 |
| 0.1% BOL | E | 28.333 | 5.426 | 6 |
| 0.5% BOL | F | 21.250 | 4.407 | 8 |
| 1% BOL | G | 28.750 | 2.950 | 8 |

Table T-12 Raw data of MPO activity in iris-ciliary body samples collected after the second paracentesis.

| Treatment | Sample | Iris-ciliary body weight (mg) | [1]Volume (μl) | [2]Δ/min | MPO Unit/g |
|---|---|---|---|---|---|
| CTR | A1-DX | 41.7 | 40 | 0.021 | 1.11 |
| | A1-SX | 42.3 | 40 | 0.024 | 1.26 |
| | A2-DX | 46.6 | 40 | 0.039 | 1.85 |
| | A2-SX | 40.5 | 40 | 0.037 | 2.02 |
| | A3-DX | 48.9 | 40 | 0.075 | 3.39 |
| | A3-SX | 51.1 | 40 | 0.049 | 2.12 |
| | A4-DX | 36.6 | 40 | 0.013 | 0.79 |
| | A4-SX | 38.8 | 40 | 0.019 | 1.08 |
| | | | | | |
| 0.03% F | B1-DX | 39.5 | 100 | 0.049 | 1.10 |
| | B1-SX | 42.7 | 100 | 0.082 | 1.70 |
| | B2-DX | 34.1 | 100 | 0.013 | 0.34 |
| | B2-SX | 36.6 | 100 | 0.031 | 0.75 |
| | B3-DX | 45.6 | 100 | 0.038 | 0.74 |
| | B3-SX | 38.0 | 100 | 0.027 | 0.63 |
| | B4-DX | 40.1 | 100 | 0.033 | 0.73 |
| | B4-SX | 42.6 | 100 | 0.061 | 1.27 |
| | | | | | |
| 0.1% Dex | C1-DX | 36.4 | 100 | 0.029 | 0.71 |
| | C1-SX | 45.8 | 100 | 0.031 | 0.60 |
| | C2-DX | 42.9 | 100 | 0.064 | 1.32 |
| | C2-SX | 42.7 | 100 | 0.023 | 0.48 |
| | C3-DX | 43.0 | 100 | 0.019 | 0.39 |
| | C3-SX | 46.8 | 100 | 0.024 | 0.45 |
| | C4-DX | 42.3 | 100 | 0.023 | 0.48 |
| | C4-SX | 36.1 | 100 | 0.021 | 0.51 |
| | | | | | |
| 0.5% LE | D1-DX | 38.9 | 200 | 0.026 | 0.30 |
| | D1-SX | 44.7 | 200 | 0.053 | 0.51 |
| | D2-DX | 35.9 | 200 | 0.067 | 0.81 |
| | D2-SX | 40.7 | 200 | 0.055 | 0.60 |
| | D3-DX | 46.3 | 200 | 0.076 | 0.73 |
| | D3-SX | 41.9 | 200 | 0.096 | 1.01 |
| | D4-DX | 46.7 | [3]N/A | N/A | N/A |
| | D4-SX | 32.9 | N/A | N/A | N/A |

(continued)

| Treatment | Sample | Iris-ciliary body weight (mg) | [1]Volume (μl) | [2]Δ/min | MPO Unit/g |
|---|---|---|---|---|---|
| 0.1% BOL | E1-DX | 43.6 | 100 | 0.051 | 1.04 |
| | E1-SX | 37.2 | 100 | 0.042 | 1.00 |
| | E2-DX | 32.6 | 100 | 0.042 | 1.14 |
| | E2-SX | 37.4 | 100 | 0.045 | 1.06 |
| | E3-DX | 36.2 | 100 | 0.050 | 1.22 |
| | E3-SX | 45.1 | 100 | 0.031 | 0.61 |
| | E4-DX | 30.4 | 100 | 0.036 | 1.05 |
| | E4-SX | 42.3 | 100 | 0.031 | 0.65 |
| | | | | | |
| 0.5% BOL | F1-DX | 45.8 | 100 | 0.044 | 0.85 |
| | F1-SX | 38.2 | 100 | 0.040 | 0.93 |
| | F2-DX | 34.9 | 100 | 0.031 | 0.79 |
| | F2-SX | 42.0 | 100 | 0.049 | 1.03 |
| | F3-DX | 39.1 | 100 | 0.033 | 0.75 |
| | F3-SX | 40.6 | 100 | 0.034 | 0.74 |
| | F4-DX | 36.2 | 100 | 0.022 | 0.54 |
| | F4-SX | 39.5 | 100 | 0.026 | 0.58 |
| | | | | | |
| 1% BOL | G1-DX | 32.4 | 100 | 0.024 | 0.66 |
| | G1-SX | 43.1 | 100 | 0.033 | 0.68 |
| | G2-DX | 30.6 | 100 | 0.017 | 0.49 |
| | G2-SX | 39.9 | 100 | 0.018 | 0.40 |
| | G3-DX | 41.3 | 100 | 0.016 | 0.34 |
| | G3-SX | 44.9 | 100 | 0.052 | 1.02 |
| | G4-DX | 36.6 | 100 | 0.013 | 0.31 |
| | G4-SX | 36.9 | 100 | 0.018 | 0.43 |

[1]Volume = aliquot (μl) of the supernatant diluted to 3ml for the analysis.
[2]Δ/min = mean of the slope of the line recorded every 15 sec for 5 min
[3]N/A = not available

Table T-13 MPO activity in iris-ciliary body samples collected after the second paracentesis (Mean ± SEM).

| Treatment | Sample Group | Mean MPO Unit/g | SEM | n |
|---|---|---|---|---|
| CTR | A | 1.703 | 0.297 | 8 |
| 0.03% F | B | 0.906 | 0.151 | 8 |
| 0.1% Dex | C | 0.618 | 0.106 | 8 |
| 0.5% LE | D | 0.661 | 0.102 | 6 |
| 0.1% BOL | E | 0.971 | 0.079 | 8 |
| 0.5% BOL | F | 0.775 | 0.058 | 8 |

(continued)

| Treatment | Sample Group | Mean MPO Unit/g | SEM | n |
|---|---|---|---|---|
| 1% BOL | G | 0.542 | 0.083 | 8 |

TESTING 2: Effect of BOL-303242-X on Inhibiting IL-1β-Induced Cytokine Expression in Human Corneal Epithelial Cells

1. BACKGROUND/RATIONALE:

Levels of cytokines associated with immune cells are direct indications of activity of these cells in an inflammatory condition. Reduced levels of these cytokines indicate a positive therapeutic effect on inflammation of a test compound. This study was designed to determine the effect of BOL-303242-X on IL-1ß -induced cytokine production in human corneal epithelial cells ("HCECs").

1. PURPOSE

To determine the effects of BOL-303242-X on IL-1ß-stimulated cytokine expression in primary human corneal epithelial cells using a 30-cytokine Luminex kit. Dexamethasone was used as a control..

3. EXPERIMENTAL DESIGN

Primary HCECs were seeded in 24-well plates. After 24 h, cells were treated with vehicle, IL-1ß, IL-1ß + dexamethasone, or IL-1ß + BOL-303242-X in basic EpiLife medium for 18 h (Table T-14). Each treatment was performed in triplicate. Media were collected and used for determination of cytokine content using a 30-cytokine Luminex kit. Cell viability was determined by alamarBlue assay (LP06013).

| Group* | Day 1 | Day 2: cells were treated with the test agents in basic EpiLife medium for 18 h | Day 3 |
|---|---|---|---|
| 1 | Cells were seeded in 24-well plates (5 X | Control (0.1% DMSO) | Media for Luminex assays; cells for cell viability |
| 2 | | 10 ng/ml IL-1ß | |
| 3 | | 10 ng/ml IL-1ß + 1 nM dexamethasone | |
| 4 | | 10 ng/ml IL-1ß + 10 nM dexamethasone | |
| 5 | | 10 ng/ml IL-1ß + 100 nM dexamethasone | |
| 6 | | 10 ng/ml IL-1ß + 1 μM dexamethasone | |

(continued)

| Group* | Day 1 | Day 2: cells were treated with the test agents in basic EpiLife medium for 18 h | Day 3 |
|---|---|---|---|
| 7 | $10^5$/well in 0.5 ml medium) in EpiLife medium | 10 ng/ml IL-1ß + 10 μM dexamethasone | assay |
| 8 | | 10 ng/ml IL-1ß + 1 nM BOL-303242-X | |
| 9 | | 10 ng/ml IL-1ß + 10 nM BOL-303242-X | |
| 10 | | 10 ng/ml IL-1ß + 100 nM BOL-303242-X | |
| 11 | | 10 ng/ml IL-1ß + 1 μM BOL-303242-X | |
| 12 | | 10 ng/ml IL-1ß + 10 μM BOL-303242-X | |
| *triplicate wells per group | | | |

Dexamethasone:

Lot Number: 016K14521
Parent MW: 392.46
Parent:Total MW Ratio = 1.0

BOL-303242-X:

Lot Number: 6286
Parent MW: 462.48
Parent:Total MW Ratio = 1.0

4. DATA ANALYSIS

Median fluorescence intensity (MFI) was used to obtain the concentration of each cytokines in pg/ml based on the standard curve of each cytokine assayed by Luminex. The linear range of the standard curve for each cytokine was used for determination of cytokine concentration. Duplicate values for each sample were averaged. Data were expressed as mean ± SD. Statistical analysis was performed using one-way ANOVA-Dunnett's test, and $P < 0.05$ was considered statistically significant.

5. RESULTS

No statistically significant effect on cellular metabolic activity (as measured by alamarBlue assay) was observed with the various treatments.

Substantial amounts of 16 out of 30 cytokines tested were detected in this study and 13 out of 14 cytokines detected were stimulated by 10 ng/ml IL-1ß (Table T-14). IL-1β was excluded from analysis because it was the stimulus. IL-1ra was excluded because the MFI was not within the standard range.

Dexamethasone and BOL-303242-X significantly inhibited IL-1ß-stimulated cytokine production with comparable potency on 6 cytokines (IL-6, IL-7, MCP-1, TGF-α, TNF-α and VEGF), and a significant inhibitory effect was observed at 1 nM on IL-6 and at 10 nM on MCP-1, TGF-α and TNF-α (Table T-14 and Figures 1A-1F).

BOL-303242-X also significantly inhibited IL-1ß-stimulated G-CSF production with better potency compared to dexamethasone, and a significant inhibitory effect was observed at 10 μg/ml by BOL-303242-X while no significant effect was observed by dexamethasone on this cytokine (Fig. 2).

BOL-303242-X also significantly inhibited IL-1ß-stimulated cytokine production with less potency compared to dexamethasone on 3 cytokines (GM-CSF, IL-8, and RANTES). A significant inhibitory effect was observed at 1 nM by

dexamethasone and at 10 nM by BOL-303242-X on GM-CSF. A significant inhibitory effect was observed at 1 μM by dexamethasone on RANTES while no significant effect was observed by BOL-303242-X on this cytokine (Figures 3A-3C).

6. CONCLUSION

BOL-303242-X and dexamethasone have comparable potency for inhibition of IL-1ß-stimulated cytokine production in HCECs for the cases of IL-6, IL-7, TGF-α, TNF-α, VGEF, and MCP-1. BOL-303242-X is more potent than dexamethasone in inhibiting IL-1ß-stimulated production of G-CSF in HCECs. BOL-303242-X is somewhat less potent than dexamethasone in inhibiting IL-1ß-stimulated production of GM-CSF, IL-8, and RANTES in HCECs.

Table T-14

| Inhibition of IL-1β stimulated cytokine production by dexamethasone and BOL-303242-X in primary human corneal epithelial cells | | | | | | | | | | | |
|---|---|---|---|---|---|---|---|---|---|---|---|
| Cytokines detected * | Stimulated by IL-1β (10 ng/ml) | Inhibited by dexamethasone (μM) | | | | | Inhibited by BOL-303242-X (μM) | | | | |
| | | 0.0 01 | 0.0 1 | 0.1 | 1 | 10 | 0.0 01 | 0.0 1 | 0.1 | 1 | 10 |
| G-CSF | X | | | | | | | | | | X |
| GM-CSF | X | X | X | X | X | X | | X | X | | X |
| IL-1α | X | | | | | | | | | | |
| IL-6 | X | X | X | X | X | X | X | X | X | X | X |
| IL-7 | X | | | x | | | | | | | X |
| IL-8 | X | | | X | X | | | X | | | |
| IP-10 | X | | | | | | | | | | |
| MCP-1 | X | | X | X | X | X | | X | X | X | X |
| MIP-1α | | | | | | | | | | | |
| MIP-1β | x | | | | | | | | | | |
| RANTES | X | | | | X | X | | | | | |
| TGF-α | X | | X | X | X | X | | X | X | X | X |
| TNF-α | X | | X | X | X | X | | X | | | X |
| VEGF | X | | | X | X | | | | | X | X |
| Notes: (*) EGF, Eotaxin, Fractalkine, IFNγ, IL-10, IL-12p40, IL-12p70, IL-13, IL15, IL-17, IL-2, IL-4, IL-5, sCD40L were not detected. IL-1β was excluded from analysis because it was the stimulus. IL-1ra was excluded because the MFI was out of range of the standards. | | | | | | | | | | | |

While specific embodiments of the present invention have been described in the foregoing, it will be appreciated by those skilled in the art that many equivalents, modifications, substitutions, and variations may be made thereto without departing from the spirit and scope of the invention as defined in the appended claims.

## Claims

1. A composition for use in treating or controlling post-operative ocular inflammation of the anterior segment of an eye, said inflammation resulting from a procedure selected from the group consisting of photorefractive keratectomy, cataract removal surgery, intraocular lens ("IOL") implantation, laser-assisted in situ keratomileusis ("LASIK"), conductive keratoplasty, and radial keratotomy; said composition comprising a dissociated glucocorticoid receptor agonist ("DIGRA"), or a pharmaceutically acceptable salt thereof; wherein the DIGRA comprises a compound having Formula I

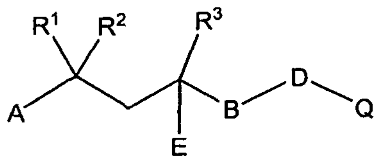

(I)

wherein A comprises a dihydrobenzofuranyl group substituted with a halogen atom; Q comprises a quinolinyl or isoquinolinyl group substituted with a $C_1$-$C_{10}$ alkyl group; $R^1$ and $R^2$ are independently selected from the group consisting of unsubstituted and substituted $C_1$-$C_5$ alkyl groups; B is a $C_1$-$C_3$ alkylene group; D is the -NH- group; E is the hydroxy group; and $R^3$ comprises a completely halogenated $C_1$-$C_{10}$ alkyl group.

2. The composition for use according to claim 1, wherein the composition causes a lower level of at least an adverse side effect in a subject than another composition comprising at least a glucocorticoid used to treat or control the same ocular inflammation, wherein said adverse side effect is an increased intraocular pressure.

3. The composition for use according to claim 2, wherein the DIGRA has Formula I

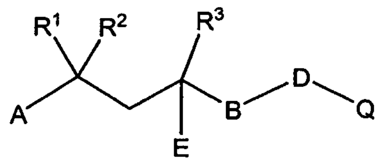

(I)

wherein A comprises a dihydrobenzofuranyl group substituted with a fluorine atom; Q comprises a quinolinyl or isoquinolinyl group substituted with a methyl group; $R^1$ and $R^2$ are independently selected from the group consisting of unsubstituted and substituted $C_1$-$C_5$ alkyl groups; B is a $C_1$-$C_3$ alkylene group; D is the -NH- group; E is the hydroxy group; and $R^3$ comprises a trifluoromethyl group.

4. The composition for use according to claim 2, wherein the DIGRA has Formula II or III

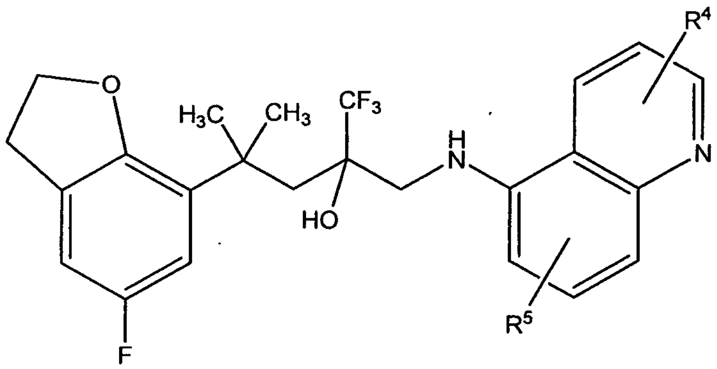

(II)

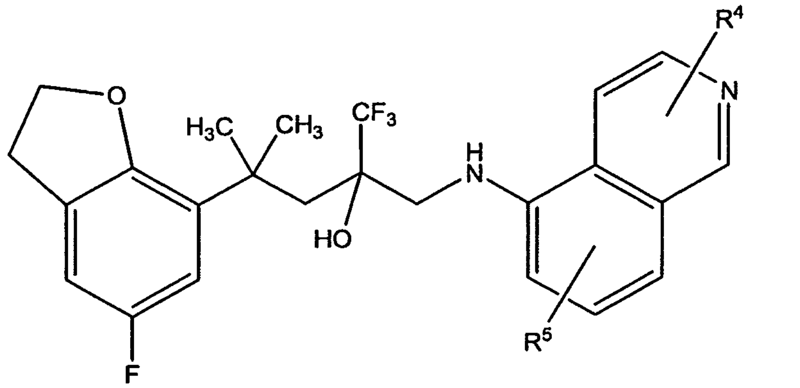

(III)

wherein $R^4$ and $R^5$ are independently selected from the group consisting of unsubstituted $C_1$-$C_{10}$ linear or branched alkyl groups, substituted $C_1$-$C_{10}$ linear or branched alkyl groups, unsubstituted $C_3$-$C_{10}$ cyclic alkyl groups, and substituted $C_3$-$C_{10}$ cyclic alkyl groups.

5. The composition for use according to claim 3, wherein the DIGRA has Formula IV

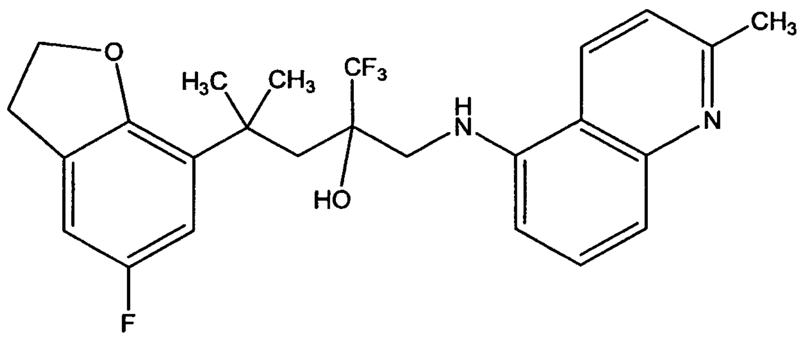

(IV)

6. The composition for use according to claim 5, further comprising an anti-inflammatory agent selected from the group consisting ofNSAIDs, PPAR agonists, combinations thereof, and mixtures thereof.

7. A method for manufacturing a composition for treating or controlling post-operative ocular inflammation of the anterior segment of an eye, said inflammation resulting from a procedure selected from the group consisting of photorefractive keratectomy, cataract removal surgery, intraocular lens ("IOL") implantation, laser-assisted in situ keratomileusis ("LASIK"), conductive keratoplasty, and radial keratotomy; the method comprising:

(a) providing a DIGRA, or a pharmaceutically acceptable salt thereof; and
(b) combining said DIGRA, or pharmaceutically acceptable salt thereof with a pharmaceutically acceptable carrier;
wherein the DIGRA comprises a compound having Formula I

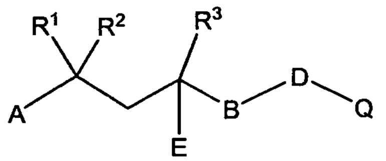

(I)

wherein A comprises a dihydrobenzofuranyl group substituted with a halogen atom; Q comprises a quinolinyl or isoquinolinyl group substituted with a $C_1$-$C_{10}$ alkyl group; $R^1$ and $R^2$ are independently selected from the group consisting of unsubstituted and substituted $C_1$-$C_5$ alkyl groups; B is a $C_1$-$C_3$ alkylene group; D is the -NH- group; E is the hydroxy group; and $R^3$ comprises a completely halogenated $C_1$-$C_{10}$ alkyl group.

8. The method of claim 7, wherein the DIGRA has Formula II or III

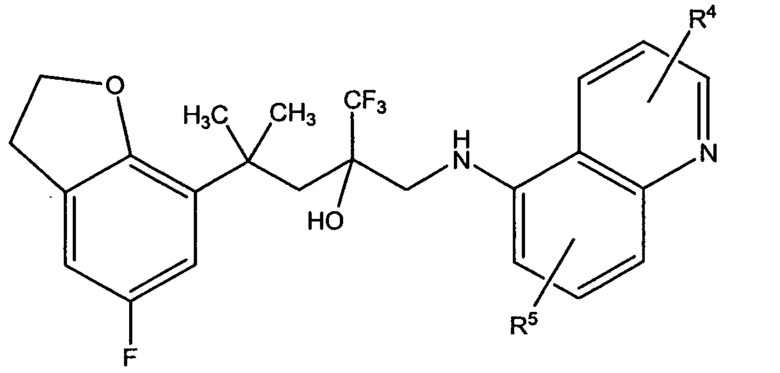

(II)

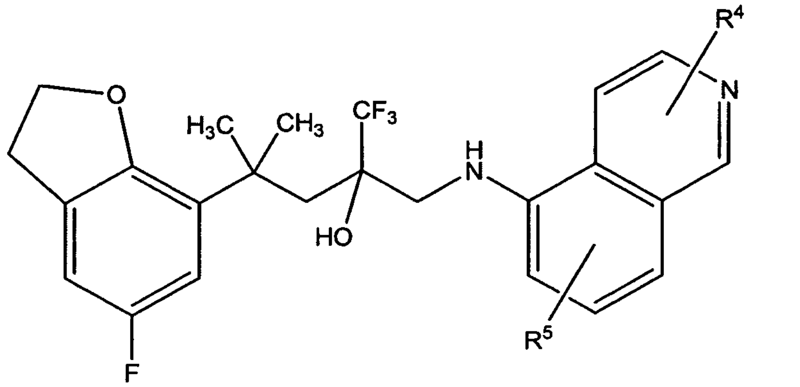

(III)

wherein $R^4$ and $R^5$ are independently selected from the group consisting of unsubstituted $C_1$-$C_{10}$ linear or branched alkyl groups, substituted $C_1$-$C_{10}$ linear or branched alkyl groups, unsubstituted $C_3$-$C_{10}$ cyclic alkyl groups, and substituted $C_3$-$C_{10}$ cyclic alkyl groups.

9. The method of claim 7, wherein the DIGRA has Formula IV

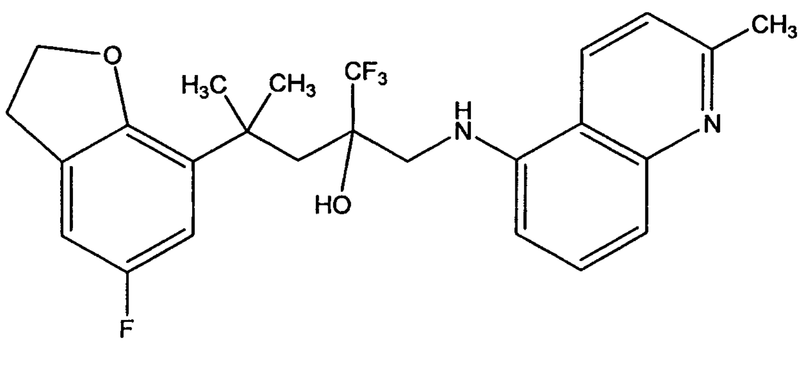

(IV)

**Patentansprüche**

1. Eine Zusammensetzung zur Verwendung in der Behandlung oder Kontrolle von postoperativer Augenentzündung des anterioren Segments eines Auges, wobei die besagte Entzündung aus einem Verfahren resultiert, das ausgewählt ist aus der Gruppe bestehend aus photorefraktiver Keratektomie, Operation zur Entfernung des Katarakts, Implantation von Intraokularlinsen ("IOL"), Laser-unterstützter in-situ-Keratomileusis ("LASIK"), konduktiver Keratoplatsik, und radialer Keratotomie; wobei die besagte Zusammensetzung einen dissoziierten Glucocorticoid-Rezeptor-Agonisten ("DIGRA") oder ein pharmazeutisch verträgliches Salz davon umfasst; wobei der DIGRA eine Verbindung der Formel I umfasst

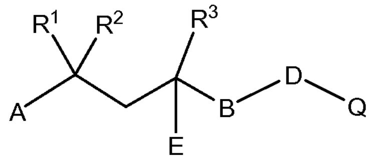

(I)

wobei A eine Dihydrobenzofuranylgruppe umfasst, die mit einem Halogenatom substituiert ist; Q eine Chinolinyl- oder Isochinolinylgruppe umfasst, die mit einer $C_1$-$C_{10}$-Alkylgruppe substituiert ist; $R^1$ und $R^2$ unabhängig ausgewählt sind aus der Gruppe bestehend aus unsubstituierten und substituierten $C_1$-$C_5$-Alkylgruppen; B eine $C_1$-$C_3$-Alkylengruppe ist; D eine -NH-Gruppe ist; E eine Hydroxygruppe ist; und $R^3$ eine vollständig halogenierte $C_1$-$C_{10}$-Alkylgruppe umfasst.

2. Die Zusammensetzung zur Verwendung gemäß Anspruch 1, wobei die Zusammensetzung einen geringeren Grad einer mindestens aversiven Nebenwirkung bei einem Subjekt verursacht als eine andere Zusammensetzung, die mindestens ein Glucocorticoid zur Behandlung oder Kontrolle der gleichen Augenentzündung umfasst, wobei die besagte aversive Nebenwirkung ein erhöhter Augeninnendruck ist.

3. Die Zusammensetzung zur Verwendung gemäß Anspruch 2, wobei der DIGRA die Formel I besitzt

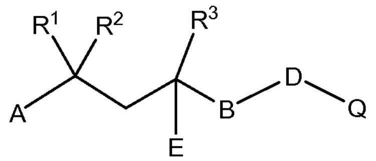

(I)

wobei A eine Dihydrobenzofuranylgruppe umfasst, die mit einem Fluoratom substituiert ist; Q eine Chinolinyl- oder Isochinolinylgruppe umfasst, die mit einer Methylgruppe substituiert ist; $R^1$ und $R^2$ unabhängig ausgewählt sind aus der Gruppe bestehend aus unsubstituierten und substituierten $C_1$-$C_5$-Alkylgruppen; B eine $C_1$-$C_3$-Alkylengruppe ist; D eine -NH-Gruppe ist; E eine Hydroxygruppe ist; und $R^3$ eine Trifluormethylgruppe umfasst.

4. Die Zusammensetzung zur Verwendung gemäß Anspruch 2, wobei der DIGRA die Formel II oder III besitzt

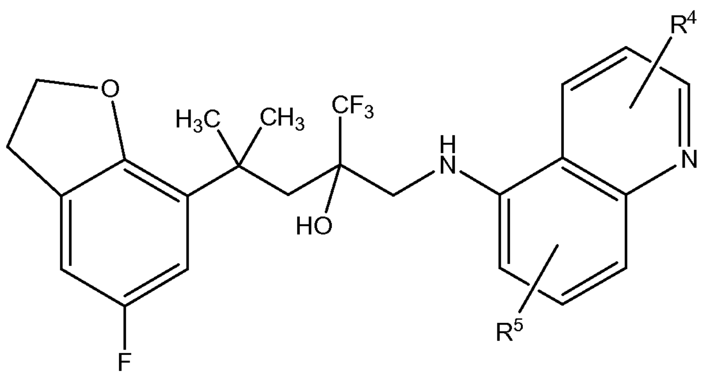

(II)

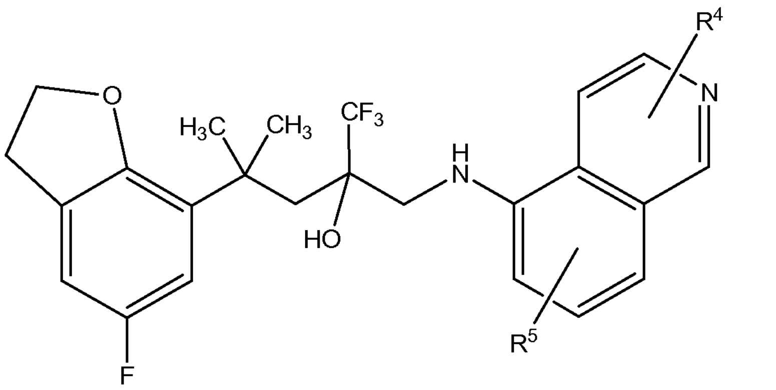

(III)

wobei $R^4$ und $R^5$ unabhängig ausgewählt sind aus der Gruppe bestehend aus unsubstituierten linearen oder verzweigten $C_1$-$C_{10}$-Alkylgruppen, substituierten linearen oder verzweigten $C_1$-$C_{10}$-Alkylgruppen, unsubstituierten cyclischen $C_3$-$C_{10}$-Alkylgruppen, und substituierten cyclischen $C_3$-$C_{10}$-Alkylgruppen.

**5.** Die Zusammensetzung zur Verwendung gemäß Anspruch 3, wobei der DIGRA die Formel IV besitzt

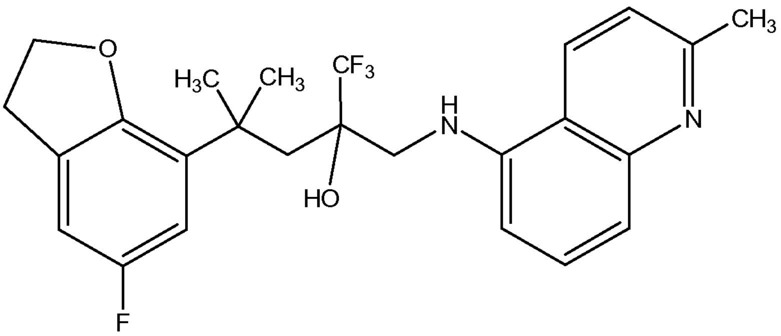

(IV)

**6.** Die Zusammensetzung zur Verwendung gemäß Anspruch 5, weiter umfassend einen entzündungshemmenden Wirkstoff, der ausgewählt ist aus der Gruppe bestehend aus NSAIDs, PPAR Agonisten, Kombinationen davon, und Mischungen davon.

**7.** Ein Verfahren zur Herstellung einer Zusammensetzung zur Behandlung oder Kontrolle von postoperativer Augenentzündung des anterioren Segments eines Auges, wobei die besagte Entzündung aus einem Verfahren resultiert, das ausgewählt ist aus der Gruppe bestehend aus photorefraktiver Keratektomie, Operation zur Entfernung des Katarakts, Implantation von Intraokularlinsen ("IOL"), Laser-unterstützter in-situ- Keratomileusis ("LASIK"), konduktiver Keratoplatsik, und radialer Keratotomie; das Verfahren umfassend:

(a) Bereitstellen eines DIGRA, oder eines pharmazeutisch verträglichen Salzes davon; und
(b) Mischen des besagten DIGRA, oder eines pharmazeutisch vertäglichen Salzes davon mit einem pharmazeutisch verträglichen Träger;

wobei der DIGRA eine Verbindung der Formel I umfasst

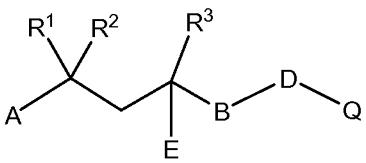

(I)

wobei A eine Dihydrobenzofuranylgruppe umfasst, die mit einem Halogenatom substituiert ist; Q eine Chinolinyl- oder Isochinolinylgruppe umfasst, die mit einer $C_1$-$C_{10}$-Alkylgruppe substituiert ist; $R^1$ und $R^2$ unabhängig ausgewählt

sind aus der Gruppe bestehend aus unsubstituierten und substituierten $C_1$-$C_5$-Alkylgruppen; B eine $C_1$-$C_3$-Alkylengruppe ist; D eine -NH-Gruppe ist; E eine Hydroxygruppe ist; und $R^3$ eine vollständig halogenierte $C_1$-$C_{10}$-Alkylgruppe umfasst.

**8.** Das Verfahren gemäß Anspruch 7, wobei der DIGRA die Formel II oder III besitzt

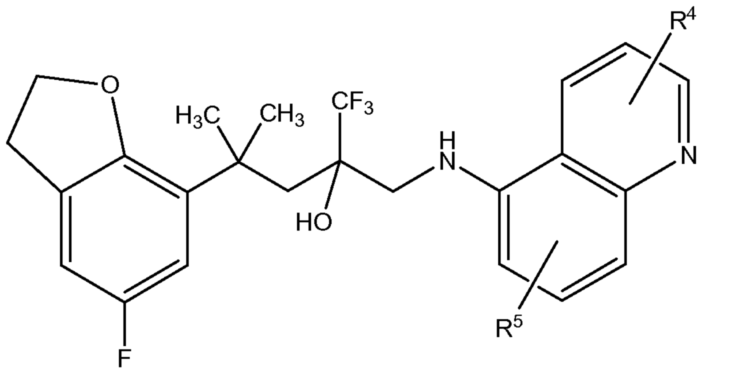

(II)

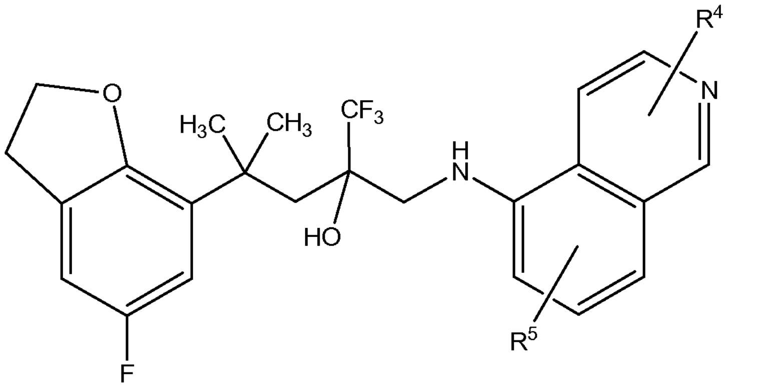

(III)

wobei $R^4$ und $R^5$ unabhängig ausgewählt sind aus der Gruppe bestehend aus unsubstituierten linearen oder verzweigten $C_1$-$C_{10}$-Alkylgruppen, substituierten linearen oder verzweigten $C_1$-$C_{10}$-Alkylgruppen, unsubstituierten cyclischen $C_3$-$C_{10}$-Alkylgruppen, und substituierten cyclischen $C_3$-$C_{10}$-Alkylgruppen.

**9.** Das Verfahren gemäß Anspruch 7, wobei der DIGRA die Formel IV besitzt

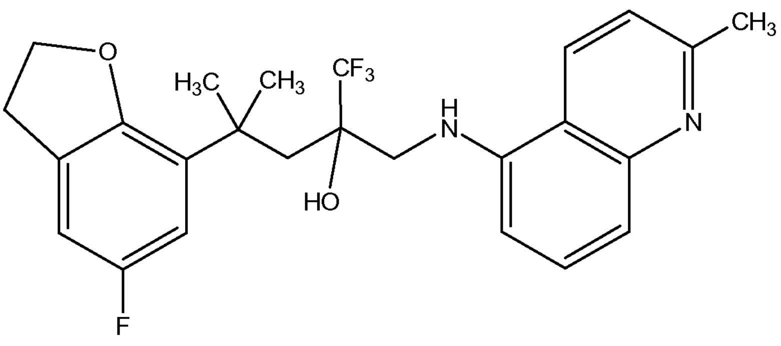

(IV)

## Revendications

**1.** Composition à utiliser pour le traitement ou le contrôle d'une inflammation oculaire postopératoire du segment antérieur de l'oeil, ladite inflammation résultant d'une procédure choisie dans le groupe constitué par la kératectomie

photoréfractive, la chirurgie de la cataracte, l'implantation d'une lentille intraoculaire (« LIO »), la kératomileusis in situ assistée au laser (« LASIK »), kératoplastie conductrice, et la kératotomie radiale ; ladite composition comprenant un agoniste de récepteur de glucocorticoïde dissocié (« DIGRA »), ou un sel pharmaceutiquement acceptable celui-ci ; où le DIGRA contient un composé ayant la formule I

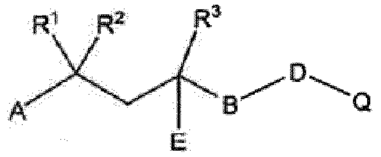

(I)

où A comprend un groupe dihydrobenzofuranyle substitué par un atome d'halogène ; Q comprend un groupe quinolinyle ou isoquinolinyle substitué par un groupe alkyle en $C_1$-$C_{10}$, $R^1$ et $R^2$ sont choisis indépendamment dans le groupe constitué par les groupes substitués et non substitués alkyle en $C_1$-$C_5$ ; B est un groupe alkylène en $C_1$-$C_3$ ; D est le groupe -NH, E est le groupe hydroxy ; et R3 comprend un groupe alkyle en $C_1$-$C_{10}$ totalement halogéné.

**2.** Composition à utiliser selon la revendication 1, où la composition entraîne un niveau inférieur d'au moins un effet secondaire indésirable chez un sujet par rapport à un autre composition comprenant au moins un glucocorticoïde utilisé pour traiter ou contrôler la même inflammation oculaire, où ledit effet secondaire indésirable est une pression intraoculaire élevée.

**3.** Composition à utiliser selon la revendication 2, où le DIGRA a la formule I

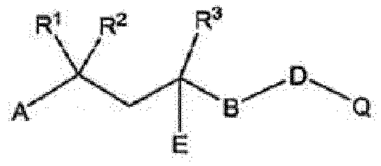

(I)

où A contient un groupe dihydrobenzofuranyle substitué par un atome de fluor ; Q comprend un groupe quinolinyle ou un groupe isoquinolinyle substitué par un groupe méthyle ; $R^1$ et $R^2$ sont choisis indépendamment dans le groupe constitué par les groupes alkyle en $C_1$-$C_5$ non substitués et substitués ; B est un groupe alkylène en $C_1$-$C_3$ ; D est le groupe -NH ; E est le groupe hydroxy et $R^3$ comprend un groupe trifluorométhyle.

**4.** Composition à utiliser selon la revendication 2, où le DIGRA a la formule II ou III

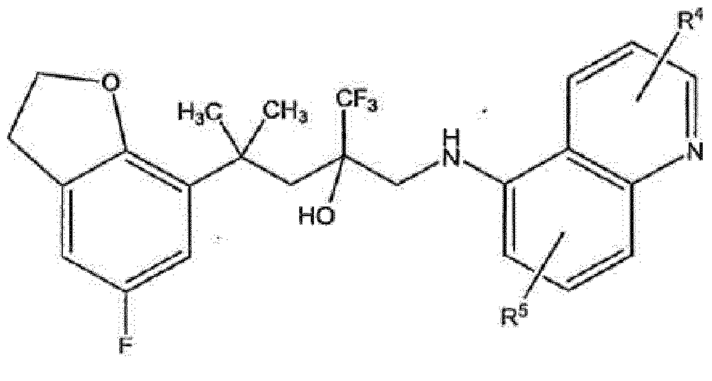

(II)

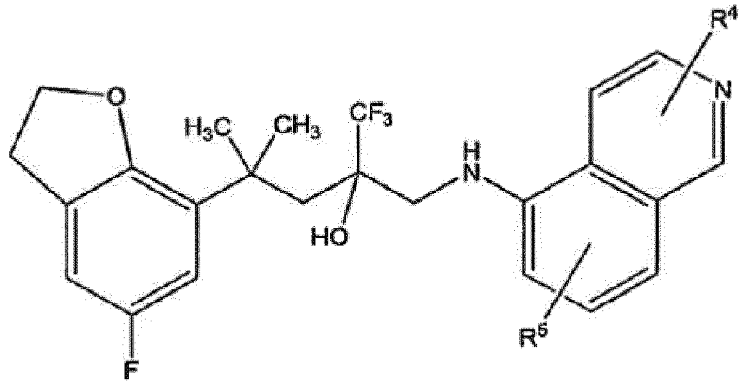

(III)

où $R^4$ et $R^5$ sont choisis indépendamment dans le groupe constitué par les groupes alkyle linéaires ou ramifiés en $C_1$-$C_{10}$ non substitués, les groupes alkyle linéaires ou ramifiés en $C_1$-$C_{10}$ substitués, les groupes alkyle cycliques en $C_3$-$C_{10}$ non substitués, et les groupes alkyle cycliques en $C_3$-$C_{10}$ substitués.

**5.** Composition à utiliser selon la revendication 3, où le DIGRA a la formule IV

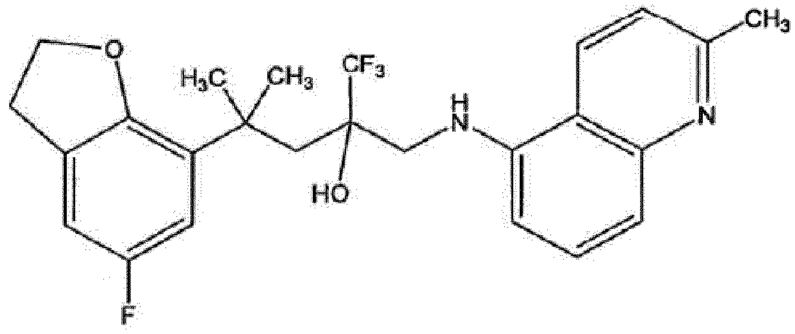

(IV)

**6.** Composition à utiliser selon la revendication 5, comprenant en outre un agent anti-inflammatoire choisi dans le groupe constitué par les AINS, les agonistes de PPAR, des combinaisons de ceux-ci et des mélanges de ceux-ci.

**7.** Procédé de fabrication d'une composition pour le traitement ou le contrôle de l'inflammation oculaire postopératoire du segment antérieur de l'oeil, ladite inflammation résultant d'une procédure choisie dans le groupe constitué par la kératectomie photoréfractive, la chirurgie de la cataracte, l'implantation d'une lentille intraoculaire (« LIO »), kératomileusis in situ assistée au laser
(« LASIK »), kératoplastie conductrice, et la kératotomie radiale ; le procédé consistant :

(a) à fournir un DIGRA, ou un sel pharmaceutiquement acceptable de celui-ci ; et
(b) à combiner ledit DIGRA, ou un sel pharmaceutiquement acceptable de
celui-ci, avec un support pharmaceutiquement acceptable ;
où le DIGRA comprend un composé ayant la formule I

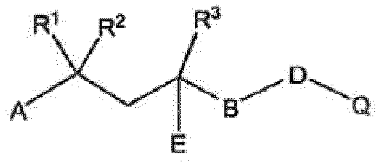

(I)

où A comprend un groupe dihydrobenzofuranyl substitué par un atome d'halogène ; Q comprend un groupe quinolinyle ou un groupe isoquinolinyle substitué par un groupe alkyle en $C_1$-$C_{10}$ ; $R^1$ et $R^2$ sont choisis indépendamment dans le groupe constitué par les groupes alkyle en $C_1$-$C_5$ non substitués et substitués ; B est un groupe alkylène en $C_1$-$C_3$ ; D est le groupe -NH ; E est le groupe hydroxy ; et $R^3$ comprend un groupe alkyle

en $C_1$-$C_{10}$ totalement halogéné.

**8.** Procédé selon la revendication 7, où le DIGRA a la formule II ou III

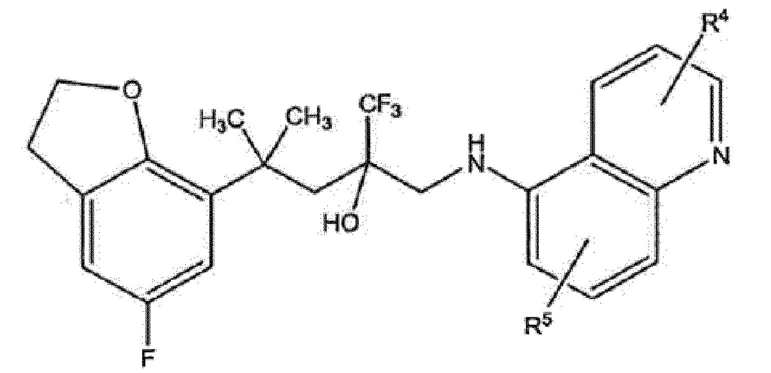

(II)

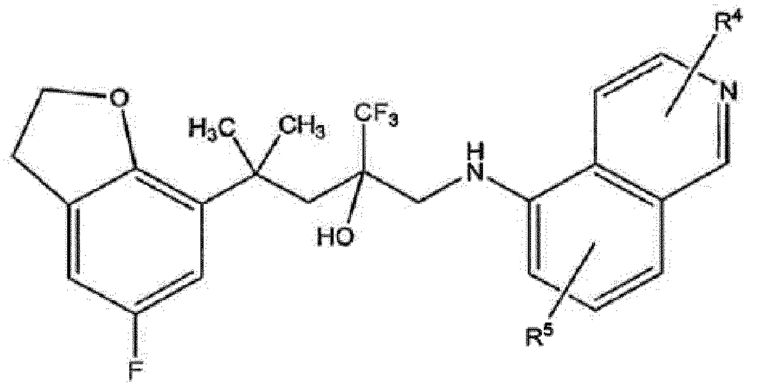

(III)

où $R^4$ et $R^5$ sont choisis indépendamment dans le groupe constitué par les groupes alkyle linéaires ou ramifiés en $C_1$-$C_{10}$ non substitués, les groupes alkyle linéaires ou ramifiés en $C_1$-$C_{10}$ substitués, les groupes alkyle cycliques en $C_3$-$C_{10}$ non substitués, et les groupes alkyle cycliques en $C_3$-$C_{10}$ substitués.

**9.** Procédé selon la revendication 7, où le DIGRA a la formule IV.

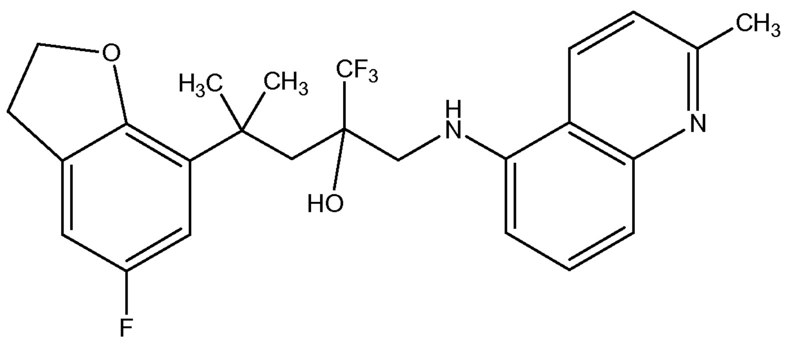

(IV)

FIG. 1A

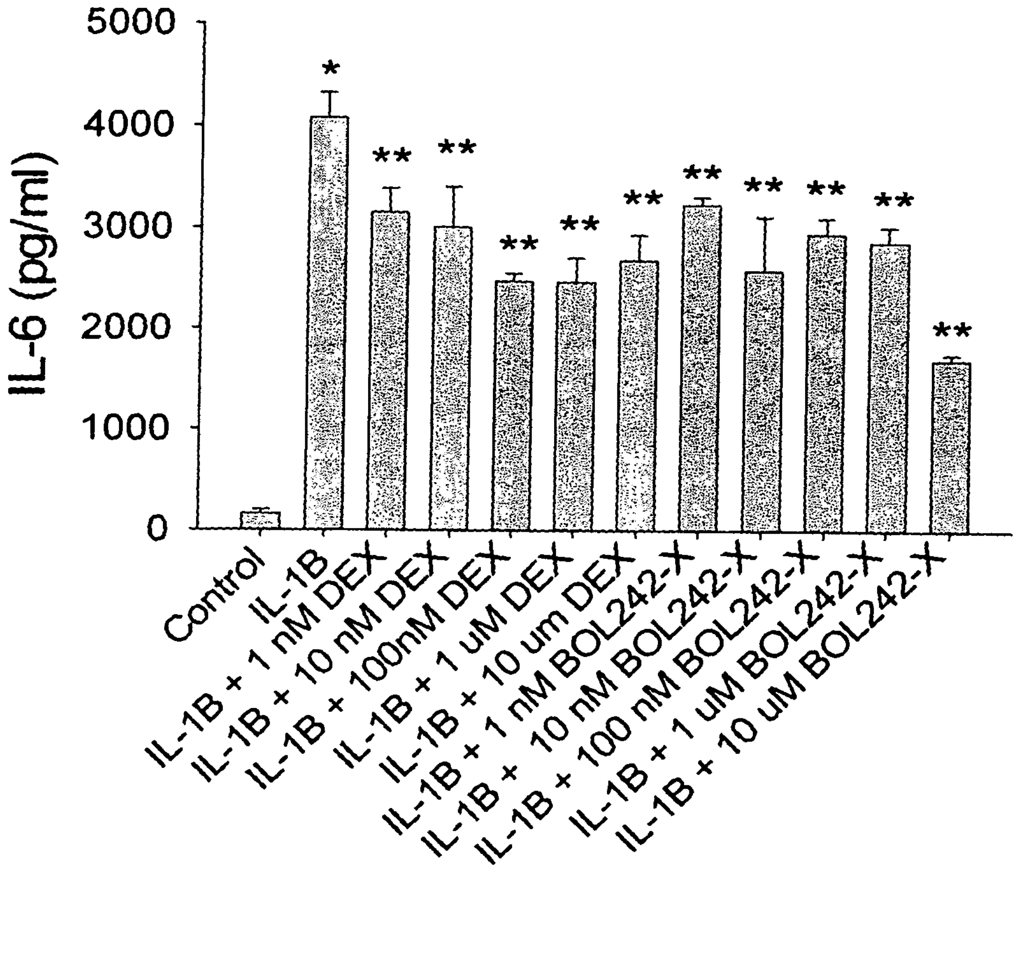
IL-6 (pg/ml)
5000
4000
3000
2000
1000
0
*
**
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X

FIG. 1B

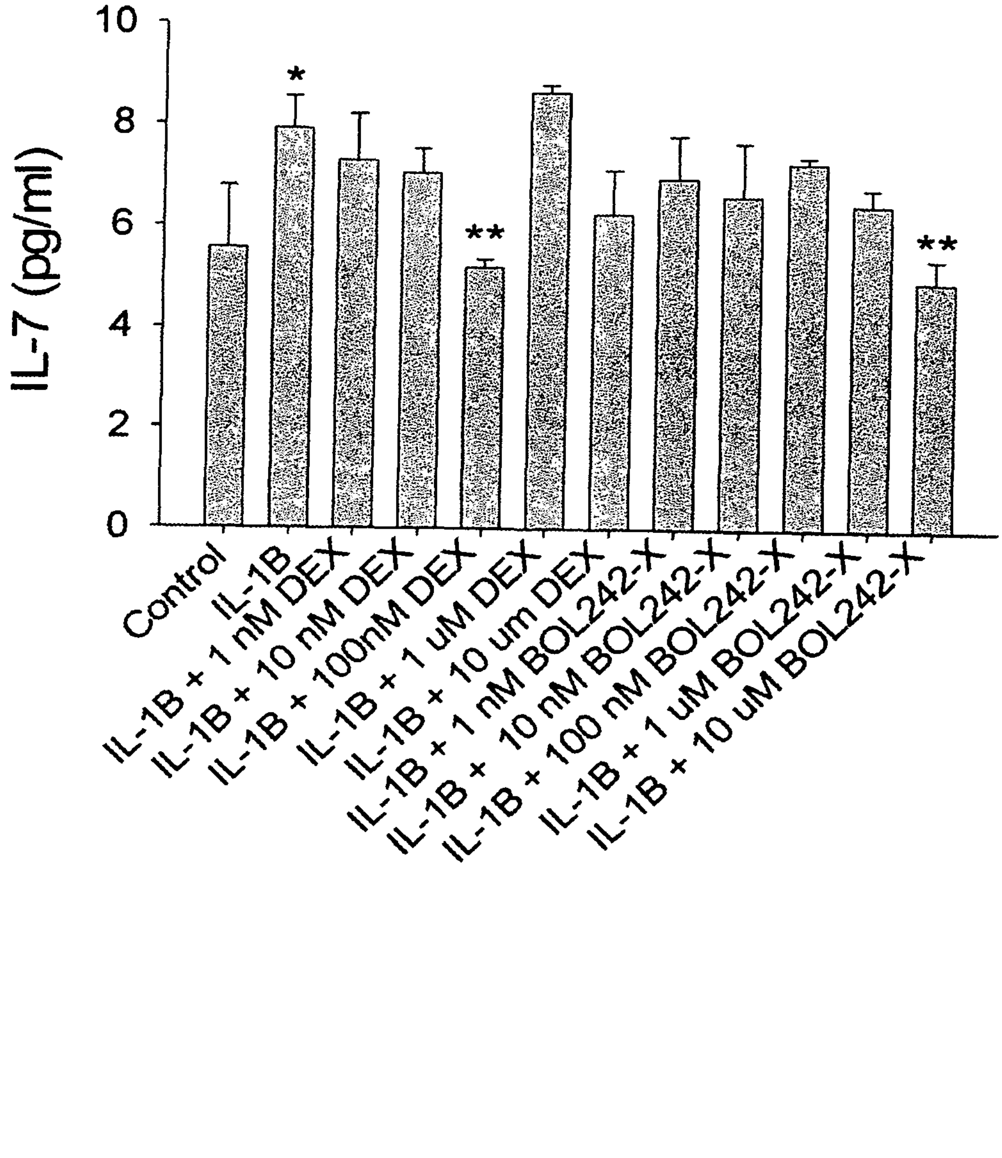

IL-7 (pg/ml)
0
2
4
6
8
10
*
**
**
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X

FIG. 1C

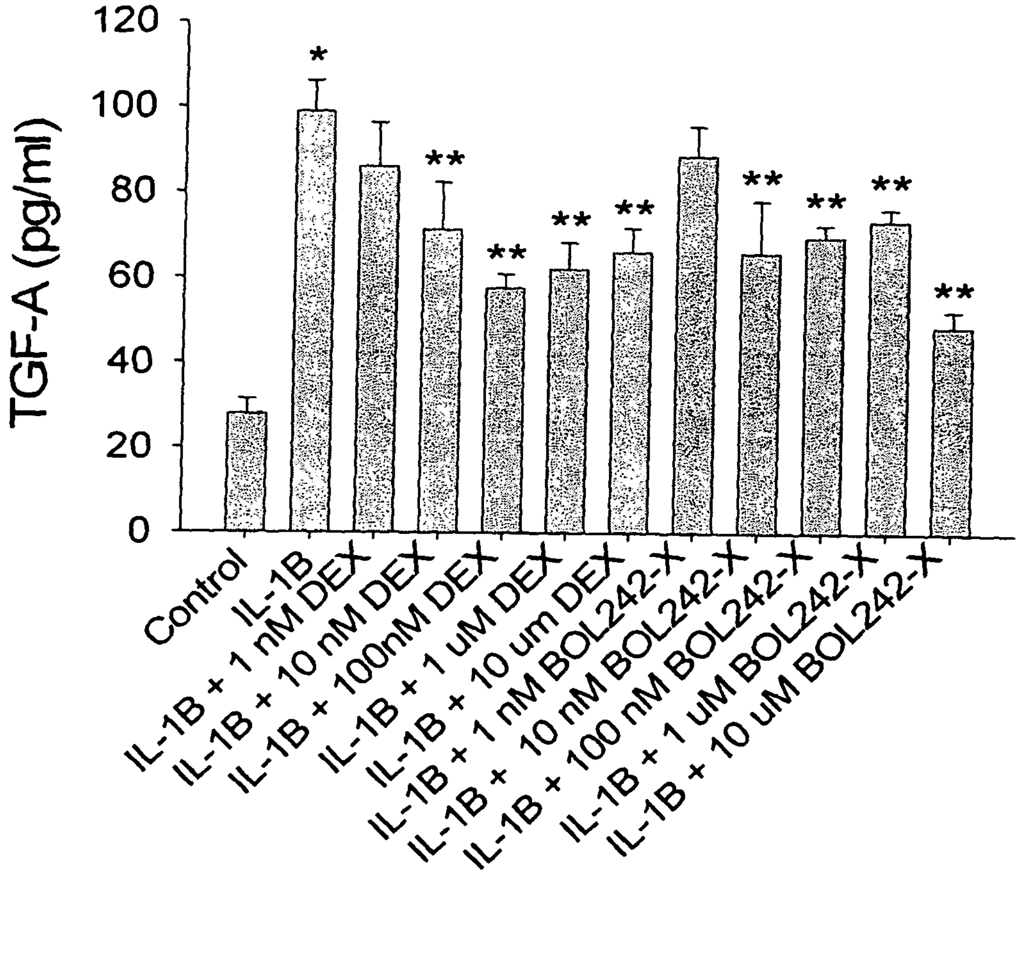
TGF-A (pg/ml)
0
20
40
60
80
100
120
*
**
**
**
**
**
**
**
**
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X

FIG. 1D

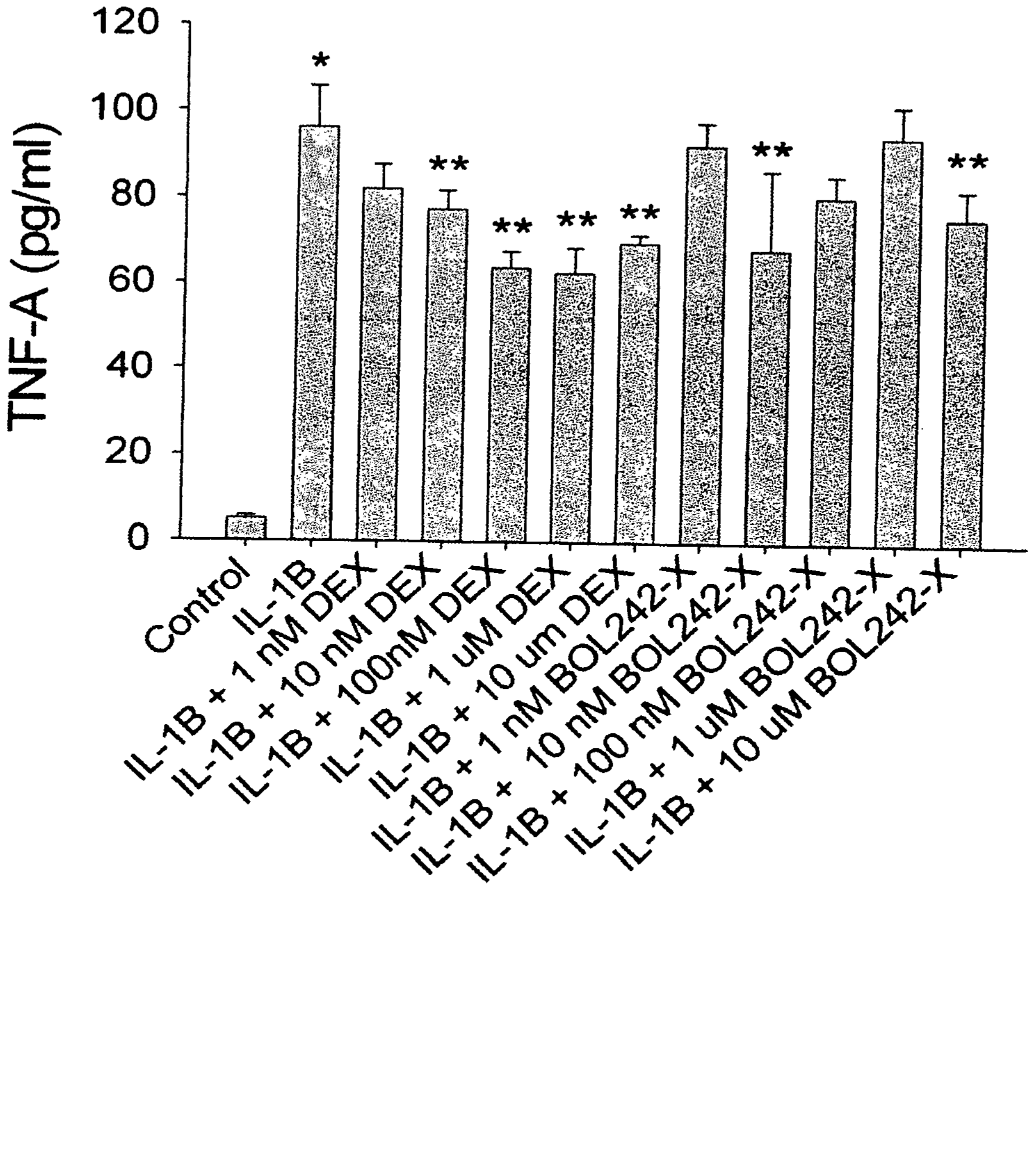
TNF-A (pg/ml)
0
20
40
60
80
100
120
*
**
**
**
**
**
**
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X

FIG. 1E

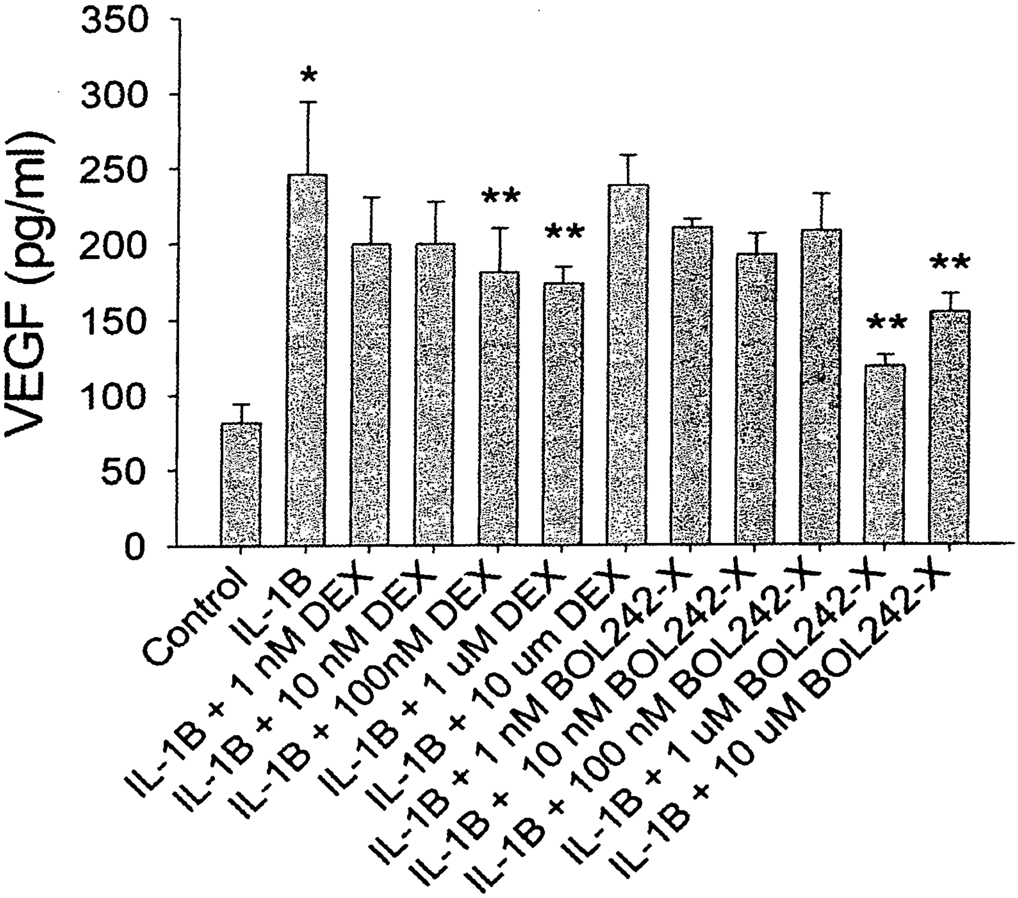
VEGF (pg/ml)
350
300
250
200
150
100
50
0
*
**
**
**
**
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X

FIG. 1F

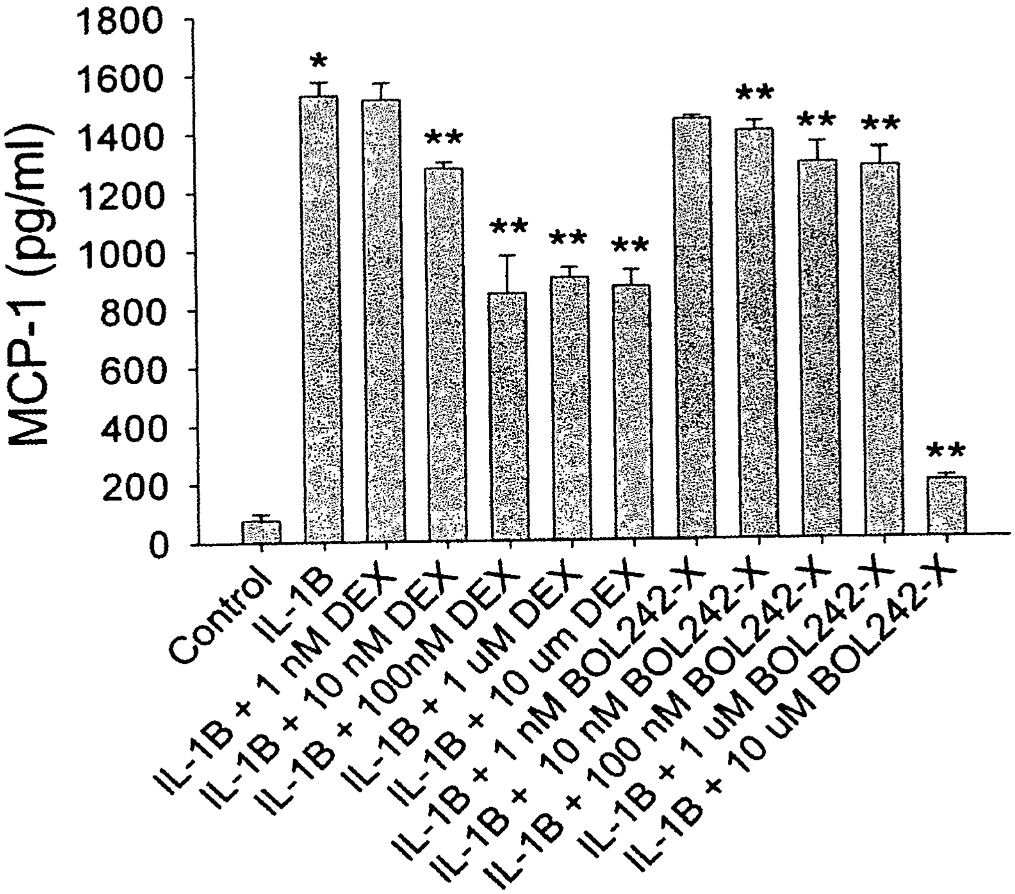
MCP-1 (pg/ml)
1800
1600
1400
1200
1000
800
600
400
200
0
*
**
**
**
**
**
**
**
**
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X

# FIG. 2

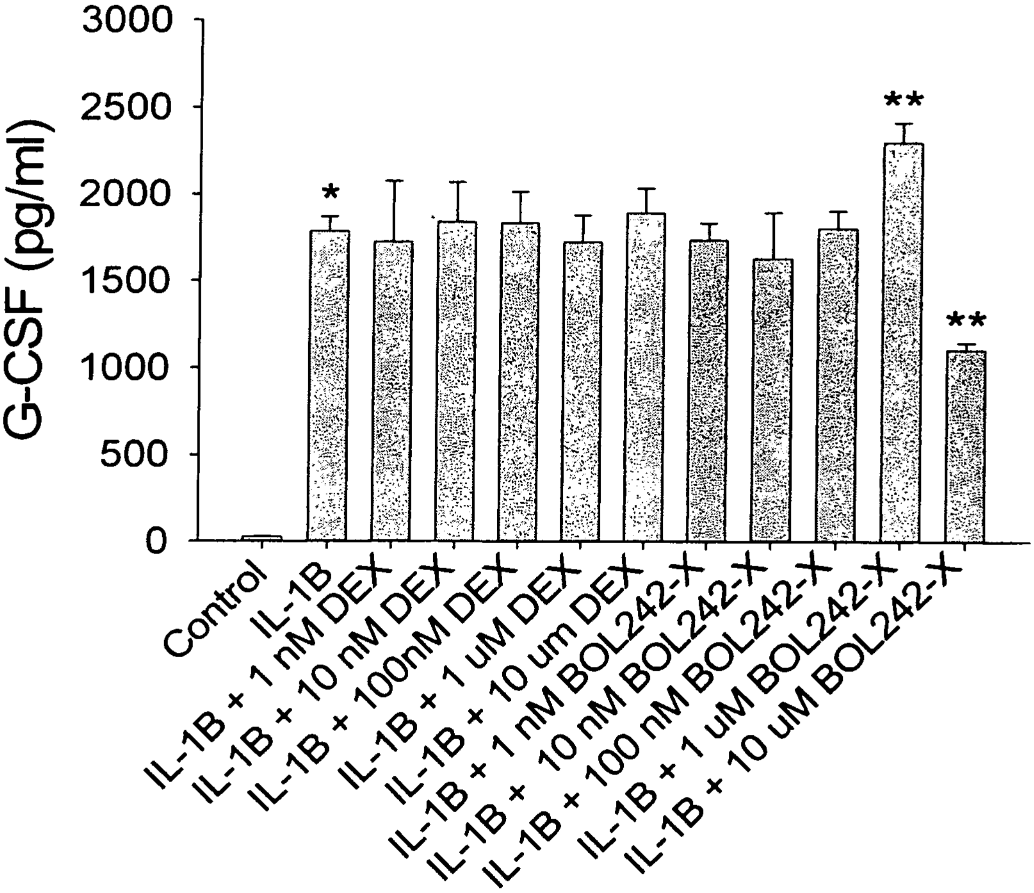

G-CSF (pg/ml)
3000
2500
2000
1500
1000
500
0
*
**
**
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X

FIG. 3A

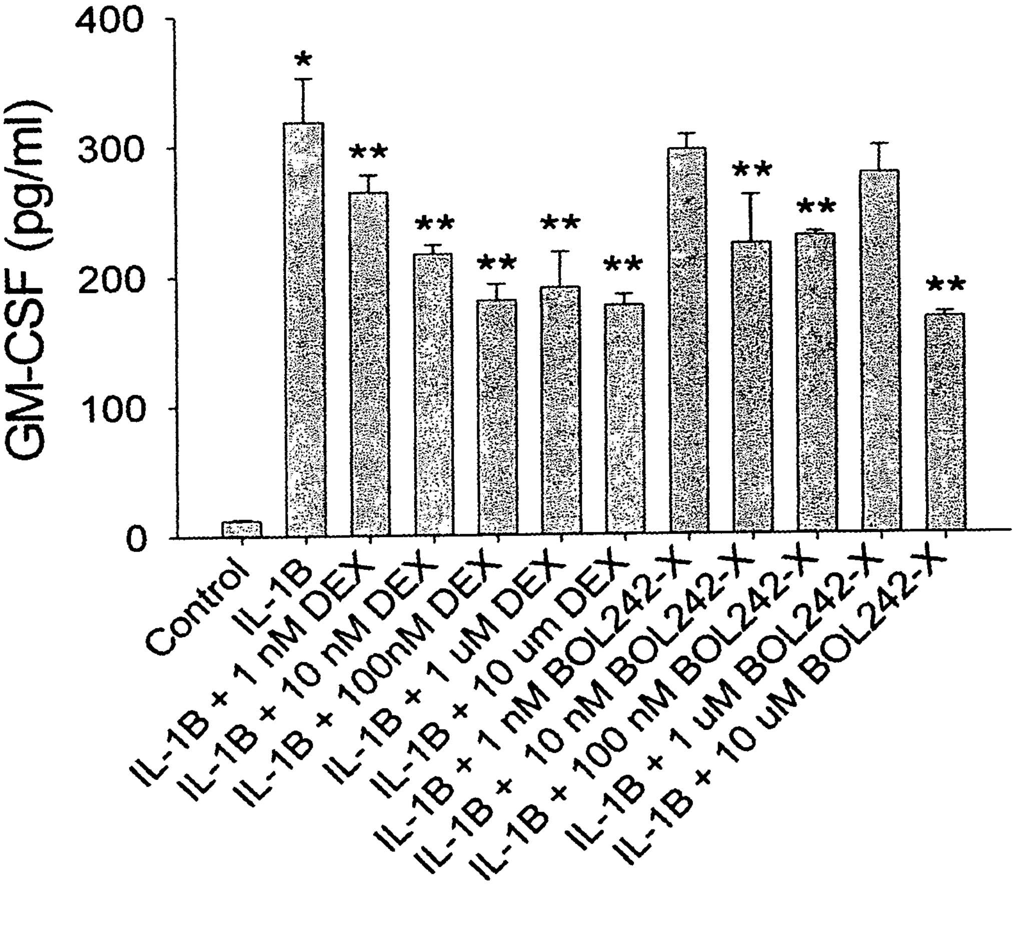

GM-CSF (pg/ml)
400
300
200
100
0
*
**
**
**
**
**
**
**
**
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X

# FIG. 3B

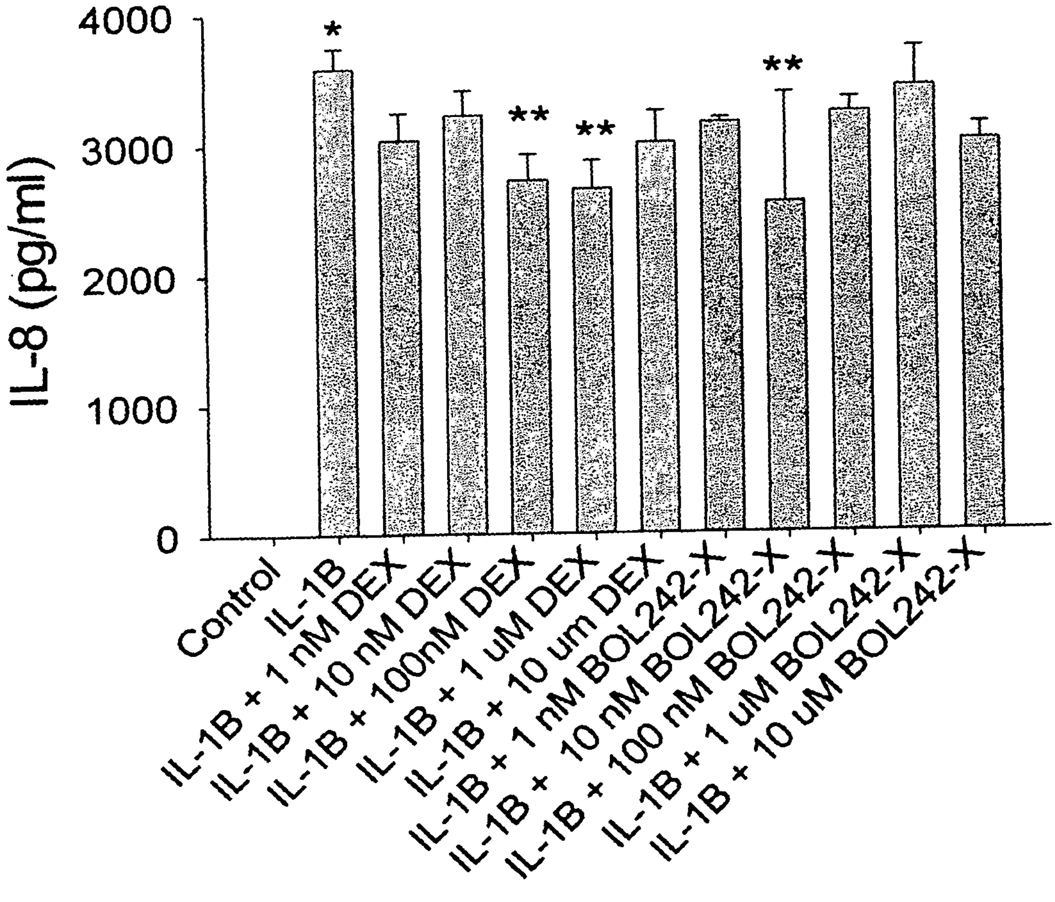
4000
3000
2000
1000
0
IL-8 (pg/ml)
*
**
**
**
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X

FIG. 3C

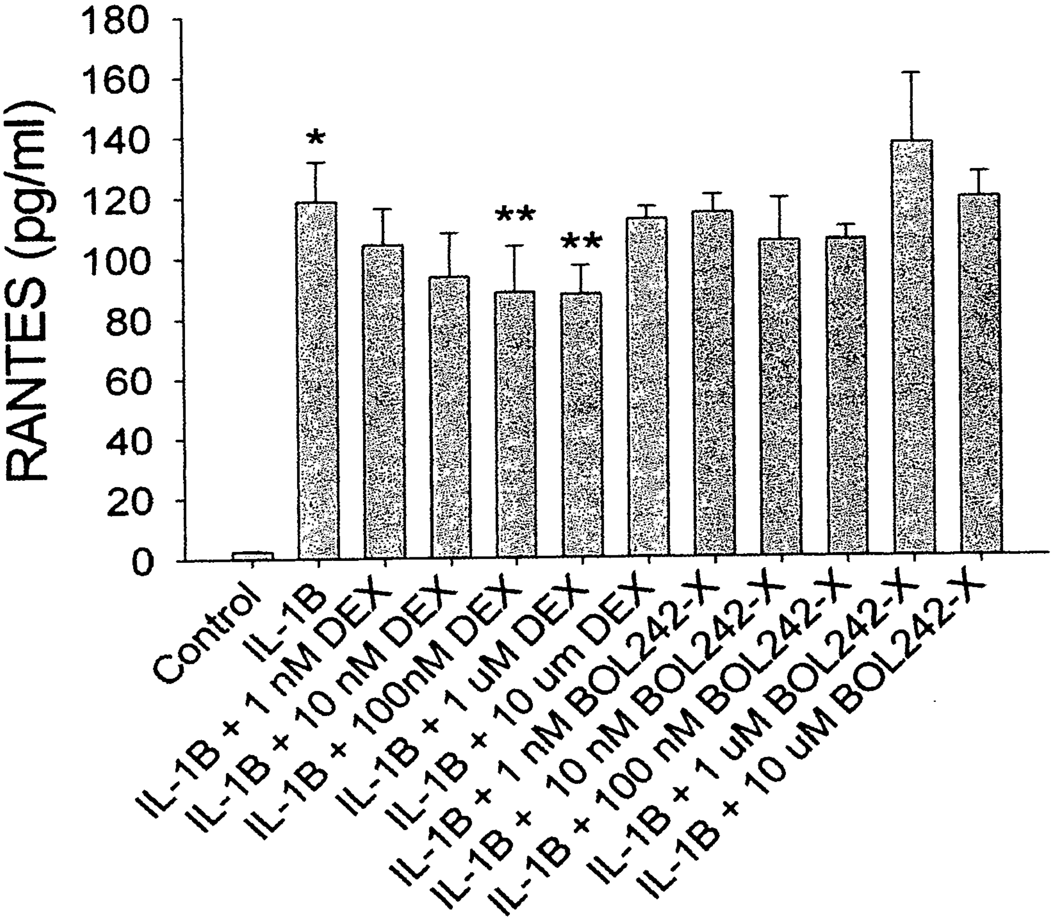
RANTES (pg/ml)
180
160
140
120
100
80
60
40
20
0
*
**
**
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6897224 B
- US 6903215 B
- US 6960581 B
- US 20060116396 A
- WO 2006050998A1 A
- US 20040029932 A
- US 20040162321 A
- US 20040224992 A
- US 20050059714 A
- US 20050176706 A
- US 20050203128 A
- US 20050234091 A
- US 20050282881 A
- US 20060014787 A
- US 20060030561 A
- US 20060189646 A
- US 20060189647 A
- US 5385900 A
- US 5447926 A
- US 6242196 B, Spiegelman
- US 6316465 B
- US 5378475 A
- US 5773019 A
- US 5902598 A
- US 6001386 A
- US 6051576 A
- US 6726918 A
- US 20040219512 A


### Non-patent literature cited in the description


- **V.W.M. VAN HINSBERGH.** *Arteriosclerosis, Thrombosis, and Vascular Biology,* 1997, vol. 17, 1018
- **P.J. BARNES.** *Clin. Sci.,* 1998, vol. 94, 557-572
- **S. WISSINK et al.** *Mol. Endocrinol.,* 1998, vol. 12 (3), 354-363
- **P.J. BARNES ; M. KARIN.** *New Engl. J. Med.,* 1997, vol. 336, 1066-1077
- **H. SCHÄCKE et al.** *Pharmacol. Ther.,* 2002, vol. 96, 23-43
- The Complete Drug Reference. Pharmaceutical Press, 2005, 1411-1416
- **REMINGTON.** The Science and Practice of Pharmacy. Lippincott Williams & Wilkins, 2006, 291
- **C-Y. JIANG et al.** *Nature,* 1998, vol. 391, 82-86
- **A.E. GIORGINI et al.** *Horm. Metab. Res.,* 1999, vol. 31, 1-4
- **X. XIN et al.** *J. Biol. Chem.,* 1999, vol. 274, 9116-9121
- **EAKINS KE.** Prostaglandin and non prostaglandin-mediated breakdown of the blood-aqueous barrier. *Exp. Eye Res.,* 1977, vol. 25, 483-498
- **NEUFELD AH ; SEARS ML.** The site of action of prostaglandin E2 on the disruption of the blood-aqueous barrier in the rabbit eye. *Exp. Eye Res.,* 1973, vol. 17, 445-448
- **UNGER WG ; COLE DP ; HAMMOND B.** Disruption of the blood-aqueous barrier following paracentesis in the rabbit. *Exp. Eye Res.,* 1975, vol. 20, 255-270
- Autacoids and Neuropeptides. **STJEMSCHANTZ J.** Pharmacology of the Eye. Springer-Verlag, 1984, 311-365
- **WILLIAMS RN ; PATERSON CA ; EAKINS KE ; BHATTACHERJEE P.** Quantification of ocular inflammation: evaluation of polymorphonuclear leukocyte infiltration by measuring myeloperoxidase activity. *Curr. Eye Res.,* 1983, vol. 2, 465-469